# EUROPÄISCHE PATENTANMELDUNG

(11) **EP 4 800 010 A1**
(43) Veröffentlichungstag der Anmeldung: **02.09.2026**
(21) Anmeldenummer: 25160630.7
(22) Anmeldetag: 27.02.2025
(51) Int. Cl.: C07D 251/52, C07D 251/54, A01N 43/70, A01P 13/02

(54) **SUBSTITUIERTE INDANYLAMINOTRIAZINE SOWIE DEREN SALZE UND IHRE VERWENDUNG ALS HERBIZIDE WIRKSTOFFE**

(71) Anmelder: Bayer Aktiengesellschaft, 51373 Leverkusen (DE)
(72) Erfinder: Die Erfindernennung liegt noch nicht vor
(74) Vertreter: BIP Patents

(57) **Zusammenfassung**

Offenbart werden substituierte Indanylaminotriazine sowie deren Salze, Verfahren zu ihrer Herstellung und ihre Verwendung als Herbizide, insbesondere zur Bekämpfung von Unkräutern und/oder Ungräsern in Nutzpflanzenkulturen und/oder als Pflanzenwachstumsregulatoren zur Beeinflussung des Wachstums von Nutzpflanzenkulturen. Auch umfaßt ist die Verwendung von substituierten Indanylaminotriazinen und deren Salzen für die Herstellung von herbizid wirkenden Mitteln, als auch herbizid wirkende Mittel enthaltend genannte Verbindungen.

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen.

Speziell betrifft diese Erfindung substituierte Indanylaminotriazine sowie deren Salze, Verfahren zu ihrer Herstellung und ihre Verwendung als Herbizide, insbesondere zur Bekämpfung von Unkräutern und/oder Ungräsern in Nutzpflanzenkulturen und/oder als Pflanzenwachstumsregulatoren zur Beeinflussung des Wachstums von Nutzpflanzenkulturen.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es, daß sie (a) keine oder aber eine unzureichende herbizide Wirkung gegen bestimmte Schadpflanzen, (b) ein zu geringes Spektrum der Schadpflanzen, das mit einem Wirkstoff bekämpft werden kann, (c) eine zu geringe Selektivität in Nutzpflanzenkulturen und/oder (d) ein toxikologisch ungünstiges Profil besitzen. Weiterhin führen manche Wirkstoffe, die als Pflanzenwachstumsregulatoren bei einigen Nutzpflanzen eingesetzt werden können, bei anderen Nutzpflanzen zu unerwünscht verminderten Ernteerträgen oder sind mit der Kulturpflanze nicht oder nur in einem engen Aufwandmengenbereich verträglich. Einige der bekannten Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab. Die herbizide Wirkung dieser bekannten Verbindungen, insbesondere bei niedrigen Aufwandmengen, bzw. deren Verträglichkeit gegenüber Kulturpflanzen bleiben verbesserungswürdig.

Es ist aus verschiedenen Schriften bekannt, daß bestimmte substituierte Aminotriazine als herbizide Wirkstoffe zur Kontrolle unerwünschter Schadpflanzen verwendet werden können (vgl. WO1997/031904, WO1990/009378 und WO1994/024086). Indanyl-substituierte Aminotriazine und ihre Wirkung zur Kontrolle unerwünschter Schadpflanzen sind ebenfalls beschrieben (vgl. WO1997/031904, US20100016158, WO1997/019936, JP08183781). Die Synthese von chiralen Indanylaminen ist in WO2004/069814, WO 2015/113903 und WO2024/201469 beschrieben.

Es ist weiterhin bekannt, daß substituierte Aminotriazine als Kaliumkanal-Inhibitoren zur Behandlung neurodegenerativer Erkrankungen verwendet werden können (WO2003066099), während bestimmte Indanylcarboxamide auch als pharmazeutische Wirkstoffe eingesetzt werden können, die beispielsweise mit dem Bradykinin 1-Rezeptor wechselwirken (US20120071461). Weiterhin sind ausgewählte Chloracetyl-substituierte Indanylamine beschrieben (vgl. WO2004/069814 und US20040157739). Besondere substituierte Indanylaminotriazine, die in der Triazineinheit neben der unsubstituierten Aminogruppe als weiteren Substituenten einen Rest tragen, bei dem es sich um einen Amino-, Oxy-, Sulfenyl-, Sulfinyl oder Sulfonylrest handelt und herbizide Wirkung zeigen, sind dagegen noch nicht beschrieben.

Überraschenderweise wurde nun gefunden, daß bestimmte substituierte Indanylaminotriazine oder deren Salze als Herbizide gut geeignet sind und besonders vorteilhaft als Wirkstoffe zur Bekämpfung von monokotylen und dikotylen Unkräutern in Nutzpflanzenkulturen eingesetzt werden können.

Ein Gegenstand der vorliegenden Erfindung sind Verbindungen der allgemeinen Formel (I) oder deren Salze, 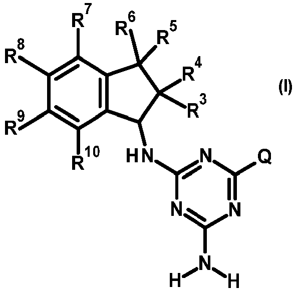 worin
- Q: für die Gruppen 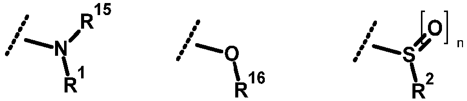 steht, wobei die gestrichelte Linie jeweils die Verknüpfungsstelle zum restlichen Teil der Formel (I) kennzeichnet,
- R¹ und R¹⁶: unabhängig für Wasserstoff, (C₁-C₈)-Alkyl, (C₃-C₈)-Cycloalkyl-(C₁-C₈)-alkyl, (C₁-C₈)-Cyanoalkyl, (C₂-C₈)-Alkenyl, (C₂-C₈)-Alkinyl, R¹³O-(C₁-C₈)-alkyl, R¹¹R¹²N-(C₁-C₈)-alkyl, R¹³O(O)C-(C₁-C₈)-alkyl, R¹¹R¹²N(O)C-(C₁-C₈)-alkyl stehen,
- R²: für (C₁-C₈)-Alkyl, (C₁-C₈)-Haloalkyl, (C₃-C₈)-Cycloalkyl, (C₃-C₈)-Halocycloalkyl, (C₃-C₈)-Cycloalkyl-(C₁-C₈)-alkyl, R¹³O-(C₁-C₈)-alkyl, R¹¹R¹²N-(C₁-C₈)-alkyl, R¹³O(O)C-(C₁-C₈)-alkyl, R¹¹R¹²N(O)C-(C₁-C₈)-alkyl, (C₂-C₈)-Alkenyl, (C₂-C₈)-Haloalkenyl, (C₄-C₈)-Cycloalkenyl, Heterocyclyl, oder für Aryl, Aryl-(C₁-C₈)-alkyl steht, wobei die Arylgruppe gegebenenfalls weiter 1 bis 5 Substituenten trägt, die unabhängig voneinander für Halogen, Cyano, Nitro, Trimethylsilyl, Pentafluorsulfanyl, -C(=S)NH₂, OR¹³, NR¹¹R¹², SR¹⁴, S(O)R¹⁴, SO₂R¹⁴, C(O)OR¹³, C(O)R¹³, (C₁-C₈)-Alkyl, (C₃-C₈)-Cycloalkyl, (C₃-C₈)-C_{Y}Cloalkyl-(C₁-C₈)-alkyl, (C₂-C₈)-Alkenyl, (C₂-C₈)-Haloalkenyl, (C₂-C₈)-Alkinyl, (C₂-C₈)-Haloalkinyl, (C₃-C₈)-Cycanocycloalkyl, (C₃-C₆)-Halocycloalkyl, (C₁-C₈)-Haloalkyl, (C₁-C₈)-Cyanoalkyl stehen, oder für Heteroaryl, Heteroaryl-(C₁-C₈)-alkyl, Heterocyclyl-(C₁-C₈)-alkyl steht und wobei Heterocyclyl n Oxogruppen trägt,
- R³: für (C₁-C₈)-Alkyl, (C₁-C₈)-Haloalkyl, (C₃-C₈)-Cycloalkyl, (C₂-C₈)-Alkenyl, R¹³O-(C₁C₈)-alkyl, (C₃-C₈)-Cycloalkyl-(C₁-C₈)-alkyl steht,
- R⁴, R⁵, R⁶: unabhängig voneinander für Wasserstoff, (C₁-C₈)-Alkyl, (C₁-C₈)-Haloalkyl stehen,
- R⁷, R⁸, R¹⁰: unabhängig voneinander für Wasserstoff, Halogen, OR¹³, (C₁-C₈)-Alkyl, (C₁-C₈)-Haloalkyl stehen,
- R⁹: für Halogen, Nitro, Cyano, (C₁-C₈)-Alkyl, R¹³O-(C₁-C₈)-alkyl, R¹⁴S-(C₁-C₈)-alkyl, (C₁-C₈)-Haloalkyl, (C₃-C₈)-Cycloalkyl, (C₃-C₈)-Halocycloalkyl, (C₂-C₈)-Alkenyl, (C₂-C₈)-Haloalkenyl, (C₄-C₈)-Cycloalkenyl, (C₂-C₈)-Alkinyl, (C₂-C₈)-Haloalkinyl, Heterocyclyl, OR¹³, NR¹¹R¹², SR¹⁴, S(O)R¹⁴, SO₂R¹⁴, C(O)OR¹³, C(O)R¹³ steht,
- R¹¹ und R¹²: gleich oder verschieden sind und unabhängig voneinander für Wasserstoff, (C₁-C₈)-Alkyl, (C₃-C₈)-Alkenyl, (C₃-C₈)-Alkinyl, (C₁-C₈)-Cyanoalkyl, (C₁-C₈)-Haloalkyl, (C₃-C₈)-Haloalkenyl, (C₃-C₈)-Haloalkinyl, (C₃-C₈)-Cycloalkyl, (C₃-C₈)-Halocycloalkyl, (C₄-C₈)-Cycloalkenyl, (C₄-C₈)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkylthio-(C₁-C₈)-alkyl, (C₁-C₈)-Haloalkylthio-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, (C₃-C₈)-Cycloalkyl-(C₁-C₈)-alkyl, (C₄-C₈)-Cycloalkenyl-(C₁-C₈)-alkyl, COR¹³, SO₂R¹⁴, Heterocyclyl, (C₁-C₈)-Alkoxycarbonyl, Bis-[(C₁-C₈)-alkyl]aminocarbonyl-(C₁-C₈)-alkyl, (C₁-C₈)-Alkyl-aminocarbonyl-(C₁-C₈)-alkyl, Aryl-(C₁-C₈)-alkyl-aminocarbonyl-(C₁-C₈)-alkyl, Aryl-(C₁-C₈)-alkoxycarbonyl, Heteroaryl-(C₁-C₈)-alkoxycarbonyl, (C₃-C₈)-Alkenyloxycarbonyl, (C₃-C₈)-Alkinyloxycarbonyl, Heterocyclyl-(C₁-C₈)-alkyl stehen, wobei Heterocyclyl n Oxogruppen trägt, oder
- R¹¹ und R¹²: mit dem Stickstoffatom, an das sie gebunden sind, einen vollständig gesättigten oder teilgesättigten, gegebenenfalls durch weitere Heteroatome unterbrochenen und gegebenenfalls weiter substituierten 3 bis 10-gliedrigen monocyclischen oder bicyclischen Ring bilden,
- R¹³: für Wasserstoff, (C₁-C₈)-Alkyl, (C₃-C₈)-Alkenyl, (C₃-C₈)-Alkinyl, (C₁-C₈)-Cyanoalkyl, (C₁-C₈)-Haloalkyl, (C₃-C₈)-Haloalkenyl, (C₃-C₈)-Haloalkinyl, (C₃-C₈)-Cycloalkyl, (C₄-C₈)-Cycloalkenyl, (C₄-C₈)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkoxy-(C₁-C₈)-haloalkyl, (C₁-C₈)-Alkoxy-(C₁-C₈)-alkoxy-(C₁-C₈)-alkyl, (C₁-C₈)-Alkoxy-(C₁-C₈)-alkoxy-(C₁-C₈)-alkoxy-(C₁-C₈)-alkyl, (C₁-C₈)-Alkoxy-(C₁-C₈)-alkoxy-(C₁-C₈)-alkoxy-(C₁-C₈)-alkoxy-(C₁-C₈)-alkyl, Aryl, Aryl-(C₁-C₈)-alkyl, Aryl-(C₁-C₈)-alkoxy-(C₁-C₈)-alkyl, Heteroaryl, Heteroaryl-(C₁-C₈)-alkyl, (C₃-C₈)-Cycloalkyl-(C₁-C₈)-alkyl, (C₄-C₈)-Cycloalkenyl-(C₁-C₈)-alkyl, Bis-[(C₁-C₈)-alkyl]aminocarbonyl-(C₁-C₈)-alkyl, (C₁-C₈)-Alkylaminocarbonyl-(C₁-C₈)-alkyl, Aryl-(C₁-C₈)-alkyl-aminocarbonyl-(C₁-C₈)-alkyl, Bis-[(C₁-C₈)-alkyl]amino-(C₂-C₆)-alkyl, (C₁-C₈)-Alkylamino-(C₂-C₆)-alkyl, Aryl-(C₁-C₈)-alkylamino-(C₂-C₆)-alkyl, R¹⁴S-(C₁-C₈)-alkyl, R¹⁴(O)S-(C₁-C₈)-alkyl, R¹⁴O₂S-(C₁-C₈)-alkyl, Hydroxycarbonyl-(C₁-Cs)-alkyl, Heterocyclyl, Heterocyclyl-(C₁-C₈)-alkyl, Tris-[(C₁-C₈)-Alkyl]silyl-(C₁-C₈)-alkyl, Bis-[(C₁-C₈)-Alkyl](aryl)silyl(C₁-C₈)-alkyl, [(C₁-C₈)-Alkyl]-bis-(aryl)silyl-(C₁-C₈)-alkyl, (C₁-C₈)-Alkylcarbonyloxy-(C₁-C₈)-alkyl, (C₃-C₈)-Cycloalkylcarbonyloxy-(C₁-C₈)-alkyl, Arylcarbonyloxy-(C₁-C₈)-alkyl, Heteroarylcarbonyloxy-(C₁-C₈)-alkyl, Heterocyclylcarbonyloxy-(C₁-C₈)-alkyl, Aryloxy-(C₁-C₈)-alkyl, Heteroaryloxy-(C₁-C₈)-alkyl, (C₁-C₈)-Alkoxycarbonyl steht, wobei Heterocyclyl n Oxogruppen trägt und wobei Cycloalkyl substituiert sein kann durch einen oder mehrere Substituenten ausgewählt aus einer Gruppe bestehend aus (C₁-C₈)-Alkyl, (C₁-C₈)-Haloalkyl, Halogen, Cyano, Carbamoyl und (C₁-C₈)-Alkylsulfonyl,
- R¹⁴: für (C₁-C₈)-Alkyl, (C₃-C₈)-Alkenyl, (C₃-C₈)-Alkinyl, (C₁-C₈)-Cyanoalkyl, (C₁-C₈)-Haloalkyl, (C₃-C₈)-Haloalkenyl, (C₃-C₈)-Haloalkinyl, (C₃-C₈)-Cycloalkyl, (C₄-C₈)-Cycloalkenyl, (C₄-C₈)-Halocycloalkenyl, (C₁-C₈)-Alkoxy-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, Heterocyclyl-(C₁-C₈)-alkyl, (C₃-C₈)-Cycloalkyl-(C₁-C₈)-alkyl, (C₄-C₈)-Cycloalkenyl-(C₁-C₈)-alkyl, Amino, Bis-[(C₁-C₈)-alkyl]amino, (C₁-C₈)-Alkyl-amino, Aryl-(C₁-C₈)-amino, Aryl-(C₁-C₆)-alkyl-amino, Aryl-[(C₁-C₈)-alkyl]amino, (C₃-C₈)-Cycloalkyl-amino, (C₃-C₈)-Cycloalkyl-[(C₁-C₈)-alkyllamino, N-Azetidinyl, N-Pyrrolidinyl, N-Piperidinyl, N-Morpholinyl steht,
wobei die zwölf letztgenannten Reste (Amino, Bis-[(C₁-C₈)-alkyl]amino, (C₁-C₈)-Alkylamino, Aryl-(C₁-C₈)-amino, Aryl-(C₁-C₆)-alkyl-amino, Aryl-[(C₁-C₈)-alkyl]amino, (C₃-C₈)-Cycloalkyl-amino, (C₃-C₈)-Cycloalkyl-[(C₁-C₈)-alkyl]amino, N-Azetidinyl, N-Pyrrolidinyl, N-Piperidinyl, N-Morpholinyl) nur an Sulfonylgruppen gebunden sind,
und wobei Heterocyclyl n Oxogruppen trägt,
und wobei Cycloalkyl substituiert sein kann durch einen oder mehrere Substituenten ausgewählt aus einer Gruppe bestehend aus (C₁-C₈)-Alkyl, (C₁-C₈)-Haloalkyl, Halogen, Cyano, Carbamoyl und (C₁-C₈)-Alkylsulfonyl,
- R¹⁵: für Wasserstoff, (C₁-C₈)-Alkyl, (C₃-C₈)-Cycloalkyl-(C₁-C₈)-alkyl, (C₃-C₈)-Alkenyl, (C₃-C₈)-Alkinyl steht und
- n: für 0, 1, 2 steht.

Bei den erfindungsgemäßen Verbindungen der allgemeinen Formel (I) und/oder deren Salzen handelt es sich um substituierte Indanylaminotriazine bzw. deren Salze.

In einer bevorzugten Ausführungsform handelt es sich bei den erfindungsgemäßen Verbindungen der allgemeinen Formel (I) und/oder deren Salzen um herbizd wirkende Verbindungen oder herbizide Verbindungen. Herbizide Verbindungen können in der Landwirtschaft zur Bekämpfung von Schadpflanzen oder als Wachstumsregulatoren eingesetzt werden.

Die Verbindungen der allgemeinen Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion. Geeignete Substituenten, die in deprotonierter Form, wie z.B. Sulfonsäuren, bestimmte Sulfonsäureamide oder Carbonsäuren, vorliegen, können innere Salze mit ihrerseits protonierbaren Gruppen, wie Aminogruppen bilden. Salzbildung kann auch durch Einwirkung einer Base auf Verbindungen der allgemeinen Formel (I) erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetall-salze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel [NR^{a}R^{b}R^{c}R^{d}]⁺, worin R^{a} bis R^{d} jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der allgemeinen Formel (I) besitzen mindestens ein Chiralitätszentrum, weshalb jeder Verbindung mindestens zwei Stereoisomere zugeordnet werden können. Die durch ihre spezifische Raumform definierten möglichen Stereoisomere, wie Enantiomere, Diastereomere, Z- und E-Isomere sind alle von der Formel (I) umfasst. Sind beispielsweise eine oder mehrere Alkenylgruppen vorhanden, so können Diastereomere (Z- und E-Isomere) auftreten. Sind beispielsweise ein oder mehrere asymmetrisch substituierte Kohlenstoffatome vorhanden, so können Enantiomere und Diastereomere auftreten. Stereoisomere lassen sich aus den bei der Herstellung anfallenden Gemischen nach üblichen Trennmethoden erhalten. Die chromatographische Trennung kann sowohl im analytischen Maßstab zur Feststellung des Enantiomerenüberschusses bzw. des Diastereomerenüberschusses, wie auch im präparativen Maßstab zur Herstellung von Prüfmustern für die biologische Ausprüfung erfolgen. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst, jedoch nicht mit ihrer spezifischen Stereoform angegeben sind, sowie deren Gemische. Getrennte Enantiomere oder Diastereomere, die von der allgemeinen Formel (I) umfasst sind, werden entweder (a) entsprechend ihrer optischen Aktivität mit (+)- und (- )gekennzeichnet, d.h. solche Isomere, die planpolarisiertes Licht im Uhrzeigersinn ((+)-Richtung) drehen, werden mit "(+)" bezeichnet; (b) entsprechend der Cahn-Ingold-Prelog Regeln hinsichtlich ihrer Konfiguration an ihren Chiralitätszentren in R- und S-Konfiguration eingeteilt oder (c) entsprechend ihrer Retentionszeit bei der Trennung mittels chiraler Chromatographie mit "Ent-1" und "Ent-2" gekennzeichnet. Verfahren zur Trennung von Enantiomeren sind im Stand der Technik gut bekannt, z.B. die Trennung mittels chiraler Chromatographie.

Sofern die Verbindungen als Feststoffe erhalten werden, kann die Reinigung auch durch Umkristallisieren oder Digerieren erfolgen. Einzelne Verbindungen (I) können auch durch Derivatisierung anderer Verbindungen (I) hergestellt werden.

Als Isolierungs-, Reinigungs- und Stereoisomerenauftrennungsverfahren von Verbindungen der Formel (I) kommen Methoden in Frage, die dem Fachmann aus analogen Fällen allgemein bekannt sind, z.B. durch physikalische Verfahren wie Kristallisation, Chromatographieverfahren, vor allem Säulenchromatographie und HPLC (Hochdruckflüssigchromatographie), Destillation, gegebenenfalls unter reduziertem Druck, Extraktion und andere Verfahren, können gegebenenfalls verbleibende Gemische in der Regel durch chromatographische Trennung, z.B. an chiralen Festphasen, getrennt werden. Für präparative Mengen oder im industriellen Maßstab kommen Verfahren in Frage wie Kristallisation, z.B. diastereomerer Salze, die aus den Stereoisomerengemischen mit optisch aktiven Säuren und gegebenenfalls bei vorhandenen sauren Gruppen mit optisch aktiven Basen erhalten werden können.

Im Folgenden werden die erfindungsgemäß verwendeten Verbindungen der

Formel (I) und ihre Salze "Verbindungen der allgemeinen Formel (I)" bezeichnet.

Bevorzugter Erfindungsgegenstand sind Verbindungen der allgemeinen Formel (I), worin
- Q: für die Gruppen 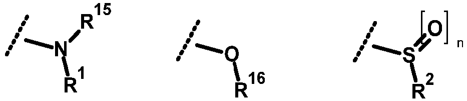 steht, wobei die gestrichelte Linie jeweils die Verknüpfungsstelle zum restlichen Teil der Formel (I) kennzeichnet,
- R¹ und R¹⁶: unabhängig für Wasserstoff, (C₁-C₆)-Alkyl, (C₃-C₆)-Cycloalkyl-(C₁-C₆)-alkyl, (C₁-C₆)-Cyanoalkyl, (C₂-C₆)-Alkenyl, (C₂-C₆)-Alkinyl, R¹³O-(C₁-C₆)-alkyl stehen,
- R²: für (C₁-C₆)-Alkyl, (C₁-C₆)-Haloalkyl, (C₃-C₆)-Cycloalkyl, (C₃-C₆)-Halocycloalkyl, (C₃-C₆)-Cycloalkyl-(C₁-C₆)-alkyl, (C₂-C₆)-Alkenyl, Heterocyclyl, oder für Aryl, Aryl-(C₁-C₆)-alkyl steht, wobei die Arylgruppe gegebenenfalls weiter 1 bis 5 Substituenten trägt, die unabhängig voneinander für Halogen, Cyano, Nitro, Trimethylsilyl, Pentafluorsulfanyl, -C(=S)NH₂, OR¹³, NR¹¹R¹², SR¹⁴, S(O)R¹⁴, SO₂R¹⁴, C(O)OR¹³, C(O)R¹³, (C₁-C₆)-Alkyl, (C₃-C₆)-Cycloalkyl, (C₃-C₆)-Cycloalkyl-(C₁-C₆)-alkyl, (C₂-C₆)-Alkenyl, (C₂-C₆)-Haloalkenyl, (C₂-C₆)-Alkinyl, (C₂-C₆)-Haloalkinyl, (C₃-C₆)-Cycanocycloalkyl, (C₃-C₆)-Halocycloalkyl, (C₁-C₆)-Haloalkyl, (C₁-C₆)-Cyanoalkyl stehen, oder für Heteroaryl, Heteroaryl-(C₁-C₆)-alkyl, Heterocyclyl-(C₁-C₆)-alkyl steht und wobei Heterocyclyl n Oxogruppen trägt,
- R³: für (C₁-C₆)-Alkyl, (C₁-C₆)-Haloalkyl, (C₃-C₆)-Cycloalkyl, (C₂-C₆)-Alkenyl, R¹³O-(C₁-C₆)-alkyl, (C₃-C₆)-Cycloalkyl-(C₁-C₆)-alkyl steht,
- R⁴, R⁵, R⁶: unabhängig voneinander für Wasserstoff, (C₁-C₆)-Alkyl, (C₁-C₆)-Haloalkyl stehen,
- R⁷, R⁸, R¹⁰: unabhängig voneinander für Wasserstoff, Halogen, OR¹³, (C₁-C₆)-Alkyl, (C₁-C₆)-Haloalkyl stehen,
- R⁹: für Halogen, Nitro, Cyano, (C₁-C₆)-Alkyl, R¹³O-(C₁-C₆)-alkyl, R¹⁴S-(C₁-C₆)-alkyl, (C₁-C₆)-Haloalkyl, (C₃-C₆)-Cycloalkyl, (C₃-C₆)-Halocycloalkyl, (C₂-C₆)-Alkenyl, (C₂-C₆)-Haloalkenyl, (C₄-C₆)-Cycloalkenyl, (C₂-C₆)-Alkinyl, (C₂-C₆)-Haloalkinyl, Heterocyclyl, OR¹³, NR¹¹R¹², SR¹⁴, S(O)R¹⁴, SO₂R¹⁴, C(O)OR¹³, C(O)R¹³ steht,
- R¹¹ und R¹²: gleich oder verschieden sind und unabhängig voneinander für Wasserstoff, (C₁-C₆)-Alkyl, (C₃-C₆)-Alkenyl, (C₃-C₆)-Alkinyl, (C₁-C₆)-Cyanoalkyl, (C₁-C₆)-Haloalkyl, (C₃-C₆)-Haloalkenyl, (C₃-C₆)-Haloalkinyl, (C₃-C₆)-Cycloalkyl, (C₃-C₆)-Halocycloalkyl, (C₄-C₆)-Cycloalkenyl, (C₄-C₆)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₄-C₆)-Cycloalkenyl-(C₁-C₆)-alkyl, COR¹³, SO₂R¹⁴, Heterocyclyl, (C₁-C₆)-Alkoxycarbonyl, Bis-[(C₁-C₆)-alkyl]aminocarbonyl-(C₁-C₆)-alkyl, (C₁-C₆)-Alkyl-aminocarbonyl-(C₁-C₆)-alkyl, Aryl-(C₁-C₆)-alkyl-aminocarbonyl-(C₁-C₆)-alkyl, Aryl-(C₁-C₆)-alkoxycarbonyl, Heteroaryl-(C₁-C₆)-alkoxycarbonyl, (C₃-C₆)-Alkenyloxycarbonyl, (C₃-C₆)-Alkinyloxycarbonyl, Heterocyclyl-(C₁-C₆)-alkyl stehen, wobei Heterocyclyl n Oxogruppen trägt, oder
- R¹¹ und R¹²: mit dem Stickstoffatom, an das sie gebunden sind, einen vollständig gesättigten oder teilgesättigten, gegebenenfalls durch weitere Heteroatome unterbrochenen und gegebenenfalls weiter substituierten 3 bis 6-gliedrigen monocyclischen oder bicyclischen Ring bilden,
- R¹³: für Wasserstoff, (C₁-C₆)-Alkyl, (C₃-C₆)-Alkenyl, (C₃-C₆)-Alkinyl, (C₁-C₆)-Cyanoalkyl, (C₁-C₆)-Haloalkyl, (C₃-C₆)-Haloalkenyl, (C₃-C₆)-Haloalkinyl, (C₃-C₆)-Cycloalkyl, (C₄-C₆)-Cycloalkenyl, (C₄-C₆)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkoxy-(C₁-C₆)-haloalkyl, (C₁-C₆)-Alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkoxy-(C₁-C₆)-alkyl, Aryl, Aryl-(C₁-C₆)-alkyl, Aryl-(C₁-C₆)-alkoxy-(C₁-C₆)-alkyl, Heteroaryl, Heteroaryl-(C₁-C₆)-alkyl, (C₃-C₆)-Cycloalkyl-(C₁-C₆)-alkyl, (C₄-C₆)-Cycloalkenyl-(C₁-C₆)-alkyl, Bis-[(C₁-C₆)-alkyl]aminocarbonyl-(C₁-C₆)-alkyl, (C₁-C₆)-Alkylaminocarbonyl-(C₁-C₆)-alkyl, Aryl-(C₁-C₆)-alkyl-aminocarbonyl-(C₁-C₆)-alkyl, Bis-[(C₁-C₆)-alkyl]amino-(C₂-C₆)-alkyl, (C₁-C₆)-Alkyl-amino-(C₂C₆)-alkyl, Aryl-(C₁-C₆)-alkylamino-(C₂-C₆)-alkyl, R¹⁴S-(C₁-C₆)-alkyl, R¹⁴(O)S-(C₁-C₆)-alkyl, R¹⁴O₂S-(C₁-C₆)-alkyl, Hydroxycarbonyl-(C₁-C₆)-alkyl, Heterocyclyl, Heterocyclyl-(C₁-C₆)-alkyl, Tris-[(C₁-C₆)-Alkyl]silyl-(C₁-C₆)-alkyl, Bis-[(C₁-C₆)-Alkyl](aryl)silyl(C₁-C₆)-alkyl, [(C₁-C₆)-Alkyl]-bis-(aryl)silyl-(C₁-C₆)-alkyl, (C₁-C₆)-Alkylcarbonyloxy-(C₁-C₆)-alkyl, (C₃-C₆)-Cycloalkylcarbonyloxy-(C₁-C₆)-alkyl, Arylcarbonyloxy-(C₁-C₆)-alkyl, Heteroarylcarbonyloxy-(C₁-C₆)-alkyl, Heterocyclylcarbonyloxy-(C₁-C₆)-alkyl, Aryloxy-(C₁-C₆)-alkyl, Heteroaryloxy-(C₁-C₆)-alkyl, (C₁-C₆)-Alkoxycarbonyl steht, wobei Heterocyclyl n Oxogruppen trägt und wobei Cycloalkyl substituiert sein kann durch einen oder mehrere Substituenten ausgewählt aus einer Gruppe bestehend aus (C₁-C₆)-Alkyl, (C₁-C₆)-Haloalkyl, Halogen, Cyano, Carbamoyl und (C₁-C₆)-Alkylsulfonyl,
- R¹⁴: für (C₁-C₆)-Alkyl, (C₃-C₆)-Alkenyl, (C₃-C₆)-Alkinyl, (C₁-C₆)-Cyanoalkyl, (C₁-C₆)-Haloalkyl, (C₃-C₆)-Haloalkenyl, (C₃-C₆)-Haloalkinyl, (C₃-C₆)-Cycloalkyl, (C₄-C₆)-Cycloalkenyl, (C₄-C₆)-Halocycloalkenyl, (C₁-C₆)-Alkoxy-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, Heterocyclyl-(C₁-C₆)-alkyl, (C₃-C₆)-Cycloalkyl-(C₁-C₆)-alkyl, (C₄-C₆)-Cycloalkenyl-(C₁-C₆)-alkyl, Amino, Bis-[(C₁-C₆)-alkyl]amino, (C₁-C₆)-Alkyl-amino, Aryl-(C₁-C₆)-amino, Aryl-(C₁-C₆)-alkyl-amino, Aryl-[(C₁-C₆)-alkyl]amino, (C₃-C₆)-Cycloalkyl-amino, (C₃-C₆)-Cycloalkyl-[(C₁-C₆)-alkyl]amino, N-Azetidinyl, N-Pyrrolidinyl, N-Piperidinyl, N-Morpholinyl steht,
wobei die zwölf letztgenannten Reste (Amino, Bis-[(C₁-C₆)-alkyl]amino, (C₁-C₆)-Alkylamino, Aryl-(C₁-C₆)-amino, Aryl-(C₁-C₆)-alkyl-amino, Aryl-[(C₁-C₆)-alkyl]amino, (C₃-C₆)-Cycloalkyl-amino, (C₃-C₆)-Cycloalkyl-[(C₁-C₆)-alkyl]amino, N-Azetidinyl, N-Pyrrolidinyl, N-Piperidinyl, N-Morpholinyl) nur an Sulfonylgruppen gebunden sind,
und wobei Heterocyclyl n Oxogruppen trägt,
und wobei Cycloalkyl substituiert sein kann durch einen oder mehrere Substituenten ausgewählt aus einer Gruppe bestehend aus (C₁-C₆)-Alkyl, (C₁-C₆)-Haloalkyl, Halogen, Cyano, Carbamoyl und (C₁-C₆)-Alkylsulfonyl,
- R¹⁵: ür Wasserstoff, (C₁-C₆)-Alkyl, (C₃-C₆)-Cycloalkyl-(C₁-C₆)-alkyl, (C₃-C₆)-Alkenyl, (C₃-C₆)-Alkinyl steht und
- n: für 0, 1, 2 steht.

Besonders bevorzugter Erfindungsgegenstand sind Verbindungen der allgemeinen Formel (I), worin
- Q: für die Gruppen 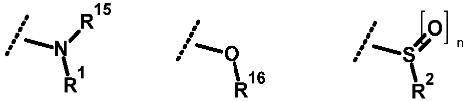 steht, wobei die gestrichelte Linie jeweils die Verknüpfungsstelle zum restlichen Teil der Formel (I) kennzeichnet,
- R¹ und R¹⁶: unabhängig für Wasserstoff, (C₁-C₅)-Alkyl, (C₃-C₆)-Cycloalkyl-(C₁-C₄)-alkyl, (C₁-C₄)-Cyanoalkyl, (C₂-C₆)-Alkenyl, (C₂-C₆)-Alkinyl, R¹³O-(C₁-C₄)-alkyl stehen,
- R²: für (C₁-C₅)-Alkyl, (C₁-C₅)-Haloalkyl, (C₃-C₆)-Cycloalkyl, (C₃-C₆)-Cycloalkyl-(C₁-C₆)-alkyl, (C₂-C₆)-Alkenyl, Heterocyclyl, oder für Aryl, Aryl-(C₁-C₆)-alkyl steht, wobei die Arylgruppe gegebenenfalls weiter 1 bis 5 Substituenten trägt, die unabhängig voneinander für Halogen, Cyano, Nitro, Trimethylsilyl, Pentafluorsulfanyl, -C(=S)NH₂, OR¹³, NR¹¹R¹², SR¹⁴, S(O)R¹⁴, SO₂R¹⁴, C(O)OR¹³, C(O)R¹³, (C₁-C₆)-Alkyl, (C₃-C₆)-Cycloalkyl, (C₃-C₆)-Cycloalkyl-(C₁-C₆)-alkyl, (C₂-C₆)-Alkenyl, (C₂-C₆)-Haloalkenyl, (C₂-C₆)-Alkinyl, (C₂-C₆)-Haloalkinyl, (C₃-C₆)-Cycanocycloalkyl, (C₃-C₆)-Halocycloalkyl, (C₁-C₆)-Haloalkyl, (C₁-C₆)-Cyanoalkyl stehen, oder für Heteroaryl, Heteroaryl-(C₁-C₆)-alkyl, Heterocyclyl-(C₁-C₆)-alkyl steht und wobei Heterocyclyl n Oxogruppen trägt,
- R³: für (C₁-C₆)-Alkyl, (C₁-C₆)-Haloalkyl, (C₂-C₆)-Alkenyl, R¹³O-(C₁-C₆)-alkyl steht,
- R⁴, R⁵, R⁶: unabhängig voneinander für Wasserstoff, (C₁-C₆)-Alkyl stehen,
- R⁷, R⁸, R¹⁰: unabhängig voneinander für Wasserstoff, Halogen, (C₁-C₆)-Alkyl, OR¹³ stehen,
- R⁹: für Halogen, Nitro, Cyano, (C₁-C₆)-Alkyl, R¹³O-(C₁-C₆)-alkyl, (C₁-C₆)-Haloalkyl, (C₃-C₆)-Cycloalkyl, (C₂-C₆)-Alkenyl, (C₂-C₆)-Alkinyl, Heterocyclyl, OR¹³ steht,
- R¹¹ und R¹²: gleich oder verschieden sind und unabhängig voneinander für Wasserstoff, (C₁-C₆)-Alkyl, (C₃-C₆)-Alkenyl, (C₃-C₆)-Alkinyl, (C₁-C₆)-Cyanoalkyl, (C₁-C₆)-Haloalkyl, (C₃-C₆)-Haloalkenyl, (C₃-C₆)-Haloalkinyl, (C₃-C₆)-Cycloalkyl, (C₃-C₆)-Halocycloalkyl, (C₄-C₆)-Cycloalkenyl, (C₄-C₆)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₄-C₆)-Cycloalkenyl-(C₁-C₆)-alkyl, COR¹³, SO₂R¹⁴, Heterocyclyl, (C₁-C₆)-Alkoxycarbonyl, Bis-[(C₁-C₆)-alkyl]aminocarbonyl-(C₁-C₆)-alkyl, (C₁-C₆)-Alkyl-aminocarbonyl-(C₁-C₆)-alkyl, Aryl-(C₁-C₆)-alkyl-aminocarbonyl-(C₁-C₆)-alkyl, Aryl-(C₁-C₆)-alkoxycarbonyl, Heteroaryl-(C₁-C₆)-alkoxycarbonyl, (C₃-C₆)-Alkenyloxycarbonyl, (C₃-C₆)-Alkinyloxycarbonyl, Heterocyclyl-(C₁-C₆)-alkyl stehen, wobei Heterocyclyl n Oxogruppen trägt, oder
- R¹¹ und R¹²: mit dem Stickstoffatom, an das sie gebunden sind, einen vollständig gesättigten oder teilgesättigten, gegebenenfalls durch weitere Heteroatome unterbrochenen und gegebenenfalls weiter substituierten 3 bis 6-gliedrigen monocyclischen oder bicyclischen Ring bilden,
- R¹³: für Wasserstoff, (C₁-C₆)-Alkyl, (C₃-C₆)-Alkenyl, (C₃-C₆)-Alkinyl, (C₁-C₆)-Cyanoalkyl, (C₁-C₆)-Haloalkyl, (C₃-C₆)-Haloalkenyl, (C₃-C₆)-Haloalkinyl, (C₃-C₆)-Cycloalkyl, (C₄-C₆)-Cycloalkenyl, (C₄-C₆)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkoxy-(C₁-C₆)-haloalkyl, (C₁-C₆)-Alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkoxy-(C₁-C₆)-alkyl, Aryl, Aryl-(C₁-C₆)-alkyl, Aryl-(C₁-C₆)-alkoxy-(C₁-C₆)-alkyl, Heteroaryl, Heteroaryl-(C₁-C₆)-alkyl, (C₃-C₆)-Cycloalkyl-(C₁-C₆)-alkyl, (C₁-C₆)-Cycloalkenyl-(C₁-C₆)-alkyl, Bis-[ (C₁-C₆)-alkyl]aminocarbonyl-(C₁-C₆)-alkyl, (C₁-C₆)-Alkylaminocarbonyl-(C₁-C₆)-alkyl, Aryl-(C₁-C₆)-alkyl-aminocarbonyl-(C₁-C₆)-alkyl, Bis-[(C₁-C₆)-alkyl]amino-(C₂-C₆)-alkyl, (C₁-C₆)-Alkyl-amino-(C₂C₆)-alkyl, Aryl-(C₁-C₆)-alkylamino-(C₂-C₆)-alkyl, R¹⁴S-(C₁-C₆)-alkyl, R¹⁴(O)S-(C₁-C₆)-alkyl, R¹⁴O₂S-(C₁-C₆)-alkyl, Hydroxycarbonyl-(C₁-C₆)-alkyl, Heterocyclyl, Heterocyclyl-(C₁-C₆)-alkyl, Tris-[(C₁-C₆)-Alkyl]silyl-(C₁-C₆)-alkyl, Bis-[(C₁-C₆)-Alkyl](aryl)silyl(C₁-C₆)-alkyl, [(C₁-C₆)-Alkyl]-bis-(aryl)silyl-(C₁-C₆)-alkyl, (C₁-C₆)-Alkylcarbonyloxy-(C₁-C₆)-alkyl, (C₃-C₆)-Cycloalkylcarbonyloxy-(C₁-C₆)-alkyl, Arylcarbonyloxy-(C₁-C₆)-alkyl, Heteroarylcarbonyloxy-(C₁-C₆)-alkyl, Heterocyclylcarbonyloxy-(C₁-C₆)-alkyl, Aryloxy-(C₁-C₆)-alkyl, Heteroaryloxy-(C₁-C₆)-alkyl, (C₁-C₆)-Alkoxycarbonyl steht, wobei Heterocyclyl n Oxogruppen trägt und wobei Cycloalkyl substituiert sein kann durch einen oder mehrere Substituenten ausgewählt aus einer Gruppe bestehend aus (C₁-C₆)-Alkyl, (C₁-C₆)-Haloalkyl, Halogen, Cyano, Carbamoyl und (C₁-C₆)-Alkylsulfonyl,
- R¹⁴: für (C₁-C₆)-Alkyl, (C₃-C₆)-Alkenyl, (C₃-C₆)-Alkinyl, (C₁-C₆)-Cyanoalkyl, (C₁-C₆)-Haloalkyl, (C₃-C₆)-Haloalkenyl, (C₃-C₆)-Haloalkinyl, (C₃-C₆)-Cycloalkyl, (C₄-C₆)-Cycloalkenyl, (C₄-C₆)-Halocycloalkenyl, (C₁-C₆)-Alkoxy-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, Heterocyclyl-(C₁-C₆)-alkyl, (C₃-C₆)-Cycloalkyl-(C₁-C₆)-alkyl, (C₄-C₆)-Cycloalkenyl-(C₁-C₆)-alkyl, Amino, Bis-[(C₁-C₆)-alkyl]amino, (C₁-C₆)-Alkyl-amino, Aryl-(C₁-C₆)-amino, Aryl-(C₁-C₆)-alkyl-amino, Aryl-[(C₁-C₆)-alkyl]amino, (C₃-C₆)-Cycloalkyl-amino, (C₃-C₆)-Cycloalkyl-[(C₁-C₆)-alkyl]amino, N-Azetidinyl, N-Pyrrolidinyl, N-Piperidinyl, N-Morpholinyl steht,
wobei die zwölf letztgenannten Reste (Amino, Bis-[(C₁-C₆)-alkyl]amino, (C₁-C₆)-Alkylamino, Aryl-(C₁-C₆)-amino, Aryl-(C₁-C₆)-alkyl-amino, Aryl-[(C₁-C₆)-alkyl]amino, (C₃-C₆)-Cycloalkyl-amino, (C₃-C₆)-Cycloalkyl-[(C₁-C₆)-alkyl]amino, N-Azetidinyl, N-Pyrrolidinyl, N-Piperidinyl, N-Morpholinyl) nur an Sulfonylgruppen gebunden sind,
und wobei Heterocyclyl n Oxogruppen trägt,
und wobei Cycloalkyl substituiert sein kann durch einen oder mehrere Substituenten ausgewählt aus einer Gruppe bestehend aus (C₁-C₆)-Alkyl, (C₁-C₆)-Haloalkyl, Halogen, Cyano, Carbamoyl und (C₁-C₆)-Alkylsulfonyl,
- R¹⁵: für Wasserstoff, (C₁-C₅)-Alkyl, (C₃-C₆)-Cycloalkyl-(C₁-C₄)-alkyl steht und
- n: für 0, 1, 2 steht.

Ganz besonders bevorzugter Erfindungsgegenstand sind Verbindungen der allgemeinen Formel (I), worin
- R³: für Methyl, Ethyl, Prop-1-yl, 1-Methylethyl, But-1-yl, 1-Methylprop-1-yl, 2-Methylprop-1-yl, 1,1-Dimethylethyl, Pent-1-yl, Vinyl, Prop-2-en-1-yl, Trifluormethyl, Methoxymethyl, Methoxyethyl, Ethoxyethyl steht,
- R⁴, R⁵, R⁶: unabhängig voneinander für Wasserstoff, Methyl, Ethyl stehen,
- R⁷, R⁸, R¹⁰: unabhängig voneinander für Wasserstoff, Fluor, Chlor, Brom, Methyl, Methoxy stehen,
- R⁹: für Fluor, Chlor, Brom, Iod, Nitro, Cyano, Methyl, Ethyl, Prop-1-yl, 1-Methylethyl, But-1-yl, 1-Methylprop-1-yl, 2-Methylprop-1-yl, 1,1-Dimethylethyl, Pent-1-yl, 1-Methylbut-1-yl, 2-Methylbut-1-yl, 3-Methylbut-1-yl, 1,1-Dimethylprop-1-yl, 1,2-Dimethylprop-1-yl, 2,2-Dimethylprop-1-yl, 1-Ethylprop-1-yl, Hex-1-yl, 1-Methylpent-1-yl, 2-Methylpent-1-yl, 3-Methylpent-1-yl, 4-Methylpent-1-yl, 1-Ethylbut-1-yl, 2-Ethylbut-1-yl, 1,2-Dimethylbut-1-yl, 1,3-Dimethylbut-1-yl, 2,2-Dimethylbut-1-yl, 3,3-Dimethylbut-1-yl, Cyclopropyl, Cyclobutyl, Cyclopentyl, Cyclohexyl, Methoxymethyl, Ethoxymethyl, Methoxyethyl, Ethoxyethyl, Vinyl, Prop-1-en-1-yl, Prop-2-en-1-yl, But-1-en-1-yl, But-2-en-1-yl, But-3-en-1-yl, Pent-1-en-1-yl, Pent-2-en-1-yl, Pent-3-en-1-yl, Pent-4-en-1-yl, 1-Methylprop-2-en-1-yl, 2-Methylprop-2-en-1-yl, 1-Methylbut-2-en-1-yl, 2-Methylbut-2-en-1-yl, 3-Methylbut-2-en-1-yl, 1-Methylbut-3-en-1-yl, 2-Methylbut-3-en-1-yl, 3-Methylbut-3-en-1-yl, 1,1-Dimethylprop-2-en-1-yl, 1,2-Dimethylprop-2-en-1-yl, Methoxy, Ethoxy, Prop-2-yloxy, Trifluormethoxy, Difluormethoxy, Trifluormethyl, Difluormethyl, Ethinyl, Prop-1-in-yl, Azetidin-1-yl, Pyrrolidin-1-yl, Piperidin-1-yl steht und
- Q: für einen Rest der nachfolgenden Formeln Q-1 bis Q-55 in der Tabelle 1 steht, wobei die gestrichelte Linie jeweils die Verknüpfungsstelle zum restlichen Teil der Formel (I) kennzeichnet,

**Tabelle 1**

| | | | | |
|---|---|---|---|---|
| 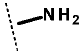 | 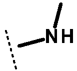 | 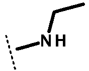 | 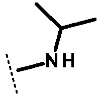 | 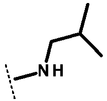 |
| Q-1 | Q-2 | Q-3 | Q-4 | Q-5 |
| | | | | |
| 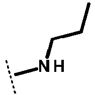 | 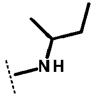 | 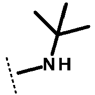 | 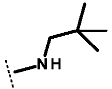 | 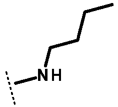 |
| Q-6 | Q-7 | Q-8 | Q-9 | Q-10 |
| | | | | |
| 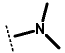 | 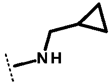 | 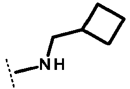 | 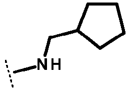 | 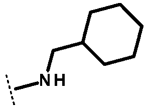 |
| Q-11 | Q-12 | Q-13 | Q-14 | Q-15 |
| | | | | |
| 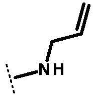 | 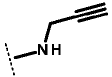 | 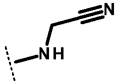 | 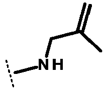 | 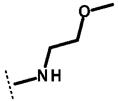 |
| Q-16 | Q-17 | Q-18 | Q-19 | Q-20 |
| | | | | |
| 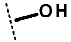 | 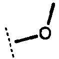 | 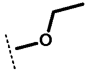 | 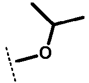 | 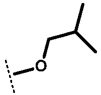 |
| Q-21 | Q-22 | Q-23 | Q-24 | Q-25 |
| | | | | |
| 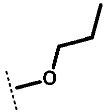 | 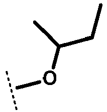 | 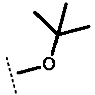 | 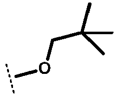 | 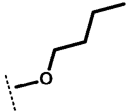 |
| Q-26 | Q-27 | Q-28 | Q-29 | Q-30 |
| 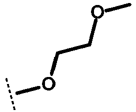 | 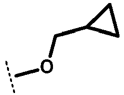 | 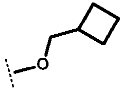 | 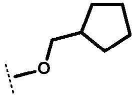 | 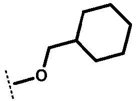 |
| Q-31 | Q-32 | Q-33 | Q-34 | Q-35 |
| | | | | |
| 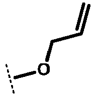 | 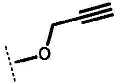 | 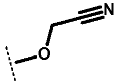 | 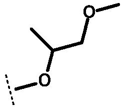 | 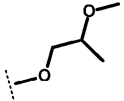 |
| Q-36 | Q-37 | Q-38 | Q-39 | Q-40 |
| | | | | |
| 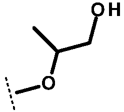 | 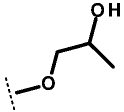 | 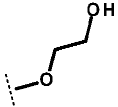 | 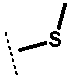 | 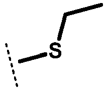 |
| Q-41 | Q-42 | Q-43 | Q-44 | Q-45 |
| | | | | |
| 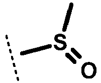 | 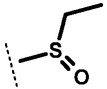 | 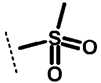 | 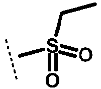 | 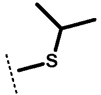 |
| Q-46 | Q-47 | Q-48 | Q-49 | Q-50 |
| | | | | |
| 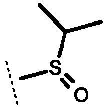 | 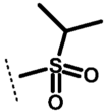 | 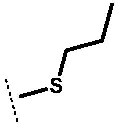 | 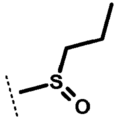 | 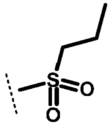 |
| Q-51 | Q-52 | Q-53 | Q-54 | Q-55 |

Bevorzugt steht Q für einen Rest der oben aufgeführten Formeln Q-1, Q-22, Q-44, Q-45, Q-23, Q-24, Q-39, Q-42 oder Q-21, mehr bevorzugt für einen Rest der oben aufgeführten Formeln Q-1, Q-22, Q-44, Q-45, Q-23, Q-24, Q-39 oder Q-42, besonders bevorzugt für einen Rest der oben aufgeführten Formeln Q-1, Q-22, Q-44, Q-45, Q-23 oder Q-24, insbesondere bevorzugt für einen Rest der oben aufgeführten Formeln Q-1, Q-22, Q-44 oder Q-45.

Im Speziellen bevorzugter Erfindungsgegenstand sind Verbindungen der allgemeinen Formel (I) und/oder deren Salze, worin
- R³: für Methyl, Ethyl, steht,
- R⁴, R⁵, R⁶, R⁸, R¹⁰: für Wasserstoff steht,
- R⁷: für Wasserstoff, Fluor, Chlor, Methyl, Methoxy steht
- R⁹: für Fluor, Chlor, Brom, Iod, Methyl, Ethyl, 1-Methylethyl, 1,1-Dimethylethyl, Cyclopropyl, Vinyl, Methoxy, Ethoxy, Trifluormethoxy, Difluormethoxy, Trifluormethyl, Difluormethyl, Ethinyl steht und
- Q: für einen Rest der oben aufgeführten Formeln Q-1 bis Q-55 steht, bevorzugt für einen Rest der Formeln Q-1, Q-22, Q-44, Q-45, Q-23, Q-24, Q-39, Q-42 oder Q21 steht, mehr bevorzugt für einen Rest der oben aufgeführten Formeln Q-1, Q-22, Q-44, Q-45, Q-23, Q-24, Q-39 oder Q-42, besonders bevorzugt für einen Rest der oben aufgeführten Formeln Q-1, Q-22, Q-44, Q-45, Q-23 oder Q-24, insbesondere bevorzugt für einen Rest der oben aufgeführten Formeln Q-1, Q-22, Q-44 oder Q-45.

Ein weiter bevorzugter Erfindungsgegenstand betrifft Verbindungen der allgemeinen Formel (I) und/oder deren Salze, worin
- R³: für Methyl steht,
- R⁴, R⁵, R⁶, R⁸, R¹⁰: für Wasserstoff steht,
- R⁷: für Wasserstoff, Methyl steht
- R⁹: für Methyl steht und
- Q: für einen Rest der oben aufgeführten Formeln Q-1 bis Q-55 steht, bevorzugt für einen Rest der Formeln Q-1, Q-22, Q-44, Q-45, Q-23, Q-24, Q-39, Q-42 oder Q-21 steht, mehr bevorzugt für einen Rest der oben aufgeführten Formeln Q-1, Q-22, Q-44, Q-45, Q-23, Q-24, Q-39 oder Q-42, besonders bevorzugt für einen Rest der oben aufgeführten Formeln Q-1, Q-22, Q-44, Q-45, Q-23 oder Q-24, insbesondere bevorzugt für einen Rest der oben aufgeführten Formeln Q-1, Q-22, Q-44 oder Q-45..

Im ganz Speziellen bevorzugter Erfindungsgegenstand sind die folgenden Verbindungen der allgemeinen Formel (I):
N²-(2,6-Dimethyl-2,3-dihydro-1H-inden-1-yl)-1,3,5-triazin-2,4,6-triamin,
N²-[(1R,2S)-2,6-Dimethyl-2,3-dihydro-1H-inden-1-yl]-1,3,5-triazin-2,4,6-triamin,
N²-[(1S,2R)-2,6-Dimethyl-2,3-dihydro-1H-inden-1-yl]-1,3,5-triazin-2,4,6-triamin,
N²-(2,6-Dimethyl-2,3-dihydro-1H-inden-1-yl)-N⁴-methyl-1,3,5-triazin-2,4,6-triamin,
N²-[(1R,2S)-2,6-Dimethyl-2,3-dihydro-1H-inden-1-yl]-N⁴-methyl-1,3,5-triazin-2,4,6-triamin,
N²-[(1S,2R)-2,6-Dimethyl-2,3-dihydro-1H-inden-1-yl]-N⁴-methyl-1,3,5-triazin-2,4,6-triamin,
N²-(2,6-Dimethyl-2,3-dihydro-1H-inden-1-yl)-N⁴-ethyl-1,3,5-triazin-2,4,6-triamin,
N²-[(1R,2S)-2,6-Dimethyl-2,3-dihydro-1H-inden-1-yl]-N⁴-ethyl-1,3,5-triazin-2,4,6-triamin,
N²-[(1S,2R)-2,6-Dimethyl-2,3-dihydro-1H-inden-1-yl]-N⁴-ethyl-1,3,5-triazin-2,4,6-triamin,
N⁴-(2,6-Dimethyl-2,3-dihydro-1H-inden-1-yl)-N²,N²-dimethyl-1,3,5-triazin-2,4,6-triamin,
N⁴-[(1R,2S)-2,6-Dimethyl-2,3-dihydro-1H-inden-1-yl]-N²,N²-dimethyl-1,3,5-triazin-2,4,6-triamin,
N⁴-[(1S,2R)-2,6-Dimethyl-2,3-dihydro-1H-inden-1-yl]-N²,N²-dimethyl-1,3,5-triazin-2,4,6-triamin,
N-(2,6-Dimethyl-2,3-dihydro-1H-inden-1-yl)-6-hydroxy-1,3,5-triazin-2,4-diamin,
N-[(1R,2S)-2,6-Dimethyl-2,3-dihydro-1H-inden-1-yl]-6-hydroxy-1,3,5-triazin-2,4-diamin,
N-[(1S,2R)-2,6-Dimethyl-2,3-dihydro-1H-inden-1-yl]-6-hydroxy-1,3,5-triazin-2,4-diamin,
N-(2,6-Dimethyl-2,3-dihydro-1H-inden-1-yl)-6-methoxy-1,3,5-triazin-2,4-diamin,
N-[(1R,2S)-2,6-Dimethyl-2,3-dihydro-1H-inden-1-yl]-6-methoxy-1,3,5-triazin-2,4-diamin,
N-[(1S,2R)-2,6-Dimethyl-2,3-dihydro-1H-inden-1-yl]-6-methoxy-1,3,5-triazin-2,4-diamin,
N-(2,6-Dimethyl-2,3-dihydro-1H-inden-1-yl)-6-ethoxy-1,3,5-triazin-2,4-diamin,
N-[(1R,2S)-2,6-Dimethyl-2,3-dihydro-1H-inden-1-yl]-6-ethoxy-1,3,5-triazin-2,4-diamin,
N-[(1S,2R)-2,6-Dimethyl-2,3-dihydro-1H-inden-1-yl]-6-ethoxy-1,3,5-triazin-2,4-diamin,
N-(2,6-Dimethyl-2,3-dihydro-1H-inden-1-yl)-6-(prop-1-yloxy)-1,3,5-triazin-2,4-diamin,
N-[(1R,2S)-2,6-Dimethyl-2,3-dihydro-1H-inden-1-yl]-6-(prop-1-yloxy)-1,3,5-triazin-2,4-diamin,
N-[(1S,2R)-2,6-Dimethyl-2,3-dihydro-1H-inden-1-yl]-6-(prop-1-yloxy)-1,3,5-triazin-2,4-diamin,
N-(2,6-Dimethyl-2,3-dihydro-1H-inden-1-yl)-6-(prop-2-yloxy)-1,3,5-triazin-2,4-diamin,
N-[(1R,2S)-2,6-Dimethyl-2,3-dihydro-1H-inden-1-yl]-6-(prop-2-yloxy)-1,3,5-triazin-2,4-diamin,
N-[(1S,2R)-2,6-Dimethyl-2,3-dihydro-1H-inden-1-yl]-6-(prop-2-yloxy)-1,3,5-triazin-2,4-diamin,
N-(2,6-Dimethyl-2,3-dihydro-1H-inden-1-yl)-6-(but-1-yloxy)-1,3,5-triazin-2,4-diamin,
N-[(1R,2S)-2,6-Dimethyl-2,3-dihydro-1H-inden-1-yl]-6-(but-1-yloxy)-1,3,5-triazin-2,4-diamin,
N-[(1S,2R)-2,6-Dimethyl-2,3-dihydro-1H-inden-1-yl]-6-(but-1-yloxy)-1,3,5-triazin-2,4-diamin,
N-(2,6-Dimethyl-2,3-dihydro-1H-inden-1-yl)-6-(prop-2-en-1-yloxy)-1,3,5-triazin-2,4-diamin,
N-[(1R,2S)-2,6-Dimethyl-2,3-dihydro-1H-inden-1-yl]-6-(prop-2-en-1-yloxy)-1,3,5-triazin-2,4-diamin,
N-[(1S,2R)-2,6-Dimethyl-2,3-dihydro-1H-inden-1-yl]-6-(prop-2-en-1-yloxy)-1,3,5-triazin-2,4-diamin,
N-(2,6-Dimethyl-2,3-dihydro-1H-inden-1-yl)-6-(prop-2-in-1-yloxy)-1,3,5-triazin-2,4-diamin,
N-[(1R,2S)-2,6-Dimethyl-2,3-dihydro-1H-inden-1-yl]-6-(prop-2-in-1-yloxy)-1,3,5-triazin-2,4-diamin,
N-[(1S,2R)-2,6-Dimethyl-2,3-dihydro-1H-inden-1-yl]-6-(prop-2-in-1-yloxy)-1,3,5-triazin-2,4-diamin,
N-(2,6-Dimethyl-2,3-dihydro-1H-inden-1-yl)-6-(2-methoxyethoxy)-1,3,5-triazin-2,4-diamin,
N-[(1R,2S)-2,6-Dimethyl-2,3-dihydro-1H-inden-1-yl]-6-(2-methoxyethoxy)-1,3,5-triazin-2,4-diamin,
N-[(1S,2R)-2,6-Dimethyl-2,3-dihydro-1H-inden-1-yl]-6-(2-methoxyethoxy)-1,3,5-triazin-2,4-diamin,
N-(2,6-Dimethyl-2,3-dihydro-1H-inden-1-yl)-6-[(1-methoxypropan-2-yl)oxy]-1,3,5-triazin-2,4-diamin,
N-[(1R,2S)-2,6-Dimethyl-2,3-dihydro-1H-inden-1-yl]-6-[(1-methoxypropan-2-yl)oxy]-1,3,5-triazin-2,4-diamin,
N-[(1S,2R)-2,6-Dimethyl-2,3-dihydro-1H-inden-1-yl]-6-[(1-methoxypropan-2-yl)oxy]-1,3,5-triazin-2,4-diamin,
N-(2,6-Dimethyl-2,3-dihydro-1H-inden-1-yl)-6-(2-methoxyprop-1-yloxy)-1,3,5-triazin-2,4-diamin,
N-[(1R,2S)-2,6-Dimethyl-2,3-dihydro-1H-inden-1-yl]-6-(2-methoxyprop-1-yloxy)-1,3,5-triazin-2,4-diamin,
N-[(1S,2R)-2,6-Dimethyl-2,3-dihydro-1H-inden-1-yl]-6-(2-methoxyprop-1-yloxy)-1,3,5-triazin-2,4-diamin,
N-(2,6-Dimethyl-2,3-dihydro-1H-inden-1-yl)-6-(methylsulfanyl)-1,3,5-triazin-2,4-diamin,
N-(2,6-Dimethyl-2,3-dihydro-1H-inden-1-yl)-6-(methylsulfinyl)-1,3,5-triazin-2,4-diamin,
N-(2,6-Dimethyl-2,3-dihydro-1H-inden-1-yl)-6-(methylsulfonyl)-1,3,5-triazin-2,4-diamin,
N-[(1R,2S)-2,6-Dimethyl-2,3-dihydro-1H-inden-1-yl]-6-(methylsulfanyl)-1,3,5-triazin-2,4-diamin,
N-[(1R,2S)-2,6-Dimethyl-2,3-dihydro-1H-inden-1-yl]-6-(methylsulfinyl)-1,3,5-triazin-2,4-diamin,
N-[(1R,2S)-2,6-Dimethyl-2,3-dihydro-1H-inden-1-yl]-6-(methylsulfonyl)-1,3,5-triazin-2,4-diamin,
N-[(1S,2R)-2,6-Dimethyl-2,3-dihydro-1H-inden-1-yl]-6-(methylsulfanyl)-1,3,5-triazin-2,4-diamin,
N-[(1S,2R)-2,6-Dimethyl-2,3-dihydro-1H-inden-1-yl]-6-(methylsulfinyl)-1,3,5-triazin-2,4-diamin,
N-[(1S,2R)-2,6-Dimethyl-2,3-dihydro-1H-inden-1-yl]-6-(methylsulfonyl)-1,3,5-triazin-2,4-diamin,
N-(2,6-Dimethyl-2,3-dihydro-1H-inden-1-yl)-6-(ethylsulfanyl)-1,3,5-triazin-2,4-diamin,
N-(2,6-Dimethyl-2,3-dihydro-1H-inden-1-yl)-6-(ethylsulfinyl)-1,3,5-triazin-2,4-diamin,
N-(2,6-Dimethyl-2,3-dihydro-1H-inden-1-yl)-6-(ethylsulfonyl)-1,3,5-triazin-2,4-diamin,
N-[(1R,2S)-2,6-Dimethyl-2,3-dihydro-1H-inden-1-yl]-6-(ethylsulfanyl)-1,3,5-triazin-2,4-diamin,
N-[(1R,2S)-2,6-Dimethyl-2,3-dihydro-1H-inden-1-yl]-6-(ethylsulfinyl)-1,3,5-triazin-2,4-diamin,
N-[(1R,2S)-2,6-Dimethyl-2,3-dihydro-1H-inden-1-yl]-6-(ethylsulfonyl)-1,3,5-triazin-2,4-diamin,
N-[(1S,2R)-2,6-Dimethyl-2,3-dihydro-1H-inden-1-yl]-6-(ethylsulfanyl)-1,3,5-triazin-2,4-diamin,
N-[(1S,2R)-2,6-Dimethyl-2,3-dihydro-1H-inden-1-yl]-6-(ethylsulfinyl)-1,3,5-triazin-2,4-diamin,
N-[(1S,2R)-2,6-Dimethyl-2,3-dihydro-1H-inden-1-yl]-6-(ethylsulfonyl)-1,3,5-triazin-2,4-diamin,
N²-(6-Brom-2-methyl-2,3-dihydro-1H-inden-1-yl)-1,3,5-triazin-2,4,6-triamin,
N²-(6-Chlor-2-methyl-2,3-dihydro-1H-inden-1-yl)-1,3,5-triazin-2,4,6-triamin,
N²-(6-Fluor-2-methyl-2,3-dihydro-1H-inden-1-yl)-1,3,5-triazin-2,4,6-triamin,
N²-(6-Ethyl-2-methyl-2,3-dihydro-1H-inden-1-yl)-1,3,5-triazin-2,4,6-triamin,
N²-(2-Ethyl-6-methyl-2,3-dihydro-1H-inden-1-yl)-1,3,5-triazin-2,4,6-triamin,
N²-(6-Methoxy-2-methyl-2,3-dihydro-1H-inden-1-yl)-1,3,5-triazin-2,4,6-triamin,
N²-(2,6-Diethyl-2,3-dihydro-1H-inden-1-yl)-1,3,5-triazin-2,4,6-triamin,
N²-(6-Brom-2-methyl-2,3-dihydro-1H-inden-1-yl)-N⁴-methyl-1,3,5-triazin-2,4,6-triamin,
N²-(6-Chlor-2-methyl-2,3-dihydro-1H-inden-1-yl)-N⁴-methyl-1,3,5-triazin-2,4,6-triamin,
N²-(6-Fluor-2-methyl-2,3-dihydro-1H-inden-1-yl)-N⁴-methyl-1,3,5-triazin-2,4,6-triamin,
N²-(6-Ethyl-2-methyl-2,3-dihydro-1H-inden-1-yl)-N⁴-methyl-1,3,5-triazin-2,4,6-triamin,
N²-(2-Ethyl-6-methyl-2,3-dihydro-1H-inden-1-yl)-N⁴-methyl-1,3,5-triazin-2,4,6-triamin,
N²-(6-Methoxy-2-methyl-2,3-dihydro-1H-inden-1-yl)-N⁴-methyl-1,3,5-triazin-2,4,6-triamin,
N²-(2,6-Diethyl-2,3-dihydro-1H-inden-1-yl)-N⁴-methyl-1,3,5-triazin-2,4,6-triamin,
N²-(6-Brom-2-methyl-2,3-dihydro-1H-inden-1-yl)-N⁴-ethyl-1,3,5-triazin-2,4,6-triamin,
N²-(6-Chlor-2-methyl-2,3-dihydro-1H-inden-1-yl)-N⁴-ethyl-1,3,5-triazin-2,4,6-triamin,
N²-(6-Fluor-2-methyl-2,3-dihydro-1H-inden-1-yl)-N⁴-ethyl-1,3,5-triazin-2,4,6-triamin,
N²-(6-Ethyl-2-methyl-2,3-dihydro-1H-inden-1-yl)-N⁴-ethyl-1,3,5-triazin-2,4,6-triamin,
N²-(2-Ethyl-6-methyl-2,3-dihydro-1H-inden-1-yl)-N⁴-ethyl-1,3,5-triazin-2,4,6-triamin,
N²-(6-Methoxy-2-methyl-2,3-dihydro-1H-inden-1-yl)-N⁴-ethyl-1,3,5-triazin-2,4,6-triamin,
N²-(2,6-Diethyl-2,3-dihydro-1H-inden-1-yl)-N⁴-ethyl-1,3,5-triazin-2,4,6-triamin,
N-(6-Brom-2-methyl-2,3-dihydro-1H-inden-1-yl)-6-methoxy-1,3,5-triazin-2,4-diamin,
N-(6-Chlor-2-methyl-2,3-dihydro-1H-inden-1-yl)-6-methoxy-1,3,5-triazin-2,4-diamin,
N-(6-Fluor-2-methyl-2,3-dihydro-1H-inden-1-yl)-6-methoxy-1,3,5-triazin-2,4-diamin,
N-(6-Ethyl-2-methyl-2,3-dihydro-1H-inden-1-yl)-6-methoxy-1,3,5-triazin-2,4-diamin,
N-(2-Ethyl-6-methyl-2,3-dihydro-1H-inden-1-yl)-6-methoxy-1,3,5-triazin-2,4-diamin,
N-(6-Methoxy-2-methyl-2,3-dihydro-1H-inden-1-yl)-6-methoxy-1,3,5-triazin-2,4-diamin,
N-(2,6-Diethyl-2,3-dihydro-1H-inden-1-yl)-6-methoxy-1,3,5-triazin-2,4-diamin,
N-(6-Brom-2-methyl-2,3-dihydro-1H-inden-1-yl)-6-hydroxy-1,3,5-triazin-2,4-diamin,
N-(6-Chlor-2-methyl-2,3-dihydro-1H-inden-1-yl)-6-hydroxy-1,3,5-triazin-2,4-diamin,
N-(6-Fluor-2-methyl-2,3-dihydro-1H-inden-1-yl)-6-hydroxy-1,3,5-triazin-2,4-diamin,
N-(6-Ethyl-2-methyl-2,3-dihydro-1H-inden-1-yl)-6-hydroxy-1,3,5-triazin-2,4-diamin,
N-(2-Ethyl-6-methyl-2,3-dihydro-1H-inden-1-yl)-6-hydroxy-1,3,5-triazin-2,4-diamin,
N-(6-Methoxy-2-methyl-2,3-dihydro-1H-inden-1-yl)-6-hydroxy-1,3,5-triazin-2,4-diamin,
N-(2,6-Diethyl-2,3-dihydro-1H-inden-1-yl)-6-hydroxy-1,3,5-triazin-2,4-diamin,
N-(6-Brom-2-methyl-2,3-dihydro-1H-inden-1-yl)-6-ethoxy-1,3,5-triazin-2,4-diamin,
N-(6-Chlor-2-methyl-2,3-dihydro-1H-inden-1-yl)-6-ethoxy-1,3,5-triazin-2,4-diamin,
N-(6-Fluor-2-methyl-2,3-dihydro-1H-inden-1-yl)-6-ethoxy-1,3,5-triazin-2,4-diamin,
N-(6-Ethyl-2-methyl-2,3-dihydro-1H-inden-1-yl)-6-ethoxy-1,3,5-triazin-2,4-diamin,
N-(2-Ethyl-6-methyl-2,3-dihydro-1H-inden-1-yl)-6-ethoxy-1,3,5-triazin-2,4-diamin,
N-(6-Methoxy-2-methyl-2,3-dihydro-1H-inden-1-yl)-6-ethoxy-1,3,5-triazin-2,4-diamin,
N-(2,6-Diethyl-2,3-dihydro-1H-inden-1-yl)-6-ethoxy-1,3,5-triazin-2,4-diamin,
N-(6-Brom-2-methyl-2,3-dihydro-1H-inden-1-yl)-6-methylsulfanyl-1,3,5-triazin-2,4-diamin,
N-(6-Chlor-2-methyl-2,3-dihydro-1H-inden-1-yl)-6-methylsulfanyl-1,3,5-triazin-2,4-diamin,
N-(6-Fluor-2-methyl-2,3-dihydro-1H-inden-1-yl)-6-methylsulfanyl-1,3,5-triazin-2,4-diamin,
N-(6-Ethyl-2-methyl-2,3-dihydro-1H-inden-1-yl)-6-methylsulfanyl-1,3,5-triazin-2,4-diamin,
N-(2-Ethyl-6-methyl-2,3-dihydro-1H-inden-1-yl)-6-methylsulfanyl-1,3,5-triazin-2,4-diamin,
N-(6-Methoxy-2-methyl-2,3-dihydro-1H-inden-1-yl)-6-methylsulfanyl-1,3,5-triazin-2,4-diamin,
N-(2,6-Diethyl-2,3-dihydro-1H-inden-1-y1)-6-methylsulfanyl-1,3,5-triazin-2,4-diamin,
N-(6-Brom-2-methyl-2,3-dihydro-1H-inden-1-yl)-6-ethylsulfanyl-1,3,5-triazin-2,4-diamin,
N-(6-Chlor-2-methyl-2,3-dihydro-1H-inden-1-yl)-6-ethylsulfanyl-1,3,5-triazin-2,4-diamin,
N-(6-Fluor-2-methyl-2,3-dihydro-1H-inden-1-yl)-6-ethylsulfanyl-1,3,5-triazin-2,4-diamin,
N-(6-Ethyl-2-methyl-2,3-dihydro-1H-inden-1-yl)-6-ethylsulfanyl-1,3,5-triazin-2,4-diamin,
N-(2-Ethyl-6-methyl-2,3-dihydro-1H-inden-1-yl)-6-ethylsulfanyl-1,3,5-triazin-2,4-diamin,
N-(6-Methoxy-2-methyl-2,3-dihydro-1H-inden-1-yl)-6-ethylsulfanyl-1,3,5-triazin-2,4-diamin,
N-(2,6-Diethyl-2,3-dihydro-1H-inden-1-yl)-6-ethylsulfanyl-1,3,5-triazin-2,4-diamin,

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen, beliebig kombiniert werden.

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:

Bei der Benennung der erfindungsgemäßen substituierten Indanylaminotriazine der allgemeinen Formel (I) entsprechend der IUPAC-Nomenklatur werden die Kennzeichnungen der Stereozentren entsprechend der Cahn-Ingold-Prelog Regeln nur angegeben, wenn es eine eindeutige Orientierung an dem betreffenden Stereozentrum gibt. Falls an dem betreffenden Stereozentrum beide Konfigurationen in der betreffenden chemischen Substanz vorliegen, erfolgt keine gesonderte Kennzeichnung. Daher enthält beispielsweise die zur IUPAC-Formel N²-[(1R,2S)-2,6-Dimethyl-2,3-dihydro-1H-inden-1-yl]-1,3,5-triazin-2,4,6-triamin gehörende chemische Substanz zwei definierte Konfigurationen an den Stereozentren, 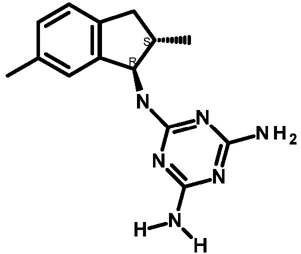 während die zur IUPAC-Formel N²-(6-Brom-2-methyl-2,3-dihydro-1H-inden-1-yl)-1,3,5-triazin-2,4,6-triamin gehörende chemische Substanz ein Gemisch aller möglichen Stereoisomere infolge der nichtdefinierten Konfigurationen an den zwei vorhandenen Stereozentren darstellt:

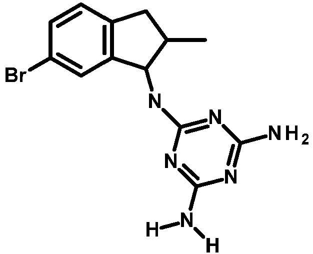

In der weiter unten ausgeführten Synthesebeschreibung wird eine weitere eindeutige Zuordnung von IUPAC-Namen und gezeichneten Strukturen beispielhaft ausgeführt.

Sofern nicht anders definiert, gilt generell für die Bezeichnung von chemischen Gruppen, daß die Anbindung an das Gerüst bzw. den Rest des Moleküls über das zuletzt genannte Strukturelement der betreffenden chemischen Gruppe erfolgt, d.h. beispielsweise im Falle von (C₂-C₈)-Alkenyloxy über das Sauerstoffatom, und im Falle von Heterocyclyl-(C₁-C₈)-alkyl oder R¹³O(O)C-(C₁-C₈)-Alkyl jeweils über das C-Atom der Alkylgruppe. In einer zusammengesetzten chemischen Gruppe wie z. B. Heterocyclyl-(C₁-C₈)-alkyl oder R¹³O(O)C-(C₁-C₈)-Alkyl steht die Bezeichnung "Alkyl" daher auch für eine Alkylengruppe. Bei den funktionellen Gruppen C(=O)R¹³, C(=O)OR¹³, C(=O)NR¹¹R¹², NR¹¹R¹², OR¹³, S(O)ₘR¹⁴ erfolgt die Anbindung an das Gerüst bzw. den Rest des Moleküls über das zuerst genannte Strukturelement der betreffenden chemischen Gruppe.

Erfindungsgemäß steht "Alkylsulfonyl" - in Alleinstellung oder als Bestandteil einer chemischen Gruppe - für geradkettiges oder verzweigtes Alkylsulfonyl, vorzugsweise mit 1 bis 8, oder mit 1 bis 6 Kohlenstoffatomen, z.B. (aber nicht beschränkt auf) (C₁-C₆)-Alkylsulfonyl wie Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl.

Erfindungsgemäß steht "Heteroarylsulfonyl" für gegebenenfalls substituiertes Pyridylsulfonyl, Pyrimidinylsulfonyl, Pyrazinylsulfonyl oder gegebenenfalls substituiertes polycyclisches Heteroarylsulfonyl, hier insbesondere gegebenenfalls substituiertes Chinolinylsulfonyl, beispielsweise substituiert durch Fluor, Chlor, Brom, Iod, Cyano, Nitro, Alkyl-, Haloalkyl-, Haloalkoxy-, Amino-, Alkylamino-, Alkylcarbonylamino-, Dialkylamino- oder Alkoxygruppen.

Erfindungsgemäß steht "Alkylthio" - in Alleinstellung oder als Bestandteil einer chemischen Gruppe - für geradkettiges oder verzweigtes S-Alkyl, vorzugsweise mit 1 bis 8, oder mit 1 bis 6 Kohlenstoffatomen, wie (C₁-C₁₀)-, (C₁-C₆)- oder (C₁-C₄)-Alkylthio, z.B. (aber nicht beschränkt auf) (C₁-C₆)-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 1,1-Dimethylpropylthio, 1,2-Dimethylpropylthio, 2,2-Dimethylpropylthio, 1-Ethylpropylthio, Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio.

"Alkenylthio" bedeutet erfindungsgemäßt ein über ein Schwefelatom gebundenen Alkenylrest, Alkinylthio bedeutet ein über ein Schwefelatom gebundenen Alkinylrest, Cycloalkylthio bedeutet ein über ein Schwefelatom gebundenen Cycloalkylrest und Cycloalkenylthio bedeutet ein über ein Schwefelatom gebundenen Cycloalkenylrest.

"Alkylsulfinyl (Alkyl-S(=O)-)", soweit nicht an anderer Stelle anders definiert steht erfindungsgemäß für Alkylreste, die über -S(=O)- an das Gerüst gebunden sind, wie (C₁-C₁₀)-, (C₁-C₆)- oder (C₁-C₄)-Alkylsulfinyl, z. B. (aber nicht beschränkt auf) (C₁-C₆)-Alkylsulfinyl wie Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 1,1-Dimethylpropylsulfinyl, 1,2-Dimethylpropylsulfinyl, 2,2-Dimethylpropylsulfinyl, 1-Ethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl.

Analog sind "Alkenylsulfinyl" und "Alkinylsulfinyl", erfindungsgemäß definiert als Alkenyl- bzw. Alkinylreste, die über -S(=O)- an das Gerüst gebunden sind, wie (C₂-C₁₀)-, (C₂-C₆)- oder (C₂-C₄)-Alkenylsulfinyl bzw. (C₃-C₁₀)-, (C₃-C₆)- oder (C₃-C₄)-Alkinylsulfinyl.

Analog sind "Alkenylsulfonyl" und "Alkinylsulfonyl" erfindungsgemäß definiert als Alkenyl- bzw. Alkinylreste, die über -S(=O)₂- an das Gerüst gebunden sind, wie (C₂-C₁₀)-, (C₂-C₆)- oder (C₂-C₄)-Alkenylsulfonyl bzw. (C₃-C₁₀)-, (C₃-C₆)- oder (C₃-C₄)-Alkinylsulfonyl.

"Alkoxy" bedeutet ein über ein Sauerstoffatom gebundenen Alkylrest, z. B. (aber nicht beschränkt auf) (C₁-C₆)-Alkoxy wie Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Alkenyloxy bedeutet ein über ein Sauerstoffatom gebundenen Alkenylrest, Alkinyloxy bedeutet ein über ein Sauerstoffatom gebundenen Alkinylrest wie (C₂-C₁₀)-, (C₂-C₆)- oder (C₂-C₄)-Alkenoxy bzw. (C₃-C₁₀)-, (C₃-C₆)- oder (C₃-C₄)-Alkinoxy.

"Cycloalkyloxy" bedeutet ein über ein Sauerstoffatom gebundenen Cycloalkylrest und Cycloalkenyloxy bedeutet ein über ein Sauerstoffatom gebundenen Cycloalkenylrest.

"Alkylcarbonyl" (Alkyl-C(=O)-), soweit nicht an anderer Stelle anders definiert, steht erfindungsgemäß für Alkylreste, die über -C(=O)- an das Gerüst gebunden sind, wie (C₁-C₁₀)-, (C₁-C₆)- oder (C₁-C₄)-Alkylcarbonyl. Die Anzahl der C-Atome bezieht sich dabei auf den Alkylrest in der Alkylcarbonylgruppe.

Analog stehen "Alkenylcarbonyl" und "Alkinylcarbonyl", soweit nicht an anderer Stelle anders definiert, erfindungsgemäß für Alkenyl- bzw. Alkinylreste, die über -C(=O)- an das Gerüst gebunden sind, wie (C₂-C₁₀)-, (C₂-C₆)- oder (C₂-C₄)-Alkenylcarbonyl bzw. (C₂-C₁₀)-, (C₂-C₆)- oder (C₂-C₄)-Alkinylcarbonyl. Die Anzahl der C-Atome bezieht sich dabei auf den Alkenyl- bzw. Alkinylrest in der Alkenyl- bzw. Alkinylcarbonylgruppe.

"Alkoxycarbonyl (Alkyl-O-C(=O)-)", soweit nicht an anderer Stelle anders definiert: Alkylreste, die über -O-C(=O)- an das Gerüst gebunden sind, wie (C₁-C₁₀)-, (C₁-C₆)- oder (C₁-C₄)-Alkoxycarbonyl. Die Anzahl der C-Atome bezieht sich dabei auf den Alkylrest in der Alkoxycarbonylgruppe. Analog stehen "Alkenyloxycarbonyl" und "Alkinyloxycarbonyl", soweit nicht an anderer Stelle anders definiert, erfindungsgemäß für Alkenyl- bzw. Alkinylreste, die über -O-C(=O)- an das Gerüst gebunden sind, wie (C₂-C₁₀)-, (C₂-C₆)- oder (C₂-C₄)-Alkenyloxycarbonyl bzw. (C₃-C₁₀)-, (C₃-C₆)- oder (C₃-C₄)-Alkinyloxycarbonyl. Die Anzahl der C-Atome bezieht sich dabei auf den Alkenyl- bzw. Alkinylrest in der Alken- bzw. Alkinyloxycarbonylgruppe.

Der Begriff "Alkylcarbonyloxy" (Alkyl-C(=O)-O-) steht erfindungsgemäß, soweit nicht an anderer Stelle anders definiert, für Alkylreste, die über eine Carbonyloxygruppe (-C(=O)-O-) mit dem Sauerstoff an das Gerüst gebunden sind, wie (C₁-C₁₀)-, (C₁-C₆)- oder (C₁-C₄)-Alkylcarbonyloxy. Die Anzahl der C-Atome bezieht sich dabei auf den Alkylrest in der Alkylcarbonyloxygruppe.

Analog sind "Alkenylcarbonyloxy" und "Alkinylcarbonyloxy" erfindungsgemäß definiert als Alkenyl- bzw. Alkinylreste, die über (-C(=O)-O-) mit dem Sauerstoff an das Gerüst gebunden sind, wie (C₂-C₁₀)-, (C₂-C₆)- oder (C₂-C₄)-Alkenylcarbonyloxy bzw. (C₂-C₁₀)-, (C₂-C₆)- oder (C₂-C₄)-Alkinylcarbonyloxy. Die Anzahl der C-Atome bezieht sich dabei auf den Alkenyl- bzw. Alkinylrest in der Alkenyl- bzw. Alkinylcarbonyloxygruppe.

In Kurzformen wie z.B. C(O)R¹³, C(O)OR¹³, OC(O)NR¹¹R¹², oder C(O)NR¹¹R¹² steht die in Klammern aufgeführte Kurzform O für ein über eine Doppelbindung an das benachbarte Kohlenstoffatom gebundenes Sauerstoffatom.

In Kurzformen wie z.B. OC(S)OR¹³, OC(S)SR¹⁴, OC(S)NR¹¹R¹², steht die in Klammern aufgeführte Kurzform S für ein über eine Doppelbindung an das benachbarte Kohlenstoffatom gebundenes Schwefelatom.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl oder Naphtyl, insbesondere vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst. Bevorzugte Aryl-Substituenten sind hier zum Beispiel Wasserstoff, Halogen, Alkyl, Cycloalkyl, Cycloalkylalkyl, Cycloalkenyl, Halocycloalkyl, Alkenyl, Alkinyl, Aryl, Arylalkyl, Arylalkenyl, Heteroaryl, Heteroarylalkyl, Heterocyclyl, Heterocyclylalkyl, Alkoxyalkyl, Alkylthio, Haloalkylthio, Haloalkyl, Alkoxy, Haloalkoxy, Cycloalkoxy, Cycloalkylalkoxy, Aryloxy, Heteroraryloxy, Alkoxyalkoxy, Alkinylalkoxy, Alkenyloxy, Bis-alkylaminoalkoxy, Tris-[alkyl]silyl, Bis-[alkyl]arylsilyl, Bis-[alkyl]alkylsilyl, Tris-[alkyl]silylalkinyl, Arylalkinyl, Heteroarylalkinyl, Alkylalkinyl, Cycloalkylalkinyl, Haloalkylalkinyl, Heterocyclyl-N-alkoxy, Nitro, Cyano, Amino, Alkylamino, Bis-alkylamino, Alkylcarbonylamino, Cycloalkylcarbonylamino, Arylcarbonylamino, Alkoxycarbonylamino, Alkoxycarbonylalkylamino, Arylalkoxycarbonylalkylamino, Hydroxycarbonyl, Alkoxycarbonyl, Aminocarbonyl, Alkylaminocarbonyl, Cycloalkylaminocarbonyl, Bis-Alkylaminocarbonyl, Heteroarylalkoxy, Arylalkoxy. Die Bezeichnung "Indan" oder "Indanyl" steht für ein "2,3-dihydro-1H-inden" oder "2,3-dihydro-1H-indenyl"-System.

Wenn nicht anders in einem bestimmten Kontext hier definiert, enthält ein "heterocyclischer Rest" ("Heterocyclyl") mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P) der gesättigt, ungesättigt, teilgesättigt oder heteroaromatisch ist und dabei unsubstituiert oder substituiert sein kann, wobei die Bindungsstelle an einem Ringatom lokalisiert ist. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfasst, wie beispielsweise 8-Aza-bicyclo[3.2.1]octanyl, 8-Azabicyclo[2.2.2]octanyl oder 1-Aza-bicyclo[2.2.1]heptyl. Im Falle von gegebenenfalls substituiertem Heterocyclyl werden auch spirocyclische Systeme umfasst, wie beispielsweise 1-Oxa-5-azaspiro[2.3]hexyl. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen, wie beispielsweise mit einem Heteroatom aus der Gruppe N, O und S 1- oder 2- oder 3-Pyrrolidinyl, 3,4-Dihydro-2H-pyrrol-2- oder 3-yl, 2,3-Dihydro-1H-pyrrol-1- oder 2- oder 3- oder 4- oder 5-yl; 2,5-Dihydro-1H-pyrrol-1- oder 2- oder 3-yl, 1- oder 2- oder 3- oder 4-Piperidinyl; 2,3,4,5-Tetrahydropyridin-2- oder 3- oder 4- oder 5-yl oder 6-yl; 1,2,3,6-Tetrahydropyridin-1- oder 2- oder 3- oder 4- oder 5- oder 6-yl; 1,2,3,4-Tetrahydropyridin-1- oder 2- oder 3- oder 4- oder 5- oder 6-yl; 1,4-Dihydropyridin-1- oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2- oder 3- oder 4-Azepanyl; 2,3,4,5-Tetrahydro-1H-azepin-1- oder 2- oder 3- oder 4- oder 5- oder 6- oder 7-yl; 2,3,4,7-Tetrahydro-1H-azepin-1- oder 2- oder 3- oder 4- oder 5- oder 6- oder 7-yl; 2,3,6,7-Tetrahydro-1H-azepin-1- oder 2- oder 3- oder 4-yl; 3,4,5,6-Tetrahydro-2H-azepin-2- oder 3- oder 4- oder 5- oder 6- oder 7-yl; 4,5-Dihydro-1H-azepin-1- oder 2- oder 3- oder 4-yl; 2,5-Dihydro-1H-azepin-1- oder -2- oder 3- oder 4- oder 5- oder 6- oder 7-yl; 2,7-Dihydro-1H-azepin-1- oder -2- oder 3- oder 4-yl; 2,3-Dihydro-1H-azepin-1- oder -2- oder 3- oder 4- oder 5- oder 6- oder 7-yl; 3,4-Dihydro-2H-azepin-2- oder 3- oder 4- oder 5- oder 6- oder 7-yl; 3,6-Dihydro-2H-azepin-2- oder 3- oder 4- oder 5- oder 6- oder 7-yl; 5,6-Dihydro-2H-azepin-2- oder 3- oder 4- oder 5- oder 6- oder 7-yl; 4,5-Dihydro-3H-azepin-2- oder 3- oder 4- oder 5- oder 6- oder 7-yl; 1H-Azepin-1- oder -2- oder 3- oder 4- oder 5- oder 6- oder 7-yl; 2H-Azepin-2- oder 3- oder 4- oder 5- oder 6- oder 7-yl; 3H-Azepin-2- oder 3- oder 4- oder 5- oder 6- oder 7-yl; 4H-Azepin-2- oder 3- oder 4- oder 5- oder 6- oder 7-yl, 2- oder 3-Oxolanyl (= 2- oder 3-Tetrahydrofuranyl); 2,3-Dihydrofuran-2- oder 3- oder 4- oder 5-yl; 2,5-Dihydrofuran-2- oder 3-yl, 2- oder 3- oder 4-Oxanyl (= 2- oder 3- oder 4-Tetrahydropyranyl); 3,4-Dihydro-2H-pyran-2- oder 3- oder 4- oder 5- oder 6-yl; 3,6-Dihydro-2H-pyran-2- oder 3-oder 4- oder 5- oder 6-yl; 2H-Pyran-2- oder 3- oder 4- oder 5- oder 6-yl; 4H-Pyran-2- oder 3- oder 4-yl, 2- oder 3- oder 4-Oxepanyl; 2,3,4,5-Tetrahydrooxepin-2- oder 3- oder 4- oder 5- oder 6- oder 7-yl; 2,3,4,7-Tetrahydrooxepin-2- oder 3- oder 4- oder 5- oder 6- oder 7-yl; 2,3,6,7-Tetrahydrooxepin-2- oder 3- oder 4-yl; 2,3-Dihydrooxepin-2- oder 3- oder 4- oder 5- oder 6- oder 7-yl; 4,5-Dihydrooxepin-2- oder 3- oder 4-yl; 2,5-Dihydrooxepin-2- oder 3- oder 4- oder 5- oder 6- oder 7-yl; Oxepin-2- oder 3- oder 4- oder 5- oder 6- oder 7-yl; 2- oder 3-Tetrahydrothiophenyl; 2,3-Dihydrothiophen-2- oder 3- oder 4- oder 5-yl; 2,5-Dihydrothiophen-2- oder 3-yl; Tetrahydro-2H-thiopyran-2- oder 3- oder 4-yl; 3,4-Dihydro-2H-thiopyran-2- oder 3- oder 4- oder 5- oder 6-yl; 3,6-Dihydro-2H-thiopyran-2- oder 3- oder 4- oder 5- oder 6-yl; 2H-Thiopyran-2- oder 3- oder 4- oder 5- oder 6-yl; 4H-Thiopyran-2- oder 3- oder 4-yl. Bevorzugte 3-Ring und 4-Ring-Heterocyclen sind beispielsweise 1- oder 2-Aziridinyl, Oxiranyl, Thiiranyl, 1- oder 2- oder 3-Azetidinyl, 2- oder 3-Oxetanyl, 2- oder 3-Thietanyl, 1,3-Dioxetan-2-yl. Weitere Beispiele für "Heterocyclyl" sind ein partiell oder vollständig hydrierter heterocyclischer Rest mit zwei Heteroatomen aus der Gruppe N, O und S, wie beispielsweise 1- oder 2- oder 3- oder 4-Pyrazolidinyl; 4,5-Dihydro-3H-pyrazol- 3- oder 4- oder 5-yl; 4,5-Dihydro-1H-pyrazol-1- oder 3- oder 4- oder 5-yl; 2,3-Dihydro-1H-pyrazol-1- oder 2- oder 3- oder 4- oder 5-yl; 1- oder 2- oder 3- oder 4- Imidazolidinyl; 2,3-Dihydro-1H-imidazol-1- oder 2- oder 3- oder 4-yl; 2,5-Dihydro-1H-imidazol-1- oder 2- oder 4- oder 5-yl; 4,5-Dihydro-1H-imidazol-1- oder 2- oder 4- oder 5-yl; Hexahydropyridazin-1- oder 2- oder 3- oder 4-yl; 1,2,3,4-Tetrahydropyridazin-1- oder 2- oder 3- oder 4- oder 5- oder 6-yl; 1,2,3,6-Tetrahydropyridazin-1- oder 2- oder 3- oder 4- oder 5- oder 6-yl; 1,4,5,6-Tetrahydropyridazin-1- oder 3- oder 4- oder 5- oder 6-yl; 3,4,5,6-Tetrahydropyridazin-3- oder 4- oder 5-yl; 4,5-Dihydropyridazin-3- oder 4-yl; 3,4-Dihydropyridazin-3- oder 4- oder 5- oder 6-yl; 3,6-Dihydropyridazin-3- oder 4-yl; 1,6-Dihydropyriazin-1- oder 3- oder 4- oder 5- oder 6-yl; Hexahydropyrimidin-1- oder 2- oder 3- oder 4-yl; 1,4,5,6-Tetrahydropyrimidin-1- oder 2- oder 4- oder 5- oder 6-yl; 1,2,5,6-Tetrahydropyrimidin-1- oder 2- oder 4- oder 5- oder 6-yl; 1,2,3,4-Tetrahydropyrimidin-1- oder 2- oder 3- oder 4- oder 5- oder 6-yl; 1,6-Dihydropyrimidin-1- oder 2- oder 4- oder 5- oder 6-yl; 1,2-Dihydropyrimidin-1- oder 2- oder 4- oder 5- oder 6-yl; 2,5-Dihydropyrimidin-2- oder 4- oder 5-yl; 4,5-Dihydropyrimidin- 4- oder 5- oder 6-yl; 1,4-Dihydropyrimidin-1- oder 2- oder 4- oder 5- oder 6-yl; 1- oder 2- oder 3-Piperazinyl; 1,2,3,6-Tetrahydropyrazin-1- oder 2- oder 3- oder 5- oder 6-yl; 1,2,3,4-Tetrahydropyrazin-1- oder 2- oder 3- oder 4- oder 5- oder 6-yl; 1,2-Dihydropyrazin-1- oder 2- oder 3- oder 5- oder 6-yl; 1,4-Dihydropyrazin-1- oder 2- oder 3-yl; 2,3-Dihydropyrazin-2- oder 3- oder 5- oder 6-yl; 2,5-Dihydropyrazin-2- oder 3-yl; 1,3-Dioxolan-2- oder 4- oder 5-yl; 1,3-Dioxol-2- oder 4-yl; 1,3-Dioxan-2- oder 4- oder 5-yl; 4H-1,3-Dioxin-2- oder 4- oder 5- oder 6-yl; 1,4-Dioxan-2- oder 3- oder 5- oder 6-yl; 2,3-Dihydro-1,4-dioxin-2- oder 3- oder 5- oder 6-yl; 1,4-Dioxin-2- oder 3-yl; 1,2-Dithiolan-3- oder 4-yl; 3H-1,2-Dithiol-3- oder 4- oder 5-yl; 1,3-Dithiolan-2- oder 4-yl; 1,3-Dithiol-2- oder 4-yl; 1,2-Dithian-3- oder 4-yl; 3,4-Dihydro-1,2-dithiin-3- oder 4- oder 5- oder 6-yl; 3,6-Dihydro-1,2-dithiin-3- oder 4-yl; 1,2-Dithiin-3- oder 4-yl; 1,3-Dithian-2- oder 4- oder 5-yl; 4H-1,3-Dithiin-2- oder 4- oder 5- oder 6-yl; Isoxazolidin-2- oder 3- oder 4- oder 5-yl; 2,3-Dihydroisoxazol-2- oder 3- oder 4- oder 5-yl; 2,5-Dihydroisoxazol-2- oder 3- oder 4- oder 5-yl; 4,5-Dihydroisoxazol-3- oder 4- oder 5-yl; 1,3-Oxazolidin-2- oder 3- oder 4- oder 5-yl; 2,3-Dihydro-1,3-oxazol-2- oder 3- oder 4- oder 5-yl; 2,5-Dihydro-1,3-oxazol-2- oder 4- oder 5-yl; 4,5-Dihydro-1,3-oxazol-2- oder 4- oder 5-yl; 1,2-Oxazinan-2- oder 3- oder 4- oder 5- oder 6-yl; 3,4-Dihydro-2H-1,2-oxazin-2- oder 3- oder 4- oder 5- oder 6-yl; 3,6-Dihydro-2H-1,2-oxazin-2- oder 3- oder 4- oder 5- oder 6-yl; 5,6-Dihydro-2H-1,2-oxazin-2- oder 3- oder 4- oder 5- oder 6-yl; 5,6-Dihydro-4H-1,2-oxazin-3- oder 4- oder 5- oder 6-yl; 2H-1,2-Oxazin-2- oder 3- oder 4- oder 5- oder 6-yl; 6H-1,2-Oxazin-3- oder 4- oder 5- oder 6-yl; 4H-1,2-Oxazin-3- oder 4- oder 5- oder 6-yl; 1,3-Oxazinan-2- oder 3- oder 4- oder 5- oder 6-yl; 3,4-Dihydro-2H-1,3-oxazin-2- oder 3- oder 4- oder 5- oder 6-yl; 3,6-Dihydro-2H-1,3-oxazin-2- oder 3- oder 4- oder 5- oder 6-yl; 5,6-Dihydro-2H-1,3-oxazin-2- oder 4- oder 5- oder 6-yl; 5,6-Dihydro-4H-1,3-oxazin-2- oder 4- oder 5- oder 6-yl; 2H-1,3-Oxazin-2- oder 4- oder 5- oder 6-yl; 6H-1,3-Oxazin-2- oder 4- oder 5- oder 6-yl; 4H-1,3-Oxazin-2- oder 4- oder 5- oder 6-yl; Morpholin-2- oder 3- oder 4-yl; 3,4-Dihydro-2H-1,4-oxazin-2- oder 3- oder 4- oder 5- oder 6-yl; 3,6-Dihydro-2H-1,4-oxazin-2- oder 3- oder 5- oder 6-yl; 2H-1,4-oxazin-2- oder 3- oder 5- oder 6-yl; 4H-1,4-oxazin-2- oder 3-yl; 1,2-Oxazepan-2- oder 3- oder 4- oder 5- oder 6- oder 7-yl; 2,3,4,5-Tetrahydro-1,2-oxazepin-2- oder 3- oder 4- oder 5- oder 6- oder 7-yl; 2,3,4,7-Tetrahydro-1,2-oxazepin-2- oder 3- oder 4- oder 5- oder 6- oder 7-yl; 2,3,6,7-Tetrahydro-1,2-oxazepin-2- oder 3- oder 4- oder 5- oder 6- oder 7-yl; 2,5,6,7-Tetrahydro-1,2-oxazepin-2- oder 3- oder 4- oder 5- oder 6- oder 7-yl; 4,5,6,7-Tetrahydro-1,2-oxazepin-3- oder 4- oder 5- oder 6- oder 7-yl; 2,3-Dihydro-1,2-oxazepin-2- oder 3- oder 4- oder 5- oder 6- oder 7-yl; 2,5-Dihydro-1,2-oxazepin-2- oder 3- oder 4- oder 5- oder 6- oder 7-yl; 2,7-Dihydro-1,2-oxazepin-2- oder 3- oder 4- oder 5- oder 6- oder 7-yl; 4,5-Dihydro-1,2-oxazepin-3- oder 4- oder 5- oder 6- oder 7-yl; 4,7-Dihydro-1,2-oxazepin-3- oder 4- oder 5- oder 6- oder 7-yl; 6,7-Dihydro-1,2-oxazepin-3- oder 4- oder 5- oder 6- oder 7-yl; 1,2-Oxazepin-3- oder 4- oder 5- oder 6- oder 7-yl; 1,3-Oxazepan-2- oder 3- oder 4- oder 5- oder 6- oder 7-yl; 2,3,4,5-Tetrahydro-1,3-oxazepin-2- oder 3- oder 4- oder 5- oder 6- oder 7-yl; 2,3,4,7-Tetrahydro-1,3-oxazepin-2- oder 3- oder 4- oder 5- oder 6- oder 7-yl; 2,3,6,7-Tetrahydro-1,3-oxazepin-2- oder 3- oder 4- oder 5- oder 6- oder 7-yl; 2,5,6,7-Tetrahydro-1,3-oxazepin-2- oder 4- oder 5- oder 6- oder 7-yl; 4,5,6,7-Tetrahydro-1,3-oxazepin-2- oder 4- oder 5- oder 6- oder 7-yl; 2,3-Dihydro-1,3-oxazepin-2- oder 3- oder 4- oder 5- oder 6- oder 7-yl; 2,5-Dihydro-1,3-oxazepin-2- oder 4- oder 5- oder 6- oder 7-yl; 2,7-Dihydro-1,3-oxazepin-2- oder 4- oder 5- oder 6- oder 7-yl; 4,5-Dihydro- 1,3-oxazepin-2- oder 4- oder 5- oder 6- oder 7-yl; 4,7-Dihydro-1,3-oxazepin-2- oder 4- oder 5- oder 6- oder 7-yl; 6,7-Dihydro-1,3-oxazepin-2- oder 4- oder 5- oder 6- oder 7-yl; 1,3-Oxazepin-2- oder 4- oder 5- oder 6- oder 7-yl; 1,4-Oxazepan-2- oder 3- oder 5- oder 6- oder 7-yl; 2,3,4,5-Tetrahydro-1,4-oxazepin- 2- oder 3- oder 4- oder 5- oder 6- oder 7-yl; 2,3,4,7-Tetrahydro-1,4-oxazepin-2- oder 3- oder 4- oder 5- oder 6- oder 7-yl; 2,3,6,7-Tetrahydro-1,4-oxazepin-2- oder 3- oder 5- oder 6- oder 7-yl; 2,5,6,7- Tetrahydro-1,4-oxazepin-2- oder 3- oder 5- oder 6- oder 7-yl; 4,5,6,7-Tetrahydro-1,4-oxazepin-2- oder 3- oder 4- oder 5- oder 6- oder 7-yl; 2,3-Dihydro-1,4-oxazepin-2- oder 3- oder 5- oder 6- oder 7-yl; 2,5- Dihydro-1,4-oxazepin-2- oder 3- oder 5- oder 6- oder 7-yl; 2,7-Dihydro-1,4-oxazepin-2- oder 3- oder 5- oder 6- oder 7-yl; 4,5-Dihydro-1,4-oxazepin-2- oder 3- oder 4- oder 5- oder 6- oder 7-yl; 4,7-Dihydro- 1,4-oxazepin-2- oder 3- oder 4- oder 5- oder 6- oder 7-yl; 6,7-Dihydro-1,4-oxazepin-2- oder 3- oder 5- oder 6- oder 7-yl; 1,4-Oxazepin-2- oder 3- oder 5- oder 6- oder 7-yl; Isothiazolidin-2- oder 3- oder 4- oder 5-yl; 2,3-Dihydroisothiazol-2- oder 3- oder 4- oder 5-yl; 2,5-Dihydroisothiazol-2- oder 3- oder 4- oder 5- yl; 4,5-Dihydroisothiazol-3- oder 4- oder 5-yl; 1,3-Thiazolidin-2- oder 3- oder 4- oder 5-yl; 2,3-Dihydro- 1,3-thiazol-2- oder 3- oder 4- oder 5-yl; 2,5-Dihydro-1,3-thiazol-2- oder 4- oder 5-yl; 4,5-Dihydro-1,3- thiazol-2- oder 4- oder 5-yl; 1,3-Thiazinan-2- oder 3- oder 4- oder 5- oder 6-yl; 3,4-Dihydro-2H-1,3- thiazin-2- oder 3- oder 4- oder 5- oder 6-yl; 3,6-Dihydro-2H-1,3-thiazin-2- oder 3- oder 4- oder 5- oder 6- yl; 5,6-Dihydro-2H-1,3-thiazin-2- oder 4- oder 5- oder 6-yl; 5,6-Dihydro-4H-1,3-thiazin-2- oder 4- oder 5- oder 6-yl; 2H-1,3-Thiazin-2- oder 4- oder 5- oder 6-yl; 6H-1,3-Thiazin-2- oder 4- oder 5- oder 6-yl; 4H-1,3-Thiazin-2- oder 4- oder 5- oder 6-yl. Weitere Beispiele für "Heterocyclyl" sind ein partiell oder vollständig hydrierter heterocyclischer Rest mit 3 Heteroatomen aus der Gruppe N, O und S, wie beispielsweise 1,4,2-Dioxazolidin-2- oder 3- oder 5-yl; 1,4,2-Dioxazol-3- oder 5-yl; 1,4,2-Dioxazinan-2- oder -3- oder 5- oder 6-yl; 5,6-Dihydro-1,4,2-dioxazin-3- oder 5- oder 6-yl; 1,4,2-Dioxazin-3- oder 5- oder 6-yl; 1,4,2-Dioxazepan-2- oder 3- oder 5- oder 6- oder 7-yl; 6,7-Dihydro-5H-1,4,2-Dioxazepin-3- oder 5- oder 6- oder 7-yl; 2,3-Dihydro-7H-1,4,2-Dioxazepin-2- oder 3- oder 5- oder 6- oder 7-yl; 2,3- Dihydro-5H-1,4,2-Dioxazepin-2- oder 3- oder 5- oder 6- oder 7-yl; 5H-1,4,2-Dioxazepin-3- oder 5- oder 6- oder 7-yl; 7H-1,4,2-Dioxazepin-3- oder 5- oder 6- oder 7-yl. Strukturbeispiele für gegebenenfalls weiter substituierte Heterocyclen sind auch im Folgenden aufgeführt:

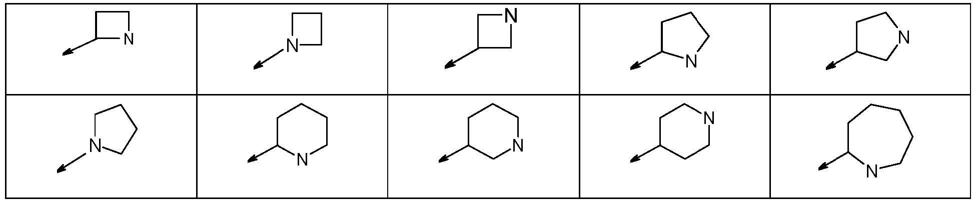

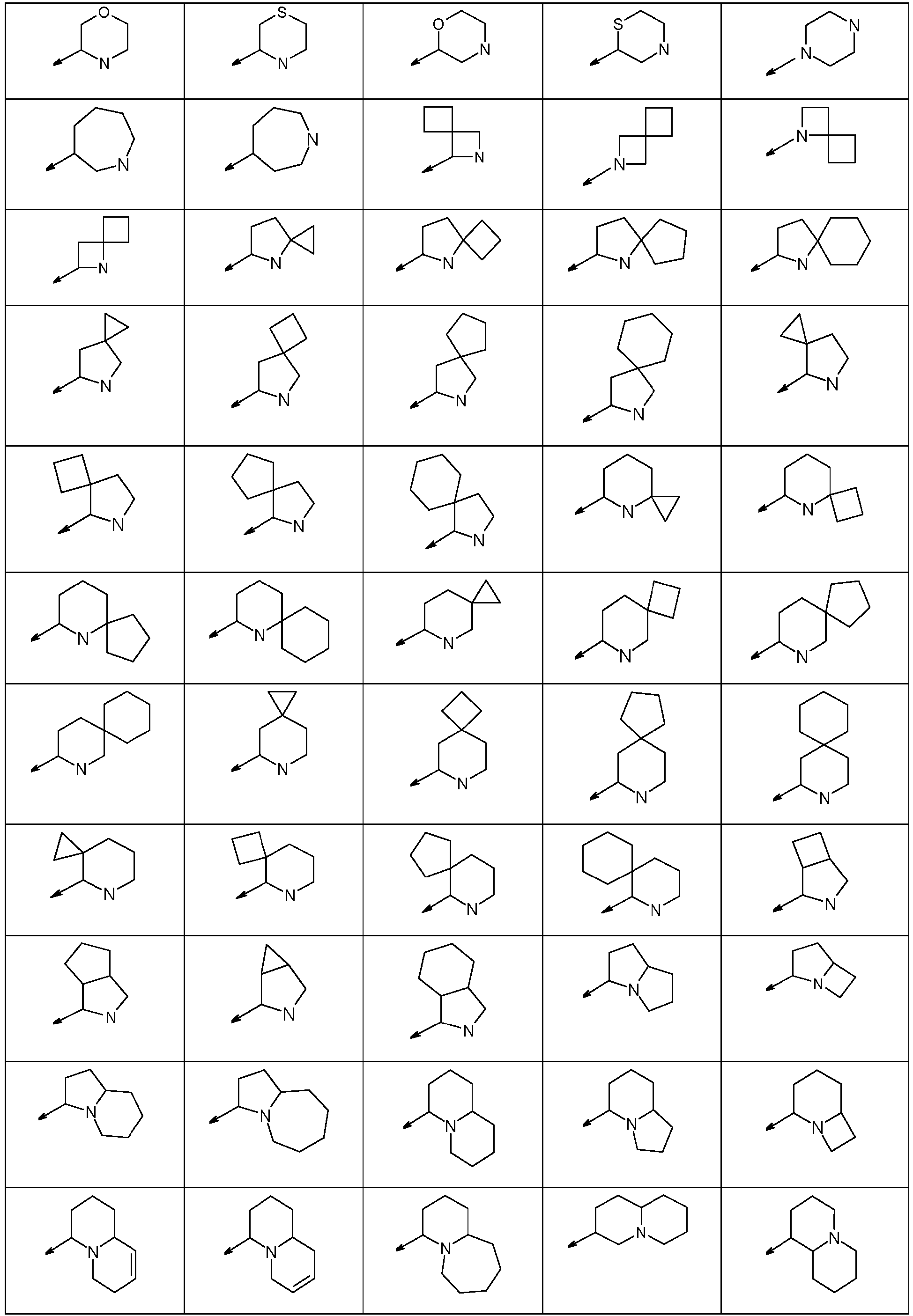

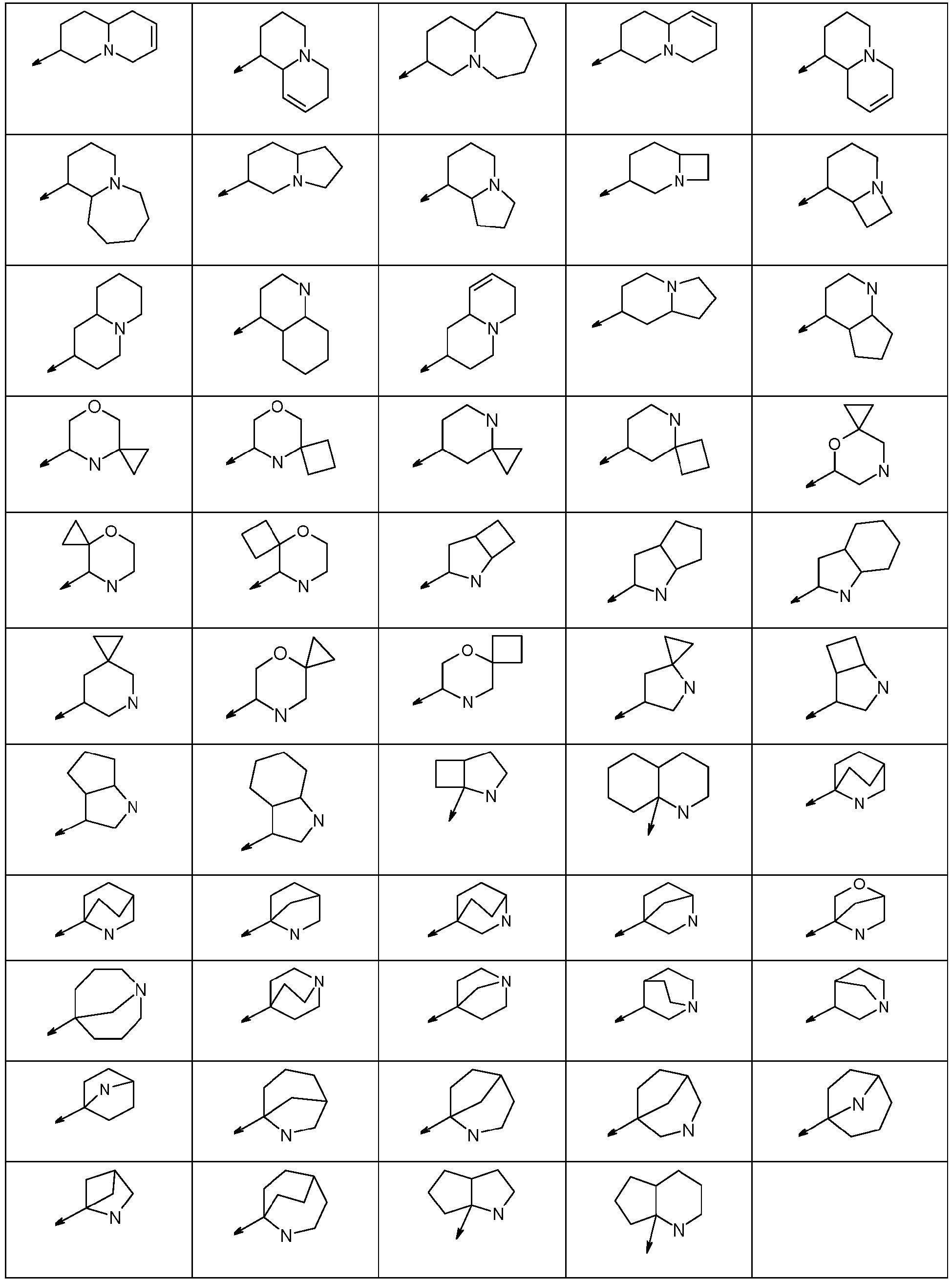

Die oben aufgeführten Heterocyclen sind bevorzugt beispielsweise durch Wasserstoff, Halogen, Alkyl, Haloalkyl, Hydroxy, Alkoxy, Cycloalkoxy, Aryloxy, Alkoxyalkyl, Alkoxyalkoxy, Cycloalkyl, Halocycloalkyl, Aryl, Arylalkyl, Heteroaryl, Heterocyclyl, Alkenyl, Alkylcarbonyl, Cycloalkylcarbonyl, Arylcarbonyl, Heteroarylcarbonyl, Alkoxycarbonyl, Hydroxycarbonyl, Cycloalkoxycarbonyl, Cycloalkylalkoxycarbonyl, Alkoxycarbonylalkyl, Arylalkoxycarbonyl, Arylalkoxycarbonylalkyl, Alkinyl, Alkinylalkyl, Alkylalkinyl, Tris-alkylsilylalkinyl, Nitro, Amino, Cyano, Haloalkoxy, Haloalkylthio, Alkylthio, Hydrothio, Hydroxyalkyl, Oxo, Heteroarylalkoxy, Arylalkoxy, Heterocyclylalkoxy, Heterocyclylalkylthio, Heterocyclyloxy, Heterocyclylthio, Heteroaryloxy, Bisalkylamino, Alkylamino, Cycloalkylamino, Hydroxycarbonylalkylamino, Alkoxycarbonylalkylamino, Arylalkoxycarbonylalkylamino, Alkoxycarbonylalkyl(alkyl)amino, Aminocarbonyl, Alkylaminocarbonyl, Bis-alkylaminocarbonyl, Cycloalkylaminocarbonyl, Hydroxycarbonylalkylaminocarbonyl, Alkoxycarbonylalkylaminocarbonyl, Arylalkoxycarbonylalkylaminocarbonyl substituiert.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Handelt es sich es sich um einen teilweise oder vollständig gesättigten Stickstoff-Heterocyclus, so kann dieser sowohl über Kohlenstoff als auch über den Stickstoff mit dem Rest des Moleküls verknüpft sein.

Als Substituenten für einen substituierten heterocyclischen Rest kommen die weiter unten genannten Substituenten in Frage, zusätzlich auch Oxo und Thioxo. Die "Oxogruppe" als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen N(O) , S(O) (auch kurz SO) und S(O)₂ (auch kurz SO₂) im heterocyclischen Ring. Im Fall von -N(O)- und -S(O)-Gruppen sind jeweils beide Enantiomere umfasst.

Erfindungsgemäß steht der Ausdruck "Heteroaryl" für heteroaromatische Verbindungen, d. h. vollständig ungesättigte aromatische heterocyclische Verbindungen, vorzugsweise für 5- bis 7-gliedrige Ringe mit 1 bis 4, vorzugsweise 1 oder 2 gleichen oder verschiedenen Heteroatomen, vorzugsweise O, S oder N. Erfindungsgemäße Heteroaryle sind beispielsweise 1H-Pyrrol-1-yl; 1H-Pyrrol-2-yl; 1H-Pyrrol-3-yl; Furan-2-yl; Furan-3-yl; Thien-2-yl; Thien-3-yl, 1H-Imidazol-1-yl; 1H-Imidazol-2-yl; 1H-Imidazol-4-yl; 1H-Imidazol-5-yl; 1H-Pyrazol-1-yl; 1H-Pyrazol-3-yl; 1H-Pyrazol-4-yl; 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-3-yl, 4H-1,2,4-Triazol-4-yl, 1,2,4-Oxadiazol-3-yl, 1,2,4- Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, Azepinyl, Pyridin-2-yl, Pyridin-3-yl, Pyridin-4-yl, Pyrazin-2-yl, Pyrazin-3-yl, Pyrimidin-2-yl, Pyrimidin- 4-yl, Pyrimidin-5-yl, Pyridazin-3-yl, Pyridazin-4-yl, 1,3,5-Triazin-2-yl, 1,2,4-Triazin-3-yl, 1,2,4-Triazin- 5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, Oxepinyl, Thiepinyl, 1,2,4-Triazolonyl und 1,2,4-Diazepinyl, 2H-1,2,3,4-Tetrazol-5-yl, 1H-1,2,3,4- Tetrazol-5-yl, 1,2,3,4-Oxatriazol-5-yl, 1,2,3,4-Thiatriazol-5-yl, 1,2,3,5-Oxatriazol-4-yl, 1,2,3,5- Thiatriazol-4-yl. Die erfindungsgemäßen Heteroarylgruppen können ferner mit einem oder mehreren, gleichen oder verschiedenen Resten substituiert sein. Sind zwei benachbarte Kohlenstoffatome Bestandteil eines weiteren aromatischen Rings, so handelt es sich um annellierte heteroaromatische Systeme, wie benzokondensierte oder mehrfach annellierte Heteroaromaten. Bevorzugt sind beispielsweise Chinoline (z. B. Chinolin-2-yl, Chinolin-3-yl, Chinolin-4-yl, Chinolin-5-yl, Chinolin-6-yl, Chinolin-7-yl, Chinolin-8-yl); Isochinoline (z. B. Isochinolin-1-yl, Isochinolin-3-yl, Isochinolin-4-yl, Isochinolin-5-yl, Isochinolin-6-yl, Isochinolin-7-yl, Isochinolin-8-yl); Chinoxalin; Chinazolin; Cinnolin; 1,5-Naphthyridin; 1,6-Naphthyridin; 1,7-Naphthyridin; 1,8-Naphthyridin; 2,6-Naphthyridin; 2,7- Naphthyridin; Phthalazin; Pyridopyrazine; Pyridopyrimidine; Pyridopyridazine; Pteridine; Pyrimidopyrimidine. Beispiele für Heteroaryl sind auch 5- oder 6-gliedrige benzokondensierte Ringe aus der Gruppe 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1-Benzofuran-2-yl, 1-Benzofuran-3-yl, 1-Benzofuran-4-yl, 1-Benzofuran-5-yl, 1- Benzofuran-6-yl, 1-Benzofuran-7-yl, 1-Benzothiophen-2-yl, 1-Benzothiophen-3-yl, 1-Benzothiophen-4- yl, 1-Benzothiophen-5-yl, 1-Benzothiophen-6-yl, 1-Benzothiophen-7-yl, 1H-Indazol-1-yl, 1H-Indazol-3- yl, 1H-Indazol-4-yl, 1H-Indazol-5-yl, 1H-Indazol-6-yl, 1H-Indazol-7-yl, 2H-Indazol-2-yl, 2H-Indazol-3- yl, 2H-Indazol-4-yl, 2H-Indazol-5-yl, 2H-Indazol-6-yl, 2H-Indazol-7-yl, 2H-Isoindol-2-yl, 2H-Isoindol- 1-yl, 2H-Isoindol-3-yl, 2H-Isoindol-4-yl, 2H-Isoindol-5-yl, 2H-Isoindol-6-yl; 2H-Isoindol-7-yl, 1H- Benzimidazol-1-yl, 1H-Benzimidazol-2-yl, 1H-Benzimidazol-4-yl, 1H-Benzimidazol-5-yl, 1H- Benzimidazol-6-yl, 1H-Benzimidazol-7-yl, 1,3-Benzoxazol-2-yl, 1,3-Benzoxazol-4-yl, 1,3-Benzoxazol- 5-yl, 1,3-Benzoxazol-6-yl, 1,3-Benzoxazol-7-yl, 1,3-Benzthiazol-2-yl, 1,3-Benzthiazol-4-yl, 1,3- Benzthiazol-5-yl, 1,3-Benzthiazol-6-yl, 1,3-Benzthiazol-7-yl, 1,2-Benzisoxazol-3-yl, 1,2-Benzisoxazol- 4-yl, 1,2-Benzisoxazol-5-yl, 1,2-Benzisoxazol-6-yl, 1,2-Benzisoxazol-7-yl, 1,2-Benzisothiazol-3-yl, 1,2- Benzisothiazol-4-yl, 1,2-Benzisothiazol-5-yl, 1,2-Benzisothiazol-6-yl, 1,2-Benzisothiazol-7-yl.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder Iodatom.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist und im letzteren Falle als "substituiertes Alkyl" bezeichnet wird. Bevorzugte Substituenten sind Halogenatome, Alkoxy-, Haloalkoxy-, Cyano-, Alkylthio, Haloalkylthio-, Amino- oder Nitrogruppen, besonders bevorzugt sind Methoxy, Methyl, Fluoralkyl, Cyano, Nitro, Fluor, Chlor, Brom oder Iod. Die Vorsilbe "Bis" schließt auch die Kombination unterschiedlicher Alkylreste ein, z. B. Methyl(Ethyl) oder Ethyl(Methyl). Alkylreste können in Abhängigkeit von der verwendeten Schreibweise unterschiedliche Bezeichnungen für die gleiche Gruppe haben, die allerdings fachlich geläufig sind. Beispielsweise sind die Bezeichnungen 1- Methyleth-1-yl, 1-Methylethyl, Prop-2-yl und iso-Propyl- synonym zu verstehen.

"Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie z. B. CH₂CH₂Cl, CH₂CH₂Br, CHClCH₃, CH₂Cl, CH₂F; Perhaloalkyl wie z. B. CCl₃, CClF₂, CFCl₂,CF₂CClF₂, CF₂CClFCF₃; Polyhaloalkyl wie z. B. CH₂CHFCl, CF₂CClFH, CF₂CBrFH, CH₂CF₃; Der Begriff Perhaloalkyl umfasst dabei auch den Begriff Perfluoralkyl.

"Haloalkoxy" ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Der hier beispielhaft genannte Ausdruck "(C₁-C₄)-Alkyl" bedeutet eine Kurzschreibweise für geradkettiges oder verzweigtes Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)-Alkyl", umfassen entsprechend auch geradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung.

Der Begriff "Alkenyl" schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl. Alkenyl bedeutet z.B. Vinyl, welches ggf. durch weitere Alkylreste substituiert sein kann, z B. (aber nicht beschränkt auf) (C₂-C₆)-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Der Begriff "Alkinyl" schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl. (C₂-C₆)-Alkinyl bedeutet z.B. Ethinyl, 1-Propinyl, 2-Propinyl, 1-Butinyl, 2- Butinyl, 3-Butinyl, 1-Methyl-2-propinyl, 1-Pentinyl, 2-Pentinyl, 3-Pentinyl, 4-Pentinyl, 1-Methyl-2- butinyl, 1-Methyl-3-butinyl, 2-Methyl-3-butinyl, 3-Methyl-1-butinyl, 1,1-Dimethyl-2-propinyl, 1-Ethyl- 2-propinyl, 1-Hexinyl, 2-Hexinyl, 3-Hexinyl, 4-Hexinyl, 5-Hexinyl, 1-Methyl-2-pentinyl, 1-Methyl-3- pentinyl, 1-Methyl-4-pentinyl, 2-Methyl-3-pentinyl, 2-Methyl-4-pentinyl, 3-Methyl-1-pentinyl, 3- Methyl-4-pentinyl, 4-Methyl-1-pentinyl, 4-Methyl-2-pentinyl, 1,1-Di-methyl-2-butinyl, 1,1-Dimethyl-3- butinyl, 1,2-Dimethyl-3-butinyl, 2,2-Dimethyl-3-butinyl, 3,3-Dimethyl-1-butinyl, 1-Ethyl-2-butinyl, 1- Ethyl-3-butinyl, 2-Ethyl-3-butinyl und 1-Ethyl-1-methyl-2-propinyl.

Der Begriff "Cycloalkyl" bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl, das gegebenenfalls weiter substituiert ist, bevorzugt durch Wasserstoff, Alkyl, Alkoxy, Oxo, Cyano, Nitro, Alkylthio, Haloalkylthio, Halogen, Alkenyl, Alkinyl, Haloalkyl, AMino, Alkylamino, Bisalkylamino, Alkocycarbonyl, Hydroxycarbonyl, Arylalkoxycarbonyl, Aminocarbonyl, Alkylaminocarbonyl, Cycloalkylaminocarbonyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfasst, wie beispielsweise Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl und Adamantan-2-yl, aber auch Systeme wie z. B. 1,1'-Bi(cyclopropyl)-1-yl, 1,1'-Bi(cyclopropyl)-2-yl. Der Ausdruck "(C₃-C₇)-Cycloalkyl" bedeutet eine Kurzschreibweise für Cycloalkyl mit drei bis 7 Kohlenstoffatomen entsprechend der Bereichsangabe für C-Atome.

Im Falle von substituiertem Cycloalkyl werden auch spirocyclische aliphatische Systeme umfasst, wie beispielsweise Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl.

"Cycloalkenyl" bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4- Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Der Begriff "Alkyliden", z. B. auch in der Form (C₁-C₁₀)-Alkyliden, bedeutet den Rest eines geradkettigen oder verzweigten offenkettigen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅. Cycloalkyliden bedeutet ein carbocyclischer Rest, der über eine Zweifachbindung gebunden ist.

"Cycloalkylalkyloxy" bedeutet ein über ein Sauerstoffatom gebundenen Cycloalkylalkylrest und "Arylalkyloxy" bedeutet ein über ein Sauerstoffatom gebundenen Arylalkylrest.

"Alkoxyalkyl" steht für einen über eine Alkylgruppe gebundenen Alkoxyrest und "Alkoxyalkoxy" bedeutet einen über ein Sauerstoffatom gebundenen Alkoxyalkylrest, z.B. (aber nicht beschränkt auf) Methoxymethoxy, Methoxyethoxy, Ethoxyethoxy, Methoxy-n-propyloxy.

"Alkylthioalkyl" steht für einen über eine Alkylgruppe gebundenen Alkylthiorest und "Alkylthioalkylthio" bedeutet einen über ein Sauerstoffatom gebundenen Alkylthioalkylrest.

"Arylalkoxyalkyl" steht für einen über eine Alkylgruppe gebundenen Aryloxyrest und "Heteroaryloxyalkyl" bedeutet einen über eine Alkylgruppe gebundenen Heteroaryloxyrest.

"Haloalkoxyalkyl" steht für einen gebundenen Haloalkoxyrest und "Haloalkylthioalkyl" bedeutet einen über eine Alkylgruppe gebundenen Haloalkylthiorest.

"Arylalkyl" steht für einen über eine Alkylgruppe gebundenen Arylrest, "Heteroarylalkyl" bedeutet einen über eine Alkylgruppe gebundenen Heteroarylrest, und "Heterocyclylalkyl" bedeutet einen über eine Alkylgruppe gebundenen Heterocyclylrest.

"Cycloalkylalkyl" steht für einen über eine Alkylgruppe gebundenen Cycloalkylrest, z. B. (aber nicht beschränkt auf) Cyclopropylmethyl, Cyclobutylmethyl, Cyclopentylmethyl, Cyclohexylmethyl, 1-Cyclopropyleth-1-yl, 2-Cyclopropyleth-1-yl, 1-Cyclopropylprop-1-yl, 3-Cyclopropylprop-1-yl.

"Arylalkenyl" steht für einen über eine Alkenylgruppe gebundenen Arylrest, "Heteroarylalkenyl" bedeutet einen über eine Alkenylgruppe gebundenen Heteroarylrest, und "Heterocyclylalkenyl" bedeutet einen über eine Alkenylgruppe gebundenen Heterocyclylrest.

"Arylalkinyl" steht für einen über eine Alkinylgruppe gebundenen Arylrest, "Heteroarylalkinyl" bedeutet einen über eine Alkinylgruppe gebundenen Heteroarylrest, und "Heterocyclylalkinyl" bedeutet einen über eine Alkinylgruppe gebundenen Heterocyclylrest.

Erfindungsgemäß steht "Haloalkylthio" - in Alleinstellung oder als Bestandteil einer chemischen Gruppe - für geradkettiges oder verzweigtes S-Halogenalkyl, vorzugsweise mit 1 bis 8, oder mit 1 bis 6 Kohlenstoffatomen, wie (C₁-C₈)-, (C₁-C₆)- oder (C₁-C₄)-Haloalkylthio, z.B. (aber nicht beschränkt auf) Trifluormethylthio oder Trifluormethylsulfanyl, Pentafluorethylthio oder Pentafluorethylsulfanyl, Difluormethyl, 2,2-Difluoreth-1-ylthio, 2,2,2-Difluoreth-1-ylthio, 3,3,3-prop-1-ylthio.

"Halocycloalkyl" und "Halocycloalkenyl" bedeuten durch gleiche oder verschiedene Halogenatome, wie z. B. F, Cl und Br, oder durch Haloalkyl, wie z. B. Trifluormethyl oder Difluormethyl teilweise oder vollständig substituiertes Cycloalkyl oder Cycloalkenyl , z.B. 1-Fluorcycloprop-1-yl, 2-Fluorcycloprop-1-yl, 2,2-Difluorcycloprop-1-yl, 1-Fluorcyclobut-1-yl, 1-Trifluormethylcycloprop-1-yl, 2-Trifluormethylcycloprop-1-yl, 1-Chlor-cycloprop-1-yl, 2-Chlorcycloprop-1-yl, 2,2-Dichlorcycloprop-1-yl, 3,3-Difluorcyclobutyl,

Erfindungsgemäß steht "Trialkylsilyl" oder "Tris(alkyl)silyl" - in Alleinstellung oder als Bestandteil einer chemischen Gruppe - für geradkettiges oder verzweigtes Si-Alkyl, vorzugsweise mit 1 bis 8, oder mit 1 bis 6 Kohlenstoffatomen, wie Tri-[(C₁-C₈)-, (C₁-C₆)- oder (C₁-C₄)-alkyl]silyl, z.B. (aber nicht beschränkt auf) Trimethylsilyl, Triethylsilyl, Tri-(n-propyl)silyl, Tri-(iso-propyl)silyl, Tri-(n-butyl)silyl, Tri-(1-methylprop-1-yl)silyl, Tri-(2-methylprop-1-yl)silyl, Tri(1,1-Dimethyleth-1-yl)silyl, Tri(2,2-Dimethyleth-1-yl)silyl.

"Trialkylsilylalkinyl" steht für einen über eine Alkinylgruppe gebundenen Trialkylsilylrest.

### Synthese von substituierten Indanylcarboxamiden der allgemeinen Formel (I):

Die Synthese der erfindungsgemäßen Verbindungen ist im Folgenden beschrieben. Ein zentrales Intermediat für die Synthese der erfindungsgemäßen Verbindungen der allgemeinen Formel (I) stellen Indanylamine der Formel (IV) dar, ihre Synthese ist in WO97/31904 und WO2004/069814 ausführlich beschrieben und in Schema 1 vereinfacht dargestellt. Indanylamine der Formel (IV) können beispielweise hergestellt werden durch Hydrierung der entsprechenden Oxime (III), die ihrerseits aus den entsprechenden Ketonen (II) hergestellt werden können. So wird beispielsweise von A. B. Sen et al. (J. Ind. Chem. Soc. 1966, 43, 521) ein analoges Verfahren beschrieben, wobei die Umsetzung von Natrium zu Natriumethanolat als Wasserstoffquelle genutzt wird. Weiterhin beschreiben Sarges et al. (J. Med. Chem. 1973, 16, 1003) ein Verfahren zur Umwandlung eines Ketons in das entsprechende Oxim und die nachfolgende Palladium-katalysierte Hydrierung zu einem Aminhydrochlorid. Außerdem sind Hydrierungen mit Raney-Nickel bekannt (J. Heterocycl. Chem. 1991, 28, 449) sowie mit Borwasserstoffverbindungen (Tetrahedron Lett. 1991, 32, 711). Weiterhin kann die reduktive Aminierung von Indanonen auch durch Umsetzung mit Ammoniumacetat und Natriumcyanoborhydrid erreicht werden. Die Herstellung von substituierten Indanylaminen durch Cyclisierung eines substituierten Phthalimids (VII) ist in WO 2015/113903 beschrieben, wobei dabei ein Palladiumtetraphenylphosphin-Katalysator verwendet wird. Ein optimiertes Herstellungsverfahren zur Synthese von substituierten Indanylaminen, das über die Aminierung eines entsprechenden Indens (VI) und Hydrosilan-vermittelte Reduktion unter Verwendung eines geeigneten mono-, bi- oder polydentaten chiralen Liganden (bevorzugt eines Phoshinliganden wie z.B. (+)-1,2-bis((2S',5S)-2,5-diphenylphospholano)ethan ((S,S')-Ph-BPE) verläuft, ist in WO2024/201469 beschrieben. Das entsprechende substituierte Inden (VI) wird dabei aus dem Keton (II) durch Reduktion zum Alkohol (V) und nachfolgende Eliminierung erhalten (Schema 1). Weitere Methoden zur durch Übergangsmetallkatalysatoren vermittelten reduktiven Aminierung von Indanylketonen wurden von Q. Hu et al. (Materials Chemistry Frontiers, 2023, 7, 2266), J. Gallardo-Donaire (J. Am. Chem. Soc. 2018, 140, 355), T. Kawada et al. (J. Org. Chem. 2022, 87, 8458) sowie in US20010003136 beschrieben. Dabei wurden beispielsweise Wasserstoff, Ameisensäure und Ammoniak als Wasserstoffquellen sowie Cobaltoxid oder Ruthenum- und Iridium-basierte Katalysatorsysteme, wie z.B. Chloro[N-[(1S)-1-(4-methoxy-3,5-dimethyl-2- pyridinyl-κN)-1-phenylethyl]methansulfonamidato-κN][(1,2, 3,4,5-η)-1,2,3,4,5-pentamethyl-2,4-cyclopentadien-1-yl]iridium oder eine Kombination aus Carbonylchlorohydrotris(triphenylphosphin)ruthenium, Natriumhexafluorphosphat und 1,1'-Bis[(11bS)-3,5-dihydro-4H-dinaphtho[2,1-c:1', 2'-e]phosphepin-4-yl]ferrocen, verwendet. Weiterhin können Indanylketone über die Herstellung von Sulfoximinen (VIII) als Zwischenstufe und nachfolgende Reduktion hergestellt werden (vgl. Bioorg. Med. Chem. 2017, 25, 6242; J. Org. Chem. 2010, 75, 5265). Diese Synthesesequenz eignet sich auch zur enantio- oder diastereoselektiven Synhtese von optisch aktiven Indanylaminen in wenigen Reaktionsschritten, deren Herstellung über eine längere Syntheseroute in WO2004/069814 beschrieben worden ist.

Die Kupplug des Indanylamins mit einem substituierten Aminotriazin zur Einführung der oben definierten Gruppen Q-1 bis Q-55 in den erfindungsgemäßen Verbindungen der allgemeinen Formel (I) kann, wie beispielhaft aber nicht einschränkend in Schema 1 dargestellt, durch Reaktion eines gegebenenfalls weiter substituierten Indanylamins (IV) mit einem geeigneten substituierten 2-Amino-4-chlor-1,3,5-triazin (IX) unter Vermittlung einer geeigneten Base (z. B. Diisopropylethylamin, Triethylamin, Kaliumcarbonat, Cäsiumcarbonat) in einem geeigneten Lösemittel (z. B. Acetonitril, Butyronitril, N,N-Dimethylacetamid, N,N-Dimethylformamid) bei Temperaturen zwischen Raumtemperatur und dem Siedepunkt des betreffenden Lösemittels erfolgen. Alternativ können Aminotriazine verwendet werden, die eine geeignete andere Abgangsgruppe tragen, z.B. Trichlormethyl, (C₁-C₄)-Alkylsulfonyl, unsubstituiertes oder substituiertes Phenyl-(C₁-C₄)-Alkylsulfonyl, (C₁-C₄)-Haloalkylsulfonyl. Die Umsetzung dieser Aminotriazine mit einem gegebenenfalls weiter substituierten Indanylamin (IV) erfolgt vorzugsweise basenkatalysiert in einem geeigneten inerten organischen Lösemittel (z.B. Tetrahydrofuran, Dioxan, Acetonitril, N,N-Dimethylformamid, Methanol oder Ethanol) bei Temperaturen zwischen -10 °C und dem Siedepunkt des jeweiligen Lösemittels. Als Basen bzw. basische Katalysatoren eignen sich Alkalihydroxide, Alkalicarbonate, Erdalkalicarbonate oder organische Basen wie Triethylamin, DIisopropylethylamin oder 1,8-Diazabicyclo[5.4.0]undec-7-en (DBU). Die entsprechenden Verfahren sind im Prinzip aus der Literatur bekannt (vgl. Comprehensive Heterocyclic Chemistry, A.R. Katritzky, C. W. rees, Pergamon Press, Oxford, New York, 1984, Vol. 3, Part 2B, S. 482). Im folgenden Schema 1 werden zur besseren Übersicht beispielhaft, aber nicht einschränkend die Synthesewege zur Herstellung von substituierten Indanylaminotriazinen der allgemeinen Formel (I.1) beschrieben, wobei die die Substituenten Q, R³, R⁷ und R⁹ die weiter oben beschriebenen Bedeutungen haben und wobei R⁴, R⁵, R⁶, R⁸ und R¹⁰ beispielhaft, aber nicht einschränkend für Wasserstoff stehen.

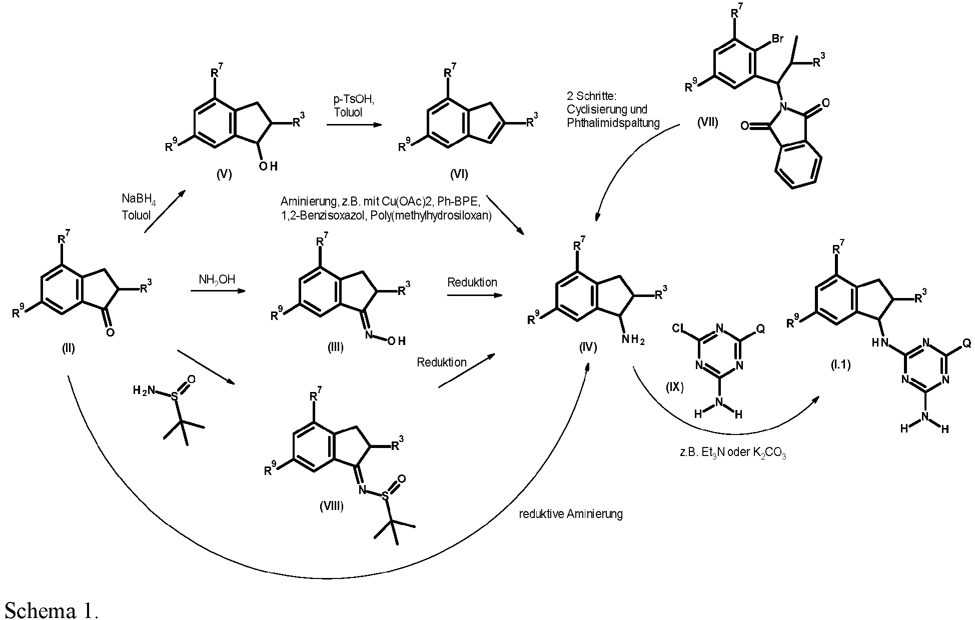

Ausgewählte detaillierte Synthesebeispiele für erfindungsgemäße Verbindungen der allgemeinen Formel (I) sind im Folgenden aufgeführt. Die angegebenen Beispielnummern entsprechen den in den nachstehenden Tabellen I.1 und I.2 genannten Nummerierungen und bezeichnen jeweils die in Tabellen 1.1 und I.2 genannten Verbindungen. Die ¹H-NMR-, ¹³C-NMR- und ¹⁹F-NMR-spektroskopischen Daten, die für die in den nachfolgenden Abschnitten beschriebenen chemischen Beispiele angegeben sind, (400 oder 600 MHz bei ¹H-NMR und 150 MHz bei ¹³C-NMR und 375 MHz bei ¹⁹F-NMR, Lösungsmittel CDCl₃, CD₃OD oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Bei Diastereomerengemischen werden entweder die jeweils signifikanten Signale beider Diastereomere oder das charakteristische Signal des Hauptdiastereomers angegeben. Die verwendeten Abkürzungen für chemische Gruppen haben beispielsweise die nachfolgenden Bedeutungen: Me = CH₃, Et oder Ethyl = CH₂CH₃, t-Hex = C(CH₃)₂CH(CH₃)₂, t-Bu = C(CH₃)₃, n-Bu = unverzweigtes Butyl, n-Pr oder Prop-1-yl = unverzweigtes Propyl, i-Pr oder Prop-2-yl = verzweigtes Propyl, c-Pr = Cyclopropyl, c-Bu = Cyclobutyl, c-Hex = Cyclohexyl.

### Synthesebeispiele:

### Nr. I.1-14: N⁴-[(1R,2S)-2,6-Dimethyl-2,3-dihydro-1H-inden-1-yl]-N²,N²-dimethyl-1,3,5-triazin-2,4,6-triamin

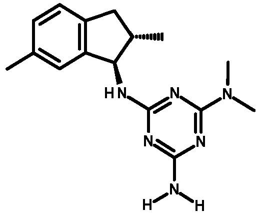

2,6-Dimethylindan-1-on (68.4 mmol) und Hydroxylaminhydrochlorid (136.8 mmol) wurden in einem Rundkolben in Ethanol gelöst, das resultierende Reaktionsgemisch wurde auf eine Temperatur von 60 °C erwärmt und mit einer Lösung von Natriumacetat (205.2 mmol) in Wasser versetzt. Das erhaltene Reaktionsgemisch wurde danach 90 Minuten lang unter Rückflußbedingungen gerührt, auf Raumtemperatur abgekühlt und der resultierende Feststoff abfiltriert. Durch Waschen des erhaltenen Rohproduktes mit Wasser und anschließende Filtration wurde 2,6-Dimethylindan-1-yloxim (81% der Theorie, 90% Reinheit) erhalten. 2,6-Dimethylindan-1-yloxim (58.9 mmol) und Palladium auf Kohle (10%) wurden mit Methanol und Essigsäure versetzt und 15 Minuten unter Argon gerührt. Danach wurde Wasserstoff eingeleitet und das Reaktionsgemisch unter Wasserstoffatmosphäre gerührt. Nach vollständiger Umsetzung wurde der Katalysator über Celite abfiltriert und das Lösemittel unter vermindertem Druck entfernt. Danach erfolgte die Zugabe von HCl in Ethanol (6N), Rühren für 10 Minuten, erneutes Einengen unter vermindertem Druck, Waschen mit einem Gemisch aus Diethylether und Aceton (10:1) und erneutes Abfiltrieren. Dadurch wurde 2,6-Dimethyl-2,3-dihydro-1H-inden-1-ylaminhydrochlorid als Diastereomerengemisch erhalten (85% der Theorie, Reinheit 95%). Im nächsten Schritt wurde 2,6-Dimethyl-2,3-dihydro-1H-inden-1-ylaminhydrochlorid (107.3 mmol) mit wäßriger NaOH-Lösung (2N) versetzt und mit Essigester mehrfach extrahiert. Die vereinigten organischen Phasen wurden über Natriumsulfat getrocknet, unter vermindertem Druck eingeengt und säulenchromatographisch gereinigt (Gradient Essigester/Triethylamin 100:1). Dabei wurden *trans*-2,6-Dimethyl-2,3-dihydro-1H-inden-1-ylamin und *cis*-2,6-Dimethyl-2,3-dihydro-1H-inden-1-ylamin getrennt. Novozym 435 (Aldrich Corp., 1.0 g) wurde danach zu einer Lösung von *trans*-2,6-Dimethyl-2,3-dihydro-1H-inden-1-ylamin (44.8 mmol) und Methyl-2-methoxyacetat (48.4 mmol) in *tert-*Butylmethylether gegeben. Das resultierende Reaktionsgemisch wurde 2 h lang unter Rückflußbedingungen gerührt, erneut mit Novozym 435 (0.5 g) versetzt und weitere 2 h lang rückflussiert. Nach dem Abkühlen auf Raumtemperatur wurde Dichlormethan zugegeben und der Biokatalysator abfiltriert. Das Filtrat wurde über Magnesiumsulfat getrocknet, eingeengt und mit wenig Dichlormethan wieder aufgenommen. Danach erfolgte die Zugabe von HCl in Ethanol (8N). Der resultierende Feststoff [(1S,2R)-2,6-Dimethyl-2,3-dihydro-1H-inden-1-ylaminhydrochlorid] wurde abfiltriert und das Filtrat {N-[(1R,2S)-2,6-Dimethyl-2,3-dihydro-1H-inden-1-yl]-2-methoxyacetamid} wurde unter vermindertem Druck eingeengt. Konzentrierte Salzsäure wurde im folgenden Schritt zu einer Lösung von N-[(1R,2S)-2,6-Dimethyl-2,3-dihydro-1H-inden-1-yl]-2-methoxyacetamid in Ethanol und Wasser gegeben und das resultierende Reaktionsgemisch wurde 12 h lang unter Rückflußbedingungen gerührt. Nach dem Abkühlen auf Raumtemperatur erfolgte die Zugabe von Essigester. Durch Abfiltrieren des ungelösten Rückstandes wurde (1R,2S)-2,6-Dimethyl-2,3-dihydro-1H-inden-1-ylaminhydrochlorid erhalten. Zu einer gerührten Suspension von (1R,2S)-2,6-Dimethyl-2,3-dihydro-1H-inden-1-ylaminhydrochlorid (61 mg, 0.31 mmol) in N,N-Dimethylacetamid (5 ml) unter Argon in einem Mikrowellengefäß wurde 6-Chlor-N²,N²-dimethyl-1,3,5-triazin-2,4-diamin (54 mg, 0.31 mmol) gegeben, gefolgt von der Zugabe von Triethylamin (0.09 ml, 0.62 mmol), woraufhin die Reaktionsmischung homogen wurde. Das Mikrowellengefäß wurde unter Argon mit einer geeigneten Schutzkappe verschlossen und 90 Minuten bei einer Temperatur von 160 °C unter Mikrowellenbedingungen gerührt. Die Reaktionsmischung wurde nach dem Abkühlen auf Raumtemperatur in eisgekühltes Wasser eingetropft und der resultierende Feststoff wurde abfiltriert, mit wenig Eiswasser gewaschen und getrocknet. Das erhaltene Rohprodukt wurde durch Säulenchromatographie über Kieselgel unter Verwendung eines Gradienten aus Heptan und Essigester gereinigt, um N⁴-[(1R,2S)-2,6-Dimethyl-2,3-dihydro-1H-inden-1-yl]-N²,N²-dimethyl-1,3,5-triazin-2,4,6-triamin (I.1-14) (26 mg, 28 % Ausbeute der Theorie) in Form eines farblosen Feststoffs zu ergeben. ¹H NMR (400 MHz, CDCl₃) δ (ppm) = 7.13 - 6.92 (m, 3H), 5.34 - 5.19 (m, 2H), 5.04 - 4.80 (br. m, 2H), 3.12 (s, 6H), 3.07 - 2.98 (m, 1H), 2.55 - 2.45 (m, 1H), 2.30 (s, 3H), 2.28 - 2.19 (m, 1H), 1.27 (d, *J =* 6.7 Hz, 3H).

### Nr. I.1-34: N-(2,6-Dimethyl-2,3-dihydro-1H-inden-1-yl)-6-(ethylsulfanyl)-1,3,5-triazin-2,4-diamin

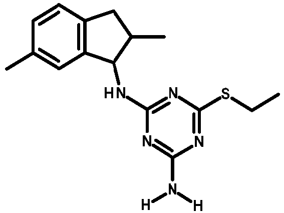

2,6-Dimethylindan-1-on (68.4 mmol) und Hydroxylaminhydrochlorid (136.8 mmol) wurden in einem Rundkolben in Ethanol gelöst, das resultierende Reaktionsgemisch wurde auf eine Temperatur von 60 °C erwärmt und mit einer Lösung von Natriumacetat (205.2 mmol) in Wasser versetzt. Das erhaltene Reaktionsgemisch wurde danach 90 Minuten lang unter Rückflußbedingungen gerührt, auf Raumtemperatur abgekühlt und der resultierende Feststoff abfiltriert. Durch Waschen des erhaltenen Rohproduktes mit Wasser und anschließende Filtration wurde 2,6-Dimethylindan-1-yloxim (81% der Theorie, 90% Reinheit) erhalten. 2,6-Dimethylindan-1-yloxim (58.9 mmol) und Palladium auf Kohle (10%) wurden mit Methanol und Essigsäure versetzt und 15 Minuten unter Argon gerührt. Danach wurde Wasserstoff eingeleitet und das Reaktionsgemisch unter Wasserstoffatmosphäre gerührt. Nach vollständiger Umsetzung wurde der Katalysator über Celite abfiltriert und das Lösemittel unter vermindertem Druck entfernt. Danach erfolgte die Zugabe von HCl in Ethanol (6N), Rühren für 10 Minuten, erneutes Einengen unter vermindertem Druck, Waschen mit einem Gemisch aus Diethylether und Aceton (10:1) und erneutes Abfiltrieren. Dadurch wurde 2,6-Dimethyl-2,3-dihydro-1H-inden-1-ylaminhydrochlorid als Diastereomerengemisch erhalten (85% der Theorie, Reinheit 95%). Zu einer gerührten Suspension von 2,6-Dimethyl-2,3-dihydro-1H-inden-1-ylaminhydrochlorid (61 mg, 0.31 mmol) in N,N-Dimethylacetamid (5 ml) unter Argon in einem Mikrowellengefäß wurde 6-Chlor-4-ethylsulfanyl-1,3,5-triazin-2-amin (59 mg, 0.31 mmol) gegeben, gefolgt von der Zugabe von Triethylamin (0.09 ml, 0.62 mmol), woraufhin die Reaktionsmischung homogen wurde. Das Mikrowellengefäß wurde unter Argon mit einer geeigneten Schutzkappe verschlossen und 90 Minuten bei einer Temperatur von 160 °C unter Mikrowellenbedingungen gerührt. Die Reaktionsmischung wurde nach dem Abkühlen auf Raumtemperatur in eisgekühltes Wasser eingetropft und der resultierende Feststoff wurde abfiltriert, mit wenig Eiswasser gewaschen und getrocknet. Das erhaltene Rohprodukt wurde durch Säulenchromatographie über Kieselgel unter Verwendung eines Gradienten aus Heptan und Essigester gereinigt, um N-(2,6-Dimethyl-2,3-dihydro-1H-inden-1-yl)-6-(ethylsulfanyl)-1,3,5-triazin-2,4-diamin (I.1-34) (64 mg, 65 % Ausbeute der Theorie) in Form eines farblosen Feststoffs zu ergeben. ¹H NMR (400 MHz, CDCl₃) δ (ppm) = 7.18 - 6.96 (m, 3H), 5.70 - 5.19 (m, 2H), 5.04 - 4.80 (br. m, 2H), 3.16 - 2.96 (m, 3H), 2.90 - 2.78 (m, 1H), 2.64 - 2.46 (m, 1H), 2.33 (s, 3H), 1.43 - 1.32 (m, 3H), 0.97 (d, *J =* 7.0 Hz, 3H).

### Nr. I.1-141: N-(2,6-Diethyl-2,3-dihydro-1H-inden-1-yl)-6-methoxy-1,3,5-triazin-2,4-diamin

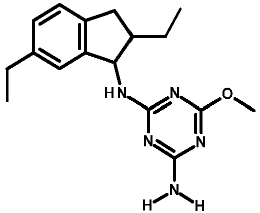

2,6-Diethylindan-1-on (34.2 mmol) und Hydroxylaminhydrochlorid (68.4 mmol) wurden in einem Rundkolben in Ethanol gelöst, das resultierende Reaktionsgemisch wurde auf eine Temperatur von 60 °C erwärmt und mit einer Lösung von Natriumacetat (102.6 mmol) in Wasser versetzt. Das erhaltene Reaktionsgemisch wurde danach 90 Minuten lang unter Rückflußbedingungen gerührt, auf Raumtemperatur abgekühlt und der resultierende Feststoff abfiltriert. Durch Waschen des erhaltenen Rohproduktes mit Wasser und anschließende Filtration wurde 2,6-Diethylindan-1-yloxim (81% der Theorie, 90% Reinheit) erhalten. 2,6-Diethylindan-1-yloxim (29.5 mmol) und Palladium auf Kohle (10%) wurden mit Methanol und Essigsäure versetzt und 15 Minuten unter Argon gerührt. Danach wurde Wasserstoff eingeleitet und das Reaktionsgemisch unter Wasserstoffatmosphäre gerührt. Nach vollständiger Umsetzung wurde der Katalysator über Celite abfiltriert und das Lösemittel unter vermindertem Druck entfernt. Danach erfolgte die Zugabe von HCl in Ethanol (6N), Rühren für 10 Minuten, erneutes Einengen unter vermindertem Druck, Waschen mit einem Gemisch aus Diethylether und Aceton (10:1) und erneutes Abfiltrieren. Dadurch wurde 2,6-Diethyl-2,3-dihydro-1H-inden-1-ylaminhydrochlorid als Diastereomerengemisch erhalten (78% der Theorie, Reinheit 92%). Zu einer gerührten Suspension von 2,6-Diethyl-2,3-dihydro-1H-inden-1-ylaminhydrochlorid (120 mg, 0.52 mmol) in abs. Butyronitril (4 ml) unter Argon in einem Mikrowellengefäß wurde 6-Chlor-4-methoxy-1,3,5-triazin-2-amin (93 mg, 0.58 mmol) gegeben, gefolgt von der Zugabe von Cäsiumcarbonat (379 mg, 1.16 mmol). Das Mikrowellengefäß wurde mit Argon gespült und unter Argon mit einer geeigneten Schutzkappe verschlossen und 120 Minuten bei einer Temperatur von 90 °C unter Mikrowellenbedingungen gerührt. Die Reaktionsmischung wurde nach dem Abkühlen auf Raumtemperatur abfiltriert, unter vermindertem Druck eingeengt und durch Säulenchromatographie über Kieselgel unter Verwendung eines Gradienten aus Heptan und Essigester gereinigt, um N-(2,6-Diethyl-2,3-dihydro-1H-inden-1-yl)-6-methoxy-1,3,5-triazin-2,4-diamin (I.1-141) (71 mg, 43 % Ausbeute der Theorie) in Form eines farblosen Feststoffs zu ergeben. ¹H NMR (400 MHz, CDCl₃) δ (ppm) = 7.16 - 7.02 (m, 3H), 5.68 - 5.58 / 5.28 - 5.20 (m, 1H), 5.43 - 5.28 (m, 1H), 5.12 - 4.98 (br. m, 2H), 3.96 - 3.87 (m, 3H), 3.09 - 2.93 (m, 1H), 2.72 - 2.49 (m, 3H), 1.60 - 1.50 (m, 1H), 1.29 - 1.17 (m, 5H), 1.02-0.98 / 0.96 - 0.92 (t, 3H).

### Nr. I.1-170: N-(2-Ethyl-6-methyl-2,3-dihydro-1H-inden-1-yl)-6-methoxy-1,3,5-triazin-2,4-diamin

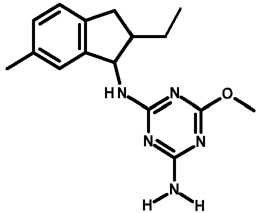

2-Ethyl-6-methylindan-1-on (34.2 mmol) und Hydroxylaminhydrochlorid (68.4 mmol) wurden in einem Rundkolben in Ethanol gelöst, das resultierende Reaktionsgemisch wurde auf eine Temperatur von 60 °C erwärmt und mit einer Lösung von Natriumacetat (102.6 mmol) in Wasser versetzt. Das erhaltene Reaktionsgemisch wurde danach 90 Minuten lang unter Rückflußbedingungen gerührt, auf Raumtemperatur abgekühlt und der resultierende Feststoff abfiltriert. Durch Waschen des erhaltenen Rohproduktes mit Wasser und anschließende Filtration wurde 2-Ethyl-6-methylindan-1-yloxim (85% der Theorie, 90% Reinheit) erhalten. 2,6-Diethylindan-1-yloxim (29.5 mmol) und Palladium auf Kohle (10%) wurden mit Methanol und Essigsäure versetzt und 15 Minuten unter Argon gerührt. Danach wurde Wasserstoff eingeleitet und das Reaktionsgemisch unter Wasserstoffatmosphäre gerührt. Nach vollständiger Umsetzung wurde der Katalysator über Celite abfiltriert und das Lösemittel unter vermindertem Druck entfernt. Danach erfolgte die Zugabe von HCl in Ethanol (6N), Rühren für 10 Minuten, erneutes Einengen unter vermindertem Druck, Waschen mit einem Gemisch aus Diethylether und Aceton (10:1) und erneutes Abfiltrieren. Dadurch wurde 2-Ethyl-6-methyl-2,3-dihydro-1H-inden-1-ylaminhydrochlorid als Diastereomerengemisch erhalten (71% der Theorie, Reinheit 94%). Zu einer gerührten Suspension von 2-Ethyl-6-methyl-2,3-dihydro-1H-inden-1-ylaminhydrochlorid (120 mg, 0.56 mmol) in abs. Butyronitril (4 ml) unter Argon wurde in einem Mikrowellengefäß 6-Chlor-4-methoxy-1,3,5-triazin-2-amin (99 mg, 0.62 mmol) gegeben, gefolgt von der Zugabe von Cäsiumcarbonat (404 mg, 1.24 mmol). Das Mikrowellengefäß wurde mit Argon gespült und unter Argon mit einer geeigneten Schutzkappe verschlossen und 90 Minuten lang bei einer Temperatur von 100 °C unter Mikrowellenbedingungen gerührt. Die Reaktionsmischung wurde nach dem Abkühlen auf Raumtemperatur abfiltriert, unter vermindertem Druck eingeengt und durch Säulenchromatographie über Kieselgel unter Verwendung eines Gradienten aus Heptan und Essigester gereinigt, um N-(2-Ethyl-6-methyl-2,3-dihydro-1H-inden-1-yl)-6-methoxy-1,3,5-triazin-2,4-diamin (I.1-170) (88 mg, 52 % Ausbeute der Theorie) in Form eines farblosen Feststoffs zu ergeben. ¹H NMR (400 MHz, CDCl₃) δ (ppm) = 7.14 - 7.10 (m, 1H), 7.09 - 7.00 (m, 2H), 5.78 - 5.57 / 5.42 - 5.24 (m, 2H), 5.15 - 4.97 (br. m, 2H), 3.92 - 3.86 (m, 3H), 3.08 - 3.02 / 3.00 - 2.94 (m, 1H), 2.72 - 2.63 / 2.56 - 2.48 (m, 1H), 2.30 (s, 3H), 2.13 - 2.087 (m, 1H), 1.88 - 1.78 (m, 2H), 1.59 - 1.50 (m, 1H), 1.02-0.98 / 0.96 - 0.92 (t, 3H).

In Analogie zu den oben angeführten und an entsprechender Stelle rezitierten Herstellungsbeispielen
und unter Berücksichtigung der allgemeinen Angaben zur Herstellung von substituierten Indanylcarboxamiden erhält man die nachfolgend genannten Verbindungen. Wenn in den Tabellen 1 und 2 ein Strukturelement durch eine Strukturformel definiert ist, welches eine gestrichelte Linie enthält, so bedeutet diese gestrichelte Linie, daß an dieser Position die betreffende Gruppe mit dem Rest des Moleküls verbunden ist. Die entsprechende stereochemische Konfiguration an den Stereozentren C-1 und C-2 der Indanyleinheit (siehe untere Formeln I.1 und I.2) wird durch die Bezeichnungen "Rac" für ein racemisches Gemisch der Enantiomere oder Diastereomere oder "R" und "S" entsprechend der Cahn-Ingold-Prelog-Regeln bei definierter Stereochemie, beziehungsweise durch "Ent-1" für das Enantiomer mit der niedrigeren Retentionszeit in der chiralen HPLC-Analytik oder chiralen präparativen SFC und "Ent-2" für das Enantiomer mit der höheren Retentionszeit in der chiralen HPLC-Analytik oder chiralen präparativen SFC gekennzeichnet. In der nachfolgenden Tabelle stehen die Bezeichnungen 1-Methyleth-1-yl, 1-Methylethyl, Prop-2-yl beispielsweise für eine iso-Propylgruppe, und die unterschiedlichen Bezeichnungen für die gleiche Gruppe sind fachlich geläufig.

Tabelle I.1: Bevorzugte Verbindungen der Formel (I.1) sind die Verbindungen I.1-1 bis I.1-174, worin Q die in der jeweiligen Zeile angegebenen Bedeutungen der Tabelle 1 hat. Die Verbindungen I.1-1 bis I.1-163 der Tabelle I.1 sind somit durch die Bedeutung der jeweiligen Einträge für die Substituenten R³, R⁷, R⁹ und Q der Tabelle 1 definiert, wobei R⁴, R⁵, R⁶, R⁸ und R¹⁰ für Wasserstoff stehen.

**Tabelle I.1:**

| 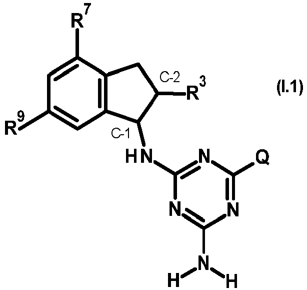 | | | | | | |
|---|---|---|---|---|---|---|
| **Verbindung** | **R³** | **Q** | **C-1** | **C-2** | **R⁷** | **R⁹** |
| I.1-1 | CH₃ | Q-1 | Rac | Rac | H | CH₃ |
| I.1-2 | CH₃ | Q-1 | R | S | H | CH₃ |
| I.1-3 | CH₃ | Q-1 | S | R | H | CH₃ |
| I.1-4 | CH₃ | Q-2 | Rac | Rac | H | CH₃ |
| I.1-5 | CH₃ | Q-2 | R | S | H | CH₃ |
| I.1-6 | CH₃ | Q-2 | S | R | H | CH₃ |
| I.1-7 | CH₃ | Q-3 | Rac | Rac | H | CH₃ |
| I.1-8 | CH₃ | Q-3 | R | S | H | CH₃ |
| I.1-9 | CH₃ | Q-3 | S | R | H | CH₃ |
| I.1-10 | CH₃ | Q-4 | Rac | Rac | H | CH₃ |
| I.1-11 | CH₃ | Q-4 | R | S | H | CH₃ |
| I.1-12 | CH₃ | Q-4 | S | R | H | CH₃ |
| I.1-13 | CH₃ | Q-11 | Rac | Rac | H | CH₃ |
| I.1-14 | CH₃ | Q-11 | R | S | H | CH₃ |
| I.1-15 | CH₃ | Q-11 | S | R | H | CH₃ |
| I.1-16 | CH₃ | Q-22 | R | S | H | CH₃ |
| I.1-17 | CH₃ | Q-22 | S | R | H | CH₃ |
| I.1-18 | CH₃ | Q-22 | Rac | Rac | H | CH₃ |
| I.1-19 | CH₃ | Q-21 | R | S | H | CH₃ |
| I.1-20 | CH₃ | Q-21 | S | R | H | CH₃ |
| I.1-21 | CH₃ | Q-21 | Rac | Rac | H | CH₃ |
| I.1-22 | CH₃ | Q-23 | R | S | H | CH₃ |
| I.1-23 | CH₃ | Q-23 | S | R | H | CH₃ |
| I.1-24 | CH₃ | Q-23 | Rac | Rac | H | CH₃ |
| I.1-25 | CH₃ | Q-31 | R | S | H | CH₃ |
| I.1-26 | CH₃ | Q-31 | S | R | H | CH₃ |
| I.1-27 | CH₃ | Q-31 | Rac | Rac | H | CH₃ |
| I.1-28 | CH₃ | Q-36 | R | S | H | CH₃ |
| I.1-29 | CH₃ | Q-36 | S | R | H | CH₃ |
| I.1-30 | CH₃ | Q-36 | Rac | Rac | H | CH₃ |

| **Verbindung** | **R³** | **Q** | **C-1** | **C-2** | **R⁷** | **R⁹** |
|---|---|---|---|---|---|---|
| I.1-31 | CH₃ | Q-44 | Rac | Rac | H | CH₃ |
| I.1-32 | CH₃ | Q-44 | R | S | H | CH₃ |
| I.1-33 | CH₃ | Q-44 | S | R | H | CH₃ |
| I.1-34 | CH₃ | Q-45 | Rac | Rac | H | CH₃ |
| I.1-35 | CH₃ | Q-45 | R | S | H | CH₃ |
| I.1-36 | CH₃ | Q-45 | S | R | H | CH₃ |
| I.1-37 | CH₃ | Q-1 | Rac | Rac | H | OCH₃ |
| I.1-38 | CH₃ | Q-1 | Rac | Rac | H | Cl |
| I.1-39 | CH₃ | Q-1 | Rac | Rac | H | Cyclopropyl |
| I.1-40 | CH₃ | Q-1 | Rac | Rac | H | Prop-2-yl |
| I.1-41 | CH₃ | Q-1 | Rac | Rac | H | Ethoxy |
| I.1-42 | CH₃ | Q-1 | Rac | Rac | H | Ethyl |
| I.1-43 | CH₃ | Q-1 | Rac | Rac | H | Br |
| I.1-44 | CH₃ | Q-1 | Rac | Rac | H | F |
| I.1-45 | CH₃ | Q-1 | Rac | Rac | CH₃ | CH₃ |
| I.1-46 | CH₃ | Q-2 | Rac | Rac | H | OCH₃ |
| I.1-47 | CH₃ | Q-2 | Rac | Rac | H | Cl |
| I.1-48 | CH₃ | Q-2 | Rac | Rac | H | Cyclopropyl |
| I.1-49 | CH₃ | Q-2 | Rac | Rac | H | Prop-2-yl |
| I.1-50 | CH₃ | Q-2 | Rac | Rac | H | Ethoxy |
| I.1-51 | CH₃ | Q-2 | Rac | Rac | H | Ethyl |
| I.1-52 | CH₃ | Q-2 | Rac | Rac | H | Br |
| I.1-53 | CH₃ | Q-2 | Rac | Rac | H | F |
| I.1-54 | CH₃ | Q-2 | Rac | Rac | CH₃ | CH₃ |
| I.1-55 | CH₃ | Q-3 | Rac | Rac | H | OCH₃ |
| I.1-56 | CH₃ | Q-3 | Rac | Rac | H | Cl |
| I.1-57 | CH₃ | Q-3 | Rac | Rac | H | Cyclopropyl |
| I.1-58 | CH₃ | Q-3 | Rac | Rac | H | Prop-2-yl |
| I.1-59 | CH₃ | Q-3 | Rac | Rac | H | Ethoxy |
| I.1-60 | CH₃ | Q-3 | Rac | Rac | H | Ethyl |
| I.1-61 | CH₃ | Q-3 | Rac | Rac | H | Br |
| I.1-62 | CH₃ | Q-3 | Rac | Rac | H | F |
| I.1-63 | CH₃ | Q-3 | Rac | Rac | CH₃ | CH₃ |
| I.1-64 | CH₃ | Q-4 | Rac | Rac | H | OCH₃ |
| I.1-65 | CH₃ | Q-4 | Rac | Rac | H | Cl |
| I.1-66 | CH₃ | Q-4 | Rac | Rac | H | Cyclopropyl |
| I.1-67 | CH₃ | Q-4 | Rac | Rac | H | Prop-2-yl |
| I.1-68 | CH₃ | Q-4 | Rac | Rac | H | Ethoxy |
| I.1-69 | CH₃ | Q-4 | Rac | Rac | H | Ethyl |
| I.1-70 | CH₃ | Q-4 | Rac | Rac | H | Br |
| I.1-71 | CH₃ | Q-4 | Rac | Rac | H | F |
| I.1-72 | CH₃ | Q-4 | Rac | Rac | CH₃ | CH₃ |
| I.1-73 | CH₃ | Q-11 | Rac | Rac | H | OCH₃ |
| I.1-74 | CH₃ | Q-11 | Rac | Rac | H | Cl |
| I.1-75 | CH₃ | Q-11 | Rac | Rac | H | Cyclopropyl |
| I.1-76 | CH₃ | Q-11 | Rac | Rac | H | Prop-2-yl |
| I.1-77 | CH₃ | Q-11 | Rac | Rac | H | Ethoxy |
| I.1-78 | CH₃ | Q-11 | Rac | Rac | H | Ethyl |
| I.1-79 | CH₃ | Q-11 | Rac | Rac | H | Br |
| I.1-80 | CH₃ | Q-11 | Rac | Rac | H | F |
| I.1-81 | CH₃ | Q-11 | Rac | Rac | CH₃ | CH₃ |
| I.1-82 | CH₃ | Q-21 | Rac | Rac | H | OCH₃ |
| I.1-83 | CH₃ | Q-21 | Rac | Rac | H | Cl |
| I.1-84 | CH₃ | Q-21 | Rac | Rac | H | Cyclopropyl |
| I.1-85 | CH₃ | Q-21 | Rac | Rac | H | Prop-2-yl |
| I.1-86 | CH₃ | Q-21 | Rac | Rac | H | Ethoxy |
| I.1-87 | CH₃ | Q-21 | Rac | Rac | H | Ethyl |
| I.1-88 | CH₃ | Q-21 | Rac | Rac | H | Br |
| I.1-89 | CH₃ | Q-21 | Rac | Rac | H | F |
| I.1-90 | CH₃ | Q-21 | Rac | Rac | CH₃ | CH₃ |
| I.1-91 | CH₃ | Q-22 | Rac | Rac | H | OCH₃ |
| I.1-92 | CH₃ | Q-22 | Rac | Rac | H | Cl |
| I.1-93 | CH₃ | Q-22 | Rac | Rac | H | Cyclopropyl |
| I.1-94 | CH₃ | Q-22 | Rac | Rac | H | Prop-2-yl |
| I.1-95 | CH₃ | Q-22 | Rac | Rac | H | Ethoxy |
| I.1-96 | CH₃ | Q-22 | Rac | Rac | H | Ethyl |
| I.1-97 | CH₃ | Q-22 | Rac | Rac | H | Br |
| I.1-98 | CH₃ | Q-22 | Rac | Rac | H | F |
| I.1-99 | CH₃ | Q-22 | Rac | Rac | CH₃ | CH₃ |
| I.1-100 | CH₃ | Q-23 | Rac | Rac | H | OCH₃ |
| I.1-101 | CH₃ | Q-23 | Rac | Rac | H | Cl |
| I.1-102 | CH₃ | Q-23 | Rac | Rac | H | Cyclopropyl |
| I.1-103 | CH₃ | Q-23 | Rac | Rac | H | Prop-2-yl |
| I.1-104 | CH₃ | Q-23 | Rac | Rac | H | Ethoxy |
| I.1-105 | CH₃ | Q-23 | Rac | Rac | H | Ethyl |
| I.1-106 | CH₃ | Q-23 | Rac | Rac | H | Br |
| I.1-107 | CH₃ | Q-23 | Rac | Rac | H | F |
| I.1-108 | CH₃ | Q-23 | Rac | Rac | CH₃ | CH₃ |
| I.1-109 | CH₃ | Q-31 | Rac | Rac | H | OCH₃ |
| I.1-110 | CH₃ | Q-31 | Rac | Rac | H | Cl |
| I.1-111 | CH₃ | Q-31 | Rac | Rac | H | Cyclopropyl |
| I.1-112 | CH₃ | Q-31 | Rac | Rac | H | Prop-2-yl |
| I.1-113 | CH₃ | Q-31 | Rac | Rac | H | Ethoxy |
| I.1-114 | CH₃ | Q-31 | Rac | Rac | H | Ethyl |
| I.1-115 | CH₃ | Q-31 | Rac | Rac | H | Br |
| I.1-116 | CH₃ | Q-31 | Rac | Rac | H | F |
| I.1-117 | CH₃ | Q-31 | Rac | Rac | CH₃ | CH₃ |
| I.1-118 | CH₃ | Q-36 | Rac | Rac | H | OCH₃ |
| I.1-119 | CH₃ | Q-36 | Rac | Rac | H | Cl |
| I.1-110 | CH₃ | Q-36 | Rac | Rac | H | Cyclopropyl |
| I.1-111 | CH₃ | Q-36 | Rac | Rac | H | Prop-2-yl |
| I.1-112 | CH₃ | Q-36 | Rac | Rac | H | Ethoxy |
| I.1-113 | CH₃ | Q-36 | Rac | Rac | H | Ethyl |
| I.1-114 | CH₃ | Q-36 | Rac | Rac | H | Br |
| I.1-115 | CH₃ | Q-36 | Rac | Rac | H | F |
| I.1-116 | CH₃ | Q-36 | Rac | Rac | CH₃ | CH₃ |
| I.1-117 | CH₃ | Q-44 | Rac | Rac | H | OCH₃ |
| I.1-118 | CH₃ | Q-44 | Rac | Rac | H | Cl |
| I.1-119 | CH₃ | Q-44 | Rac | Rac | H | Cyclopropyl |
| I.1-120 | CH₃ | Q-44 | Rac | Rac | H | Prop-2-yl |
| I.1-121 | CH₃ | Q-44 | Rac | Rac | H | Ethoxy |
| I.1-122 | CH₃ | Q-44 | Rac | Rac | H | Ethyl |
| I.1-123 | CH₃ | Q-44 | Rac | Rac | H | Br |
| I.1-124 | CH₃ | Q-44 | Rac | Rac | H | F |
| I.1-125 | CH₃ | Q-44 | Rac | Rac | CH₃ | CH₃ |
| I.1-126 | CH₃ | Q-45 | Rac | Rac | H | OCH₃ |
| I.1-127 | CH₃ | Q-45 | Rac | Rac | H | Cl |
| I.1-128 | CH₃ | Q-45 | Rac | Rac | H | Cyclopropyl |
| I.1-129 | CH₃ | Q-45 | Rac | Rac | H | Prop-2-yl |
| I.1-130 | CH₃ | Q-45 | Rac | Rac | H | Ethoxy |
| I.1-131 | CH₃ | Q-45 | Rac | Rac | H | Ethyl |
| I.1-132 | CH₃ | Q-45 | Rac | Rac | H | Br |
| I.1-133 | CH₃ | Q-45 | Rac | Rac | H | F |
| I.1-134 | CH₃ | Q-45 | Rac | Rac | CH₃ | CH₃ |
| I.1-135 | Ethyl | Q-1 | Rac | Rac | H | Ethyl |
| I.1-136 | Ethyl | Q-2 | Rac | Rac | H | Ethyl |
| I.1-137 | Ethyl | Q-3 | Rac | Rac | H | Ethyl |
| I.1-138 | Ethyl | Q-4 | Rac | Rac | H | Ethyl |
| I.1-139 | Ethyl | Q-11 | Rac | Rac | H | Ethyl |
| I.1-140 | Ethyl | Q-21 | Rac | Rac | H | Ethyl |
| I.1-141 | Ethyl | Q-22 | Rac | Rac | H | Ethyl |
| I.1-142 | Ethyl | Q-23 | Rac | Rac | H | Ethyl |
| I.1-143 | Ethyl | Q-31 | Rac | Rac | H | Ethyl |
| I.1-144 | Ethyl | Q-44 | Rac | Rac | H | Ethyl |
| I.1-145 | Ethyl | Q-45 | Rac | Rac | H | Ethyl |
| I.1-146 | CH₃ | Q-24 | Rac | Rac | H | CH₃ |
| I.1-147 | CH₃ | Q-24 | R | S | H | CH₃ |
| I.1-148 | CH₃ | Q-24 | S | R | H | CH₃ |
| I.1-149 | CH₃ | Q-38 | Rac | Rac | H | CH₃ |
| I.1-150 | CH₃ | Q-38 | R | S | H | CH₃ |
| I.1-151 | CH₃ | Q-38 | S | R | H | CH₃ |
| I.1-152 | CH₃ | Q-39 | Rac | Rac | H | CH₃ |
| I.1-153 | CH₃ | Q-39 | R | S | H | CH₃ |
| I.1-154 | CH₃ | Q-39 | S | R | H | CH₃ |
| I.1-155 | CH₃ | Q-40 | Rac | Rac | H | CH₃ |
| I.1-156 | CH₃ | Q-40 | R | S | H | CH₃ |
| I.1-157 | CH₃ | Q-40 | S | R | H | CH₃ |
| I.1-158 | CH₃ | Q-41 | Rac | Rac | H | CH₃ |
| I.1-159 | CH₃ | Q-41 | R | S | H | CH₃ |
| I.1-160 | CH₃ | Q-41 | S | R | H | CH₃ |
| I.1-161 | CH₃ | Q-42 | Rac | Rac | H | CH₃ |
| I.1-162 | CH₃ | Q-42 | R | S | H | CH₃ |
| I.1-163 | CH₃ | Q-42 | S | R | H | CH₃ |
| I.1-164 | Ethyl | Q-1 | Rac | Rac | H | CH₃ |
| I.1-165 | Ethyl | Q-2 | Rac | Rac | H | CH₃ |
| I.1-166 | Ethyl | Q-3 | Rac | Rac | H | CH₃ |
| I.1-167 | Ethyl | Q-4 | Rac | Rac | H | CH₃ |
| I.1-168 | Ethyl | Q-11 | Rac | Rac | H | CH₃ |
| I.1-169 | Ethyl | Q-21 | Rac | Rac | H | CH₃ |
| I.1-170 | Ethyl | Q-22 | Rac | Rac | H | CH₃ |
| I.1-171 | Ethyl | Q-23 | Rac | Rac | H | CH₃ |
| I.1-172 | Ethyl | Q-31 | Rac | Rac | H | CH₃ |
| I.1-173 | Ethyl | Q-44 | Rac | Rac | H | CH₃ |
| I.1-174 | Ethyl | Q-45 | Rac | Rac | H | CH₃ |

Bevorzugte erfindungsgemäße Verbindungen der allgemeinen Formel (I) und/oder deren Salze sind die Verbindungen I.1-1, I.1-16, I.1-18, I.1-32, I.1-35, I.1-22, I.1-147, I.1-153, I.1-62, I.1-19 und I.1-99, weiter bevorzugt 1-1, I.1-16, I.1-18, I.1-32, I.1-35, I.1-22, I.1-147, I.1-153 und I.1-62, besonders bevorzugt 1-1, I.1-16, I.1-18, I.1-32 und I.1-35, insbesondere bevorzugt I.1-1, I.1-16, I.1-18, I.1-32 und I.1-35.

Tabelle I.2: Bevorzugte Verbindungen der Formel (I.2) sind die Verbindungen I.2-1 bis I.2-52, worin Q die in der jeweiligen Zeile angegebenen Bedeutungen der Tabelle 1 hat. Die Verbindungen I.2-1 bis I.2-52 der Tabelle I.2 sind somit durch die Bedeutung der jeweiligen Einträge für die Substituenten R³, R⁴, R⁷, R⁹ und Q der Tabelle 2 definiert, wobei R⁵, R⁶, R⁸ und R¹⁰ für Wasserstoff stehen.

**Tabelle I.2:**

| 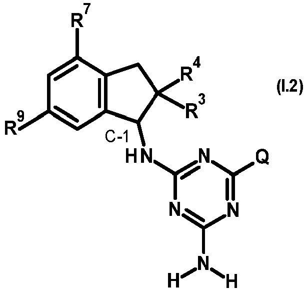 | | | | | | |
|---|---|---|---|---|---|---|
| **Verbindung** | **R³** | **Q** | **C-1** | **R⁴** | **R⁷** | **R⁹** |
| I.2-1 | CH₃ | Q-1 | Rac | CH₃ | H | CH₃ |
| I.2-2 | CH₃ | Q-1 | Ent-1 | CH₃ | H | CH₃ |
| I.2-3 | CH₃ | Q-1 | Ent-2 | CH₃ | H | CH₃ |
| I.2-4 | Et | Q-1 | Rac | Et | H | Et |
| I.2-5 | Et | Q-1 | Ent-1 | Et | H | Et |
| I.2-6 | Et | Q-1 | Ent-2 | Et | H | Et |
| I.2-7 | CH₃ | Q-1 | Rac | CH₃ | H | Et |
| I.2-8 | CH₃ | Q-1 | Ent-1 | CH₃ | H | Et |
| I.2-9 | CH₃ | Q-1 | Ent-2 | CH₃ | H | Et |
| I.2-10 | CH₃ | Q-2 | Rac | CH₃ | H | CH₃ |
| I.2-11 | CH₃ | Q-2 | Ent-1 | CH₃ | H | CH₃ |
| I.2-12 | CH₃ | Q-2 | Ent-2 | CH₃ | H | CH₃ |
| I.2-13 | Et | Q-2 | Rac | Et | H | Et |
| I.2-14 | Et | Q-2 | Ent-1 | Et | H | Et |
| I.2-15 | Et | Q-2 | Ent-2 | Et | H | Et |
| I.2-16 | CH₃ | Q-2 | Rac | CH₃ | H | Et |
| I.2-17 | CH₃ | Q-2 | Ent-1 | CH₃ | H | Et |
| I.2-18 | CH₃ | Q-2 | Ent-2 | CH₃ | H | Et |
| I.2-19 | CH₃ | Q-11 | Rac | CH₃ | H | CH₃ |
| I.2-20 | CH₃ | Q-11 | Ent-1 | CH₃ | H | CH₃ |
| I.2-21 | CH₃ | Q-11 | Ent-2 | CH₃ | H | CH₃ |
| I.2-22 | Et | Q-11 | Rac | Et | H | Et |
| I.2-23 | Et | Q-11 | Ent-1 | Et | H | Et |
| I.2-24 | Et | Q-11 | Ent-2 | Et | H | Et |
| I.2-25 | CH₃ | Q-11 | Rac | CH₃ | H | Et |
| I.2-26 | CH₃ | Q-11 | Ent-1 | CH₃ | H | Et |
| I.2-27 | CH₃ | Q-11 | Ent-2 | CH₃ | H | Et |
| I.2-28 | CH₃ | Q-22 | Rac | CH₃ | H | CH₃ |
| I.2-29 | CH₃ | Q-22 | Ent-1 | CH₃ | H | CH₃ |
| I.2-30 | CH₃ | Q-22 | Ent-2 | CH₃ | H | CH₃ |
| I.2-31 | Et | Q-22 | Rac | Et | H | Et |
| I.2-32 | Et | Q-22 | Ent-1 | Et | H | Et |
| I.2-33 | Et | Q-22 | Ent-2 | Et | H | Et |
| I.2-34 | CH₃ | Q-22 | Rac | CH₃ | H | Et |
| I.2-35 | CH₃ | Q-22 | Ent-1 | CH₃ | H | Et |
| I.2-36 | CH₃ | Q-22 | Ent-2 | CH₃ | H | Et |
| I.2-37 | CH₃ | Q-44 | Ent-1 | CH₃ | H | CH₃ |
| I.2-38 | CH₃ | Q-44 | Ent-2 | CH₃ | H | CH₃ |
| I.2-39 | Et | Q-44 | Rac | Et | H | Et |
| I.2-40 | Et | Q-44 | Ent-1 | Et | H | Et |
| I.2-41 | Et | Q-44 | Ent-2 | Et | H | Et |
| I.2-42 | CH₃ | Q-44 | Rac | CH₃ | H | Et |
| I.2-43 | CH₃ | Q-44 | Ent-1 | CH₃ | H | Et |
| I.2-44 | CH₃ | Q-44 | Ent-2 | CH₃ | H | Et |
| I.2-45 | CH₃ | Q-45 | Ent-1 | CH₃ | H | CH₃ |
| I.2-46 | CH₃ | Q-45 | Ent-2 | CH₃ | H | CH₃ |
| I.2-47 | Et | Q-45 | Rac | Et | H | Et |
| I.2-48 | Et | Q-45 | Ent-1 | Et | H | Et |
| I.2-49 | Et | Q-45 | Ent-2 | Et | H | Et |
| I.2-50 | CH₃ | Q-45 | Rac | CH₃ | H | Et |
| I.2-51 | CH₃ | Q-45 | Ent-1 | CH₃ | H | Et |
| I.2-52 | CH₃ | Q-45 | Ent-2 | CH₃ | H | Et |

### NMR-Daten ausgewählter Beispiele

### a) Klassische Auswertung:

### Beispiel Nr. I.1-19:

¹H NMR (400 MHz, CDCl₃) δ (ppm) = 8.98 (br. s, 1H), 7.13 - 7.01 (m, 3H), 5.34 - 5.19 (m, 2H), 5.04 - 4.80 (br. m, 2H), 3.04 - 2.99 (m, 1H), 2.54 - 2.47 (m, 1H), 2.30 (s, 3H), 2.28 - 2.19 (m, 1H), 1.27 (d, *J =* 6.7 Hz, 3H).

### Beispiel Nr. I.1-87:

¹H NMR (400 MHz, CDCl₃) δ (ppm) = 7.17 - 7.14 (m, 2H), 7.09 - 7.07 (m, 1H), 5.66 - 5.55 (m, 1H), 5.45 - 5.35 (m, 1H), 5.13 - 5.09 (br. s, 1H), 5.01 - 4.98 (br. s, 1H), 4.08 - 4.05 (br. m, 1H), 3.07 - 3.00 (m, 1H), 2.90 - 2.83 / 2.28 - 2.20 (m, 1H), 2.63 - 2.58 (m, 2H), 2.56 - 2.48 (m, 1H), 1.28 - 1.26 / 0.96 - 0.93 (d, 3H), 1.23 - 1.18 (m, 3H).

### Beispiel Nr. I.1-96:

¹H NMR (400 MHz, CDCl₃) δ (ppm) = 7.15 - 7.04 (m, 3H), 5.67 - 5.62 / 5.58 - 5.54 (m, 1H), 5.48 - 5.45 / 5.14 - 5.10 (m, 1H), 5.34 - 5.28 / 5.25 - 5.19 (m, 1H), 5.00 - 4.95 (br. m, 1H), 3.93 - 3.87 (m, 3H), 3.07 - 3.00 (m, 1H), 2.90 - 2.83 / 2.28 - 2.20 (m, 1H), 2.63 - 2.58 (m, 2H), 2.56 - 2.48 (m, 1H), 1.28 - 1.26 / 0.96 - 0.93 (d, 3H), 1.23 - 1.18 (m, 3H).

### Beispiel Nr. I.1-105:

¹H NMR (400 MHz, CDCl₃) δ (ppm) = 7.14 - 7.04 (m, 3H), 5.65 - 5.53 (m, 1H), 5.31 - 5.22 (m, 1H), 5.09 - 5.02 (br. m, 1H), 4.94 - 4.89 (m, 1H), 4.42 - 4.19 (m, 2H), 3.08 - 3.02 (m, 1H), 2.92 - 2.83 / 2.30 - 2.23 (m, 1H), 2.63 - 2.58 (m, 2H), 2.57 - 2.49 (m, 1H), 1.42 - 1.33 (m, 3H), 1.30 - 1.18 (m, 3H), 0.95-0.93 / 0.88 - 0.86 (d, 3H).

### Beispiel Nr. I.1-147:

¹H NMR (400 MHz, CDCl₃) δ (ppm) = 7.08 - 7.01 (m, 3H), 5.27 - 5.14 (m, 3H), 4.98 - 4.94 (br. s, 1H), 4.87 - 4.83 (br. s, 1H), 3.05 - 2.99 (m, 1H), 2.54 - 2.48 (m, 1H), 2.31 (s, 3H), 2.28 - 2.19 (m, 1H), 1.34 - 1.33 (d, 6H), 1.28 - 1.26 (d, 3H).

### Beispiel Nr. I.1-153:

¹H NMR (400 MHz, CDCl₃) δ (ppm) = 7.08 - 7.02 (m, 3H), 5.34 - 5.23 (m, 2H), 5.21 - 5.18 (m, 1H), 4.99 - 4.95 (br. s, 1H), 4.89 - 4.85 (br. s, 1H), 3.62 - 3.56 (m, 1H), 3.46 - 3.41 (m, 1H), 3.39 / 3.38 (s, 3H), 3.05 - 2.99 (m, 1H), 2.53 - 2.47 (m, 1H), 2.31 (s, 3H), 2.26 - 2.19 (m, 1H), 1.33 - 1.31 (d, 3H), 1.28 - 1.26 (d, 3H).

### Beispiel Nr. I.1-162:

¹H NMR (400 MHz, CDCl₃) δ (ppm) = 7.09 - 7.02 (m, 3H), 5.37 - 5.32 (m, 1H), 5.25 - 5.18 (m, 1H), 5.05 - 5.01 (br. s, 1H), 4.94 - 4.90 (br. s, 1H), 4.34 - 4.28 / 3.75 - 3.73 (m, 1H), 4.19 - 4.15 (m, 1H), 3.07 - 2.99 (m, 1H), 2.55 - 2.47 (m, 1H), 2.31 (s, 3H), 2.26 - 2.19 (m, 1H), 1.33 - 1.33 / 1.28 - 1.26 (d, 3H), 1.25 - 1.21 (m, 3H).

### b) Auswertung über das Peaklistenverfahren

Die ¹H-NMR-Daten ausgewählter Beispiele werden in Form von ¹H-NMR-Peaklisten oder über Auswertung der Peaks durch Expertinnen und Experten notiert. Zu jedem Signalpeak wird beim Peaklistenverfahren erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet. Die Peakliste eines Beispiels hat daher die Form:
δ₁ (Intensität₁⁾; δ₂ (Intensität₂);... ... ..; δᵢ (Intensitätᵢ⁾;... ...; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden. Zur Kalibrierung der chemischen Verschiebung von ¹H-NMR-Spektren wird Tetramethylsilan verwendet in Kombination mit der charakteristischen chemischen Verschiebung des Lösemittels, besonders im Falle von Spektren, die in DMSO gemessen werden. Daher kann in NMR-Peaklisten der Tetramethylsilan-Peak vorkommen, muss es aber nicht. Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösemittelsignale, Signale von Stereoisomeren der Zielverbindungen, die ebenfalls Gegenstand der Erfindung sind, und/oder Peaks von Verunreinigungen zeigen. Bei der Angabe von Verbindungssignalen im Delta-Bereich von Lösemitteln und/oder Wasser sind in unseren Listen von 1H-NMR-Peaks die gewöhnlichen Lösemittelpeaks, zum Beispiel Peaks von DMSO in DMSO-D₆ und der Peak von Wasser, gezeigt, die gewöhnlich im Durchschnitt eine hohe Intensität aufweisen. Die Peaks von Stereoisomeren der Zielverbindungen und/oder Peaks von Verunreinigungen haben gewöhnlich im Durchschnitt eine geringere Intensität als die Peaks der Zielverbindungen (zum Beispiel mit einer Reinheit von >90%). Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen. Einem Experten oder einer Expertin, der/die die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation. Weitere Details zu ¹H-NMR-Peaklisten können der Research Disclosure Database Number 564025 entnommen werden.

### Beispiel Nr. I.1-16:

¹H-NMR(400.6 MHz, d₆-DMSO): δ= 7.0639 (0.5); 7.0453 (0.8); 6.9714 (0.5); 6.8778 (0.6); 6.8687 (0.7); 6.7488 (0.6); 3.7598 (3.9); 3.7369 (2.9); 3.3207 (16.0); 3.0761 (1.0); 2.5188 (0.6); 2.5100 (9.4); 2.5054 (20.5); 2.5009 (28.6); 2.4963 (19.8); 2.4917 (8.8); 2.2320 (3.1); 1.1596 (2.6); 1.1436 (2.8); 1.1058 (3.0); 0.0080 (0.7); -0.0002 (23.7); -0.0085 (0.7)

### Beispiel Nr. 1.1-22:

¹H NMR (400 MHz, CDCl₃) δ (ppm) = 7.5198 (1.1); 7.2945 (0.6); 7.2912 (0.7); 7.2809 (1.1); 7.2793 (1.1); 7.2737 (1.6); 7.2608 (175.8); 7.1146 (1.9); 7.0954 (4.3); 7.0679 (3.5); 7.0484 (1.4); 6.9968 (2.5); 6.9887 (3.0); 5.2676 (0.9); 5.2459 (1.8); 5.2242 (0.9); 5.1931 (0.8); 4.5067 (0.9); 4.4887 (3.2); 4.4690 (6.5); 4.4511 (6.1); 4.4333 (1.9); 3.1333 (1.3); 3.1142 (1.5); 3.0949 (1.6); 3.0759 (1.6); 3.0617 (0.9); 2.5678 (1.2); 2.5455 (1.6); 2.5293 (1.0); 2.5071 (1.5); 2.4647 (0.5); 2.4453 (0.8); 2.4258 (0.9); 2.4080 (0.8); 2.3542 (0.5); 2.3214 (16.0); 1.4389 (2.9); 1.4255 (6.9); 1.4212 (6.4); 1.4078 (13.9); 1.4035 (3.7); 1.3900 (6.4); 1.2841 (11.3); 1.2675 (11.3); 0.0080 (1.4); -0.0002 (49.8); -0.0085 (1.6)

### Beispiel Nr. I.1-32:

¹H NMR (400 MHz, CDCl₃) δ (ppm) = 7.2600 (50.9); 7.2558 (0.7); 7.0802 (0.5); 7.0616 (1.1); 7.0495 (1.3); 7.0413 (1.6); 7.0327 (2.3); 5.3161 (1.1); 5.3016 (1.0); 5.3008 (1.0); 5.2974 (7.4); 5.1767 (0.6); 5.0247 (0.8); 4.9220 (1.0); 3.0485 (0.8); 3.0291 (1.0); 3.0110 (16.0); 2.9906 (1.0); 2.9399 (8.9); 2.9393 (8.9); 2.5344 (0.7); 2.5114 (0.8); 2.4960 (0.6); 2.4728 (0.7); 2.4512 (10.1); 2.4484 (11.7); 2.3080 (7.1); 2.2353 (0.6); 2.2170 (0.5); 2.0806 (9.8); 1.6925 (0.5); 1.2688 (5.0); 1.2526 (4.7); 0.8818 (0.5); -0.0002 (14.5); -0.0027 (0.7).

### Beispiel Nr. I.1-35:

¹H NMR (400 MHz, CDCl₃) δ (ppm) = 7.5179 (0.8); 7.2661 (1.5); 7.2590 (155.7); 7.2540 (1.4); 7.2532 (1.0); 7.2524 (0.8); 7.2515 (0.5); 7.2490 (1.0); 7.2476 (0.5); 7.0896 (0.8); 7.0714 (1.7); 7.0580 (1.5); 7.0382 (3.8); 7.0070 (0.6); 6.9950 (0.9); 5.2821 (0.9); 5.2613 (1.0); 5.2463 (1.2); 5.2264 (1.0); 5.1915 (0.7); 5.1701 (0.8); 4.9853 (1.2); 4.8925 (1.5); 3.0875 (1.5); 3.0774 (0.6); 3.0690 (4.7); 3.0591 (2.0); 3.0506 (5.2); 3.0407 (2.0); 3.0360 (2.0); 3.0322 (2.2); 3.0223 (1.4); 3.0026 (1.0); 2.5391 (0.7); 2.5162 (0.8); 2.5010 (0.6); 2.4782 (0.7); 2.3092 (16.0); 2.2563 (0.9); 2.2375 (1.1); 2.2181 (0.8); 2.0433 (1.6); 1.6403 (3.2); 1.3814 (0.8); 1.3723 (2.3); 1.3629 (3.9); 1.3539 (4.8); 1.3438 (5.6); 1.3356 (2.6); 1.3251 (3.0); 1.3042 (1.3); 1.2911 (1.5); 1.2869 (1.7); 1.2688 (13.2); 1.2583 (5.9); 1.2534 (9.6); 1.2406 (1.3); 1.1612 (0.7); 0.8990 (3.5); 0.8820 (13.4); 0.8643 (4.8); 0.0080 (1.2); -0.0002 (41.7); -0.0085 (1.2)

Gegenstand der vorliegenden Erfindung ist weiterhin die Verwendung einer oder mehrerer erfindungsgemäßer Verbindungen der allgemeinen Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (I.1-1) bis (I.2-52) und/oder deren Salze, jeweils wie oben definiert, als Herbizid und/oder Pflanzenwachstumsregulator, vorzugsweise in Kulturen von Nutz- und/oder Zierpflanzen.

Gegenstand der vorliegenden Erfindung ist ferner ein Verfahren zur Bekämpfung von Schadpflanzen und/oder zur Wachstumsregulierung von Pflanzen, dadurch gekennzeichnet, daß eine wirksame Menge
- einer oder mehrerer erfindungsgemäßer Verbindungen der allgemeinen Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (I.1-1) bis (I.2-52) und/oder deren Salze, jeweils wie oben definiert, oder
- eines erfindungsgemäßen Mittels, wie nachstehend definiert,
auf die (Schad)Pflanzen, (Schad)Pflanzensamen, den Boden, in dem oder auf dem die (Schad)Pflanzen wachsen, oder die Anbaufläche appliziert wird.

Das Verfahren zur Bekämpfung von Schadpflanzen ist dadurch gekennzeichnet, daß eine wirksame Menge, einer oder mehrerer erfindungsgemäßen Verbindungen der Formel (I) und/oder deren Salze eine wirksame Menge eines erfindungsgemäßen Mittels auf die Schadpflanzen, Schadpflanzensamen, den Boden, in dem oder auf dem die Schadpflanzen wachsen oder in dem oder auf dem sich die Schadpflanzensamen befinden oder auf eine Anbaufläche appliziert wird.

"Wirksame Menge zur Bekämpfung von Schadpflanzen" ist so zu verstehen, daß es sich um eine Menge der Verbindungen der Formel (I) und/oder deren Salze handelt, die geeignet ist, das Wachstum von Schadpflanzen signifikant einzuschränken oder vollständig zu verhindern. Betreffend ein Mittel/eine Zusammensetzung enthaltend eine Verbindung der allgemeinen Formel (I) und/oder deren Salze, dadurch gekennzeichnet, daß das Mittel mindestens einen weiteren herbiziden (oder herbizid wirkenden) agronomischen Wirkstoff enthält, bedeutet "wirksame Menge zur Bekämpfung von Schadpflanzen" daß die Menge der Verbindungen der Formel (I) und/oder deren Salze zusammen mit der Menge des weiteren herbiziden (oder herbizid wirkenden) agronomischen Wirkstoffes geeignet sind, um das Wachstum von Schadpflanzen signifikant einzuschränken oder vollständig zu verhindern.

Gegenstand der vorliegenden Erfindung ist auch ein Verfahren zur Bekämpfung von unerwünschten Pflanzen, vorzugsweise in Nutzpflanzenkulturen, dadurch gekennzeichnet, daß eine wirksame Menge
- einer oder mehrerer Verbindungen der allgemeinen Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (I.1-1) bis (I.2-52) und/oder deren Salze, jeweils wie oben definiert, oder
- eines erfindungsgemäßen Mittels, wie nachstehend definiert,
auf unerwünschte Pflanzen (z.B. Schadpflanzen wie mono- oder dikotyle Unkräuter oder unerwünschte Kulturpflanzen), das Saatgut der unerwünschten Pflanzen (d.h. Pflanzensamen, z.B. Körner, Samen oder vegetative Vermehrungsorgane wie Knollen oder Sprossteile mit Knospen), den Boden, in dem oder auf dem die unerwünschte Pflanzen wachsen, (z.B. den Boden von Kulturland oder Nicht-Kulturland) oder die Anbaufläche (d.h. Fläche, auf der die unerwünschten Pflanzen wachsen werden) appliziert wird.

Gegenstand der vorliegenden Erfindung ist ferner auch ein Verfahren zur Wachstumsregulierung von Pflanzen, vorzugsweise von Nutzpflanzen, dadurch gekennzeichnet, daß eine wirksame Menge
- einer oder mehrerer Verbindungen der allgmeinenen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (I.1-1) bis (I.2-52) und/oder deren Salze, jeweils wie oben definiert, oder
- eines erfindungsgemäßen Mittels, wie nachstehend definiert,
die Pflanze, Teile der Pflanze (z.B. Saatgut der Pflanze, d.h. Pflanzensamen, z.B. Körner, Samen oder vegetative Vermehrungsorgane wie Knollen oder Sprossteile mit Knospen), den Boden, in dem oder auf dem die Pflanzen wachsen, (z.B. den Boden von Kulturland oder Nicht-Kulturland) oder die Anbaufläche (d.h. Fläche, auf der die Pflanzen wachsen werden) appliziert wird.

Das Verfahren zur Wachstumsregulierung von Pflanzen ist dadurch gekennzeichnet, daß eine wirksame Menge, einer oder mehrerer Verbindungen der allgemeinen Formel (I) und/oder deren Salze oder eine wirksame Menge eines erfindungsgemäßen Mittels auf die Pflanzen, Teile der Pflanzen, deren Wachstum reguliert werden soll oder den Boden, in dem oder auf dem die Pflanzen, deren Wachstum reguliert werden soll, wachsen, appliziert wird.

"Wirksame Menge zur Wachstumsregulierung" sind solche Mengen, die das Wachstum einer Pflanze beinflussen, z.B. die Halmhöhe in Getreidepfanzen verringern, ohne jedoch einen signifikanten negativen Einfluß auf den (unter gegebenen Umweltbedingungen zu erwartenden) Ernteertrag zu nehmen. Betreffend ein Mittel/eine Zusammensetzung enthaltend eine Verbindung der allgemeinen Formel (I) und/oder deren Salze, dadurch gekennzeichnet, daß das Mittel mindestens einen weiteren wachstumsregulierenden (oder wachstumsregulierend wirkenden) agronomischen Wirkstoff enthält, bedeutet "Wirksame Menge zur Wachstumsregulierung", daß die Menge der Verbindungen der Formel (I) und/oder deren Salze zusammen mit der Menge des weiteren wachstumsregulierenden (oder wachstumsregulierend wirkenden) agronomischen Wirkstoffes das Wachstum einer Pflanze beinflussen, z.B. die Halmhöhe in Getreidepfanzen verringern, ohne jedoch eine signifikanten negativen Einfluß auf den (unter gegebenen Umweltbedingungen zu erwartenden) Ernteertrag zu nehmen.

"Teile von Pflanzen" können Blätter, Stengel, Wurzeln, Samen, Keimlinge und vegetatives Vermehrungsmaterial wie Knollen, Stecklinge sein.

Der hierin verwendete Begriff "Mittel" ist gleichbedeutend mit dem Begriff "Zusammensetzung". Beide Begriffe werden Synonym verwendet.

Dabei können die erfindungsgemäßen Verbindungen der allgemeinen Formel (I) bzw. die erfindungsgemäßen Mittel z.B. im Vorsaat- (ggf. auch durch Einarbeitung in den Boden), Vorauflauf- und/oder Nachauflaufverfahren ausgebracht werden. Im Einzelnen seien beispielhaft einige Vertreter der mono- und dikotylen Unkrautflora genannt, die durch die die erfindungsgemäßen Verbindungen kontrolliert werden können, ohne daß durch die Nennung eine Beschränkung auf bestimmte Arten erfolgen soll.

Die erfindungsgemäßen Verbindungen der allgemeinen Formel (I) und/oder deren Salze eignen sich zur Bekämpfung der folgenden Gattungen von monokotylen und dikotylen Schadpflanzen:
Monokotyle Schadpflanzen der Gattungen: Aegilops, Agropyron, Agrostis, Alopecurus, Apera, Avena, Brachiaria, Bromus, Cenchrus, Commelina, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Phalaris, Phleum, Poa, Rottboellia, Sagittaria, Scirpus, Setaria, Sorghum.
Dikotyle Schadpflanzen der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Conyza, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen der allgemeinen Formel (I) vor dem Keimen der Schadpflanzen (Ungräser und/oder Unkräuter) auf die Erdoberfläche appliziert (Vorauflaufverfahren), so wird entweder das Auflaufen der Ungras- bzw. Unkrautkeimlinge vollständig verhindert oder diese wachsen bis zum Keimblattstadium heran, stellen jedoch dann ihr Wachstum ein und sterben schließlich nach Ablauf von drei bis vier Wochen vollkommen ab.

Bei Applikation der erfindungsgemäßen Verbindungen der allgemeinen Formel (I) auf die grünen Schadpflanzenpflanzenteile im Nachauflaufverfahren tritt nach der Behandlung ein Wachstumsstop ein und die Schadpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen der allgemeinen Formel (I) eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, sowie in Früchte-tragenden, und Holz produzierenden Büschen, Bäumen oder generell Dauerkulturen, Reben und Hopfen wie Kulturpflanzen die zur Gattung der Annonaceae, Eucalyptus, Citrus, Malus, Pyrus, Prunus, Vitis, Musa, oder Olea gehören oder monokotyler Kulturen der Gattungen Allium, Ananas, Asparagus, Avena, Hordeum, Oryza, Panicum, Saccharum, Secale, Sorghum, Triticale, Triticum, Zea, und auch monokotyle Zierpflanzen der Arten Cynodon, Eremochloa, Paspalum, Stenotaphrum, Zoysia abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen der allgemeinen Formel (I) (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die erfindungsgemäßen Verbindungen der allgemeinen Formel (I) und/oder deren Salze auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Vorzugsweise werden in einem erfindungsgemäßen Verfahren zur Bekämpfung von Schadpflanzen oder zur Wachstumsregulierung von Pflanzen eine oder mehrere Verbindungen der allgemeinen Formel (I) und/oder deren Salze zur Bekämpfung von Schadpflanzen oder zur Wachstumsregulierung in Kulturen von Nutzpflanzen oder Zierpflanzen eingesetzt, wobei die Nutzpflanzen oder Zierpflanzen in einer bevorzugten Ausgestaltung transgene Pflanzen sind.

Bevorzugt bezüglich transgener Kulturen ist die Anwendung der erfindungsgemäßen Verbindungen der allgemeinen Formel (I) und/oder deren Salze in wirtschaftlich bedeutenden transgenen Kulturen von Nutz und Zierpflanzen, z.B. von Getreide wie Weizen, Gerste, Roggen, Hafer, Hirse, Reis und Mais oder auch Kulturen von Zuckerrohr, Zuckerrübe, Baumwolle, Soja, Raps, Kartoffel, Apfel, Tomate, Erbse und anderen Gemüsesorten.

Vorzugsweise können die erfindungsgemäßen Verbindungen der allgemeinen Formel (I) auch als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die erfindungsgemäßen Verbindungen der allgemeinen Formel (I) auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der allgemeinen Formel (I) und/oder deren Salze in wirtschaftlich bedeutenden transgenen Kulturen von Nutz-und Zierpflanzen, z.B. von Getreide wie Weizen, Gerste, Roggen, Hafer, Triticale, Hirse, Reis, Maniok und Mais oder auch Kulturen von Zuckerrohr, Zuckerrübe, Baumwolle, Soja, Raps, Kartoffel, Apfel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden.

Zahlreiche molekularbiologische Techniken, mit denen neue transgene Pflanzen mit veränderten Eigenschaften hergestellt werden können, sind dem Fachmann bekannt. Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) und/oder deren Salze in transgenen Kulturen eingesetzt werden, welche gegen Wuchsstoffe, wie z.B. Dicamba und 2,4-D oder gegen Herbizide, die essentielle Pflanzenenzyme, z.B. Acetolactatsynthasen (ALS), EPSP Synthasen, Glutaminsynthasen (GS), Protoporphyrinogen-Oxidasen (PPO), Acetyl CoA Carboxylase (ACCase) oder Hydoxyphenylpyruvat Dioxygenasen (HPPD), Photosysteme, insbesondere Photosystem II hemmen, respektive gegen Herbizide aus der Gruppe der Sulfonylharnstoffe, der Glyphosate, Glufosinate, N-Phenyl-Imide, Nitrile oder Benzoylisoxazole und analogen Wirkstoffe, resistent sind.

Bei der Anwendung der erfindungsgemäßen Verbindungen der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) und/oder deren Salze als Herbizide zur Bekämpfung von Schadpflanzen in Kulturen von Nutz- oder Zierpflanzen, gegebenenfalls in transgenen Kulturpflanzen.

Bevorzugt ist die Verwendung in Kulturpflanzen umfassend Getreide, dabei vorzugsweise Mais, Weizen, Gerste, Roggen, Hafer, Hirse, oder Reis, sowie in Früchte-tragenden, und Holz produzierenden Büschen, Bäumen oder generell Dauerkulturen, Reben und Hopfen wie Kulturpflanzen die zur Gattung der Annonaceae, Eucalyptus, Citrus, Malus, Pyrus, Prunus, Vitis, Musa, oder Olea und Arecales gehören und auch in monokotylen Zierpflanzen der Arten Cynodon, Eremochloa, Paspalum, Stenotaphrum, Zoysia , im Vor- oder Nachauflauf der jeweiligen Unkräuter.

Bevorzugt ist auch die Verwendung in Soja im Vor- oder Nachauflauf der Sojapflanze und/oder im Vor- oder Nachauflauf der Schadpflanze.

Ebenfalls bevorzugt ist die Verwendung in Plantagen, wie Plantagen in denen Kulturpflanzen wie z.B. Mango, Mirabelle, Nektarine, Pfirsich, Pflaume, Zwetschge, Aprikose Olive, Kirsche, Kokosnuß, Mandel, Pistazie, Apfel, Birne, Banane, Erdbeere, Himbeere, Johannisbeere, Blaubeeren, Ölpalmen, Kokospalmen, Haselnuß, Erdnuß, Walnuß, Kaffee, Kakao, Tee, Baumwolle, Kiwi oder Zuckerrohr angebaut werden.

Weiter bevorzugt ist die Verwendung in Zuckerrohr Plantagen/Feldern, wobei die erfindungsgemäße Verbindung vorzugsweise zwischen den Zuckerrohrpflanzen auf den Boden appliziert wird.

Auch die Verwendung auf Brach- und/oder Industrieflächen, Wegrändern, Feldrändern, Schienen und auch in monokotylen Zierpflanzen der Arten Cynodon, Eremochloa, Paspalum, Stenotaphrum, Zoysia ist bevorzugt.

Die erfindungsgemäße Verwendung zur Bekämpfung von Schadpflanzen oder zur Wachstumsregulierung von Pflanzen schließt auch den Fall ein, bei dem eine Verbindung der allgemeinen Formel (I) oder deren Salz erst nach der Ausbringung auf der Pflanze, in der Pflanze oder im Boden aus einer Vorläufersubstanz ("Prodrug") gebildet wird.

Gegenstand der Erfindung ist auch die Verwendung einer oder mehrerer Verbindungen der allgemeinen Formel (I) oder deren Salzen bzw. eines erfindungsgemäßen Mittels (wie nachstehend definiert) (in einem Verfahren) zur Bekämpfung von Schadpflanzen oder zur Wachstumsregulierung von Pflanzen, dadurch gekennzeichnet, daß man eine wirksame Menge einer oder mehreren Verbindungen der allgemeinen Formel (I) oder deren Salzen auf die Pflanzen (Schadpflanzen, ggf. zusammen mit den Nutzpflanzen) Pflanzensamen, den Boden, in dem oder auf dem die Pflanzen wachsen, oder die Anbaufläche appliziert.

Gegenstand der Erfindung ist auch ein herbizides und/oder pflanzenwachstumsregulierendes Mittel, dadurch gekennzeichnet, daß das Mittel
(a) eine oder mehrere Verbindungen der allgemeinen Formel (I) und/oder deren Salze enthält wie oben definiert, vorzugsweise in einer der als bevorzugt bzw. besonders bevorzugt gekennzeichneten Ausgestaltung, insbesondere eine oder mehrere Verbindungen der Formeln (I.1-1) bis (I.2-52) und/oder deren Salze, jeweils wie oben definiert,
   und
(b) ein oder mehrere weitere Stoffe ausgewählt aus den Gruppen (i) und/oder (ii):
   (i) ein oder mehrere weitere agrochemisch wirksame Stoffe, vorzugsweise ausgewählt aus der Gruppe bestehend aus Insektiziden, Akariziden, Nematiziden, weiteren Herbiziden (d.h. solche, die nicht der oben definierten allgemeinen Formel (I) entsprechen), Fungiziden, Safenern, Düngemitteln und/oder weiteren Wachstumsregulatoren,
   (ii) ein oder mehrere im Pflanzenschutz übliche Formulierungshilfsmittel.

Die weiteren agrochemischen wirksamen Stoffe des Bestandteils (i) eines erfindungsgemäßen Mittels sind dabei vorzugsweise ausgewählt aus der Gruppe der Stoffe, die in "The Pesticide Manual", 19th edition, The British Crop Protection Council and the Royal Soc. of Chemistry, 2021, genannt sind.

Gegenstand der Erfindung ist auch ein Mittel/eine Zusammensetzung enthaltend eine Verbindung der allgemeinen Formel (I) und/oder deren Salze, dadurch gekennzeichnet, daß das Mittel (zusätzlich) einen oder mehrere im Pflanzenschutz übliche Formulierungshilfsmittel enthält.

Ein erfindungsgemäßes herbizides oder pflanzenwachstumsregulierendes Mittel, umfasst vorzugsweise ein, zwei, drei oder mehr im Pflanzenschutz übliche Formulierungshilfsmittel (ii) ausgewählt aus der Gruppe bestehend aus Tensiden, Emulgatoren, Dispergiermitteln, Filmbildnern, Verdickungsmitteln, anorganischen Salzen, Stäubemitteln, bei 25 °C und 1013 mbar festen Trägerstoffen, vorzugsweise adsorptionsfähigen, granulierten Inertmaterialien, Netzmitteln, Antioxidationsmitteln, Stabilisatoren, Puffersubstanzen, Antischaummitteln, Wasser, organischen Lösungsmitteln, vorzugsweise bei 25 °C und 1013 mbar mit Wasser in jedem beliebigen Verhältnis mischbare organische Lösungsmittel.

Die erfindungsgemäßen Verbindungen der allgemeinen Formel (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der allgemeinen Formel (I) und/oder deren Salze enthalten.

Die erfindungsgemäßen Verbindungen der allgemeinen Formel (I) und/oder deren Salze können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen und die Formulierungshilfsmittel wie Inertmaterialien, Tenside, Lösungsmittel und weitere Zusatzstoffe sind dem Fachmann bekannt, und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Nass-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wässrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulaten siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen, vorzugsweise herbizide oder pflanzenwachstumsregulierende Mittel der vorliegenden Erfindung enthalten vorzugsweise eine Gesamtmenge von 0,1 bis 99 Gew.-%, bevorzugt 0,5 bis 95 Gew.-%, weiter bevorzugt 1 bis 90 Gew.-%, insbesondere bevorzugt 2 bis 80 Gew.-%, an Wirkstoffen der allgemeinen Formel (I) und deren Salzen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die erfindungsgemäßen Verbindungen der allgemeinen Formel (I) oder deren Salze können als solche oder in Form ihrer Zubereitungen (Formulierungen) mit anderen agronomisch wirksamen Stoffen, wie z.B. Pestiziden (Insektiziden, Akariziden, Nematiziden, Herbiziden, Fungiziden), Safenern, Düngemitteln und/oder Wachstumsregulatoren kombiniert eingesetzt werden, z.B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Ein weiterer Gegenstand der Erfindung betrifft daher Mittel/Zusammensetzungen enthaltend eine erfindungsgemäße Verbindung der allgemeinen Formel (I) und/oder deren Salze und mindestens einen weiteren agronomisch wirksamen Stoff. Der agronomisch wirksame Stoff kann ein Kombinationspartner sein, wobei der Kombinationspartner bevorzugt einen im Pflanzenschutz üblichen Wirkstoff und/oder einen Safener und/oder einen Pflanzenwachstumsregulator darstellt. Bevorzugt handelt es sich bei dem im Pflanzenschutz üblichen Wirkstoff um ein Herbizid, ein Fungizid, ein Insektizid, ein Akarizid oder ein Nematizid, bevorzugt um ein Herbizid, ein Fungizid oder ein Insektizid. Bevorzugte Herbizide, Safener und Wachstumsregulatoren, die als Kombinationspartner für erfindungsgemäße Zusammensetzungen in Frage kommen sind weiter unten genannt.

Eine mögliche Ausführungsform der Erfindung betrifft Mittel enthaltend eine erfindungsgemäße Verbindung der allgemeinen Formel (I) und/oder deren Salze und einen Safener.

Eine weitere Ausführungsform der Erfindung betrifft Mittel enthaltend eine erfindungsgemäße Verbindung der allgemeinen Formel (I) und/oder deren Salze und (mindestens) ein weiteres Herbizid.

In einer anderen Ausführungsform der erfindungsgemäßen Mittel handelt es sich um ein Mittel enthaltend eine erfindungsgemäße Verbindung der allgemeinen Formel (I) und/oder deren Salze und (mindestens) ein weiteres Herbizid und einen Safener.

Auch Gegenstand der Erfindung sind Mittel enthaltend eine erfindungsgemäße Verbindung der allgemeinen Formel (I) und/oder deren Salze und einen Wachstumsregulator.

Auch Gegenstand der Erfindung sind Mittel enthaltend eine erfindungsgemäße Verbindung der allgemeinen Formel (I) und/oder deren Salze und (mindestens) ein weiteres Herbizid und einen Wachstumsregulator.

Weiterhin sind Mittel Gegenstand der Erfindung, die eine erfindungsgemäße Verbindung der allgemeinen Formel (I) und/oder deren Salze und (mindestens) ein weiteres Herbizid und einen Wachstumsregulator und einen Safener enthalten.

Als Kombinationspartner für die erfindungsgemäßen Verbindungen der allgemeinen Formel (I) in Mischungsformulierungen oder im Tank-Mix sind beispielsweise bekannte Wirkstoffe, die auf einer Inhibition von beispielsweise des Microtubuli-Aufbaus, einer Acetolactat-Synthase, einer Acetyl-CoA-Carboxylase, einer Cellulose-Synthase, einer Enolpyruvylshikimat-3-phosphat-Synthase, einer Glutamin-Synthetase, einer p-Hydroxyphenylpyruvat-Dioxygenase, einer Phytoendesaturase, des Photosystems I, Photosystems II, einer Protoporphyrinogen-Oxidase beruhen, einsetzbar, wie sie z.B. aus Weed Research 26 (1986) 441-445 oder "The Pesticide Manual", 19th edition, The British Crop Protection Council and the Royal Soc. of Chemistry, 2021 und dort zitierter Literatur beschrieben sind.

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der allgemeinen Formel (I) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z.B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der allgemeinen Formel (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Äußere Bedingungen wie Temperatur, Feuchtigkeit etc. beeinflussen zu einem gewissen Teil die Aufwandmenge der erfindungsgemäßen Verbindungen der allgemeinen Formel (I) und/oder deren Salze. Die Aufwandmenge kann dabei innerhalb weiter Grenzen variieren. Für die Anwendung als Herbizid zur Bekämpfung von Schadpflanzen liegt die Gesamtmenge an erfindungsgemäßen Verbindungen der allgemeinen Formel (I) und deren Salze vorzugsweise im Bereich von 0,001 bis 10,0 kg/ha, bevorzugt im Bereich von 0,005 bis 5 kg/ha, weiter bevorzugt im Bereich von 0,01 bis 1,5 kg/ha, insbesondere bevorzugt im Bereich von 0,05 bis 1 kg/ha. Dies gilt sowohl für die Anwendung im Vorauflauf oder im Nachauflauf.

Bei der Anwendung von erfindungsgemäßen Verbindungen der allgemeinen Formel (I) und/oder deren Salzen als Pflanzenwachstumsregulator, beispielsweise als Halmverkürzer bei Kulturpflanzen, wie sie oben genannt worden sind, vorzugsweise bei Getreidepflanzen wie Weizen, Gerste, Roggen, Triticale, Hirse, Reis oder Mais, liegt die Gesamt-Aufwandmenge vorzugsweise im Bereich von 0,001 bis 2 kg/ha, vorzugsweise im Bereich von 0,005 bis 1 kg/ha, insbesondere im Bereich von 10 bis 500 g/ha, ganz besonders bevorzugt im Bereich von 20 bis 250 g/ha. Dies gilt sowohl für die Anwendung im Vorauflauf oder im Nachauflauf.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Bekannte Wachstumsregulatoren, die in erfindungsgemäßen Zusammensetzungen verwendet werden können, sind z,B.: Indol-3-essigsäure (IAA), 4-(Indol-3-yl)buttersäure (IBA), 1-Naphthylessigsäure (NAA), Gibberellinsäure, Dichlorprop (2,4-DP), 2,4,5-T, 2,4-D, Maleinsäurehydrazid, Tecnazen, 3-CPA, Kinetin, Benzyladenin (BAP), Thidiazuron, Gibberellin GA3, Carbaryl, 4-CPA, Chlorpropham (CIPC), Chlormequat (CCC), Ethephon, Abscisinsäure, Abscisin II, Chlorflurenol, Daminozid, Ancymidol, Mepiquat, Dimethipin, Chlorphoniumchlorid, Dikegulac, Mefluidid, Ethylchlozat, Flumetralin, Brassinosteroide, Oligosaccharide, Uniconazol-P, Forchlorfenuron, Paclobutrazol, Cyanwasserstoff, Flurprimidol, Inabenfid, Phthalimid, Trinexapac, Cyclanilid, Prohexadion, 1-Methylcyclopropen, Jasmonate, Aviglycin (Aminoethoxyvinylglycin), Monoterpene, Phospholipide oder Trans-2-ketone.

Als bekannte Herbizide, die mit Verbindungen der allgemeinen Formel (I) kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:

Acetochlor, Acifluorfen, Acifluorfen-methyl, Acifluorfen-Natrium, Aclonifen, Alachlor, Allidochlor, Alloxydim, Alloxydim-Natrium, Amidochlor, Amidosulfuron, 4-Amino-3-chlor-6-(4-chlor-2-fluor-3-methylphenyl)-5-fluorpyridin-2-carbonsäure, Aminocyclopyrachlor, Aminocyclopyrachlor-Kalium, Aminocyclopyrachlor-methyl, Aminopyralid, Aminopyralid-dimethylammonium, Aminopyralidtripromine, Ammoniumsulfamate, Anilofos, Asulam, Asulam-Kalium, Asulam-Natrium, Azafenidin, Azimsulfuron, Beflubutamid, (S)-(-)-Beflubutamid, Beflubutamid-M, Benazolin, Benazolin-ethyl, Benazolin-dimethylammonium, Benazolin-Kalium, Benfluralin, Benfuresate, Bensulfuron, Bensulfuronmethyl, Bensulid, Bentazon, Bentazon-Natrium, Benzobicyclon, Benzofenap, Bifenox, Bilanafos, Bilanafos-Natrium, Bipyrazone, Bispyribac, Bispyribac-Natium, Bixlozon, Bromacil, Bromacil-lithium, Bromacil-Natrium, Bromobutid, Bromofenoxim, Bromoxynil, Bromoxynilbutyrat, Bromoxynil-Kalium, Bromoxynil-heptanoat und Bromoxynil-octanoat, Busoxinon, Butachlor, Butafenacil, Butamifos, Butenachlor, Butralin, Butroxydim, Butylat, Cafenstrol, Cambendichlor, Carbetamide, Carfentrazon, Carfentrazon-Ethyl, Chloramben, Chloramben-ammonium, Chloramben-diolamin, Chlroamben-methyl, Chloramben-methylammonium, Chloramben-Natrium, Chlorbromuron, Chlorfenac, Chlorfenacammonium, Chlorfenac-Natrium, Chlorfenprop, Chlorfenprop-methyl, Chlorflurenol, Chlorflurenolmethyl, Chloridazon, Chlorimuron, Chlorimuron-ethyl, Chlorophthalim, Chlorotoluron, Chlorsulfuron, Chlorthal, Chlorthal-dimethyl, Chlorthal-monomethyl, Cinflubrolin, Cinidon, Cinidon-ethyl, Cinmethylin, exo-(+)-Cinmethylin, d.h. (1R,2S,4S)-4-isopropyl-1-methyl-2-[(2-methylbenzyl)oxy]-7-oxabicyclo[2.2.1]heptan, exo-(-)-Cinmethylin, d.h. (1R,2S,4S)-4-isopropyl-1-methyl-2-[(2-methylbenzyl)oxy]-7-oxabicyclo[2.2.1]heptan, Cinosulfuron, Clacyfos, Clethodim, Clodinafop, Clodinafop-ethyl, Clodinafop-propargyl, Clomeprop, Clopyralid, Clopyralid-methyl, Clopyralid-olamin, Clopyralid-Kalium, Clopyralid-tripomin, Cloransulam, Cloransulam-methyl, Cumyluron, Cyanamide, Cyanazine, Cycloat, Cyclopyranil, Cyclopyrimorat, Cyclosulfamuron, Cycloxydim, Cyhalofop, Cyhalofop-butyl, Cyprazin, 2,4-D (sowie die Ammonium, Butotyl, Butyl, Cholin, Diethylammonium, Dimethylammonium, Diolamin, Doboxyl, Dodecylammonium, Etexyl, Ethyl, 2-Ethylhexyl, Heptylammonium, Isobutyl, Isooctyl, Isopropyl, Isopropylammonium, Lithium, Meptyl, Methyl, Kalium, Tetradecylammonium, Triethylammonium, Triisopropanolammonium, Tripromin and Trolamin Salze davon), 2,4-DB, 2,4-DB-butyl, 2,4-DB-Dimethylammonium, 2,4-DB-isooctyl, 2,4-DB-Kalium und 2,4-DB-Natrium, Daimuron (Dymron), Dalapon, Dalapon-Calcium, Dalapon-Magnesium, Dalapon-Natium, Dazomet, Dazomet-Natrium, n-Decanol, 7-Deoxy-D-sedoheptulose, Desmedipham, Detosyl-pyrazolat (DTP), Dicamba und seine Salze (z.B. Dicamba-biproamin, Dicamba-N,N-Bis(3-aminopropyl)methylamin, Dicamba-butotyl, Dicamba-cholin, Dicamba-Diglycolamin, Dicamba-Dimethylammonium, Dicamba-Diethanolaminemmonium, Dicamba-Diethylammonium, Dicambaisopropylammonium, Dicamba-methyl, Dicamba-monoethanolamin, Dicamba-olamin, Dicamba-Kalium, Dicamba-Natrium, Dicamba-Triethanolamin), Dichlobenil, 2-(2,4-Dichlorbenzyl)-4,4-dimethyl-1,2-oxazolidin-3-on, 2-(2,5-Dichlorbenzyl)-4,4-dimethyl-1,2-oxazolidin-3-on, Dichlorprop, Dichlorpropbutotyl, Dichlorprop-Dimethylammonium, Dichhlorprop-etexyl, Dichlorprop-ethylammonium, Dichlorprop-isoctyl, Dichlorprop-methyl, Dichlorprop-Kalium, Dichlorprop-Natrium, Dichlorprop-P, Dichlorprop-P-Dimethylammonium, Dichlorprop-P-etexyl, Dichlorprop-P-Kalium, Dichlorprop-Natrium, Diclofop, Diclofop-methyl, Diclofop-P, Diclofop-P-methyl, Diclosulam, Difenzoquat, Difenzoquat-metilsulfate, Diflufenican, Diflufenzopyr, Diflufenzopyr-Natrium, Dimefuron, Dimepiperate, Dimesulfazet, Dimethachlor, Dimethametryn, Dimethenamid, Dimethenamid-P, Dimetrasulfuron, Dinitramine, Dinoterb, Dinoterb-Acetate, Diphenamid, Diquat, Diquat-Dibromid, Diquat-Dichlorid, Dithiopyr, DNOC, DNOC-Ammonium, DNOC-Kalium, DNOC-Natrium, Endothal, Endothal-Diammonium, Endothal-Dikalium, Endothal-Dinatrium, EPTC, Esprocarb, Ethalfluralin, Ethametsulfuron, Ethametsulfuron-Methyl, Ethiozin, Ethofumesate, Ethoxyfen, Ethoxyfen-Ethyl, Ethoxysulfuron, Etobenzanid, F-5231, d.h. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, Fenoxaprop, Fenoxaprop-P, Fenoxaprop-Ethyl, Fenoxaprop-P-Ethyl, Fenoxasulfone, Fenpyrazone, Fenquinotrione, Fentrazamid, Flamprop, Flamprop-Isoproyl, Flamprop-Methyl, Flamprop-M-Isopropyl, Flamprop-M-Methyl, Florasulam, Florpyrauxifen, Florpyrauxifen-benzyl, Fluazifop, Fluazifop-Butyl, Fluazifop-Methyl, Fluazifop-P, Fluazifop-P-Butyl, Flucarbazone, Flucarbazone-Natrium, Flucetosulfuron, Fluchloralin, Flufenacet, Flufenpyr, Flufenpyr-Ethyl, Flumetsulam, Flumiclorac, Flumiclorac-Pentyl, Flumioxazin, Fluometuron, Flurenol, Flurenol-Butyl, -Dimethylammonium und -Methyl, Fluoroglycofen, Fluoroglycofen-Ethyl, Flupropanat, Flupropanat-Natrium, Flupyrsulfuron, Flupyrsulfuron-Methyl, Flupyrsulfuron-Methyl-Natrium, Fluridon, Flurochloridon, Fluroxypyr, Fluroxypyr-Butometyl, Fluroxypyr-Meptyl, Flurtamon, Fluthiacet, Fluthiacet-Methyl, Fomesafen, Fomesafen-Natrium, Foramsulfuron, Foramsulfuron-Natrium, Fosamine, Fosamine-Ammonium, H-9201, d.h. O-(2,4-Dimethyl-6-nitrophenyl)-O-ethyl-isopropylphosphoramidothioat, Halauxifen, Halauxifen-methyl, Halosafen, Halosulfuron, Halosulfuron-Methyl, Haloxyfop, Haloxyfop-P, Haloxyfop-Ethoxyethyl, Haloxyfop-P-Ethoxyethyl, Haloxyfop-Methyl, Haloxyfop-P-Methyl, Haloxifop-Natrium, HNPC-A8169, d.h. Prop-2-in-1-yl (2S)-2-{3-[(5-tert-butylpyridin-2-yl)oxy]phenoxy}propanoat, HW-02, d.h. 1-(Dimethoxyphosphoryl)-ethyl-(2,4-dichlorphenoxy)acetat, Hydantocidin, Imazamethabenz, Imazamethabenz-Methyl, Imazamox, Imazamox-Ammonium, , Imazaquin, Imazaquin-Ammonium, Imazaquin-Methyl, Imazethapyr, Imazethapyr-Ammonium, Imazosulfuron, Indanofan, Indolauxipyr, Iodosulfuron, Iodosulfuron-Methyl, Iodosulfuron-Methyl-Natrium, Ioxynil, Ioxynil-Lithium, -Octanoat, - Kalium und Natrium, Ipfencarbazon, Iptriazopyrid, d.h. 3-[(Isopropylsulfonyl)methyl]-N-(5-methyl-1,3,4-oxadiazol-2-yl)-5-(trifluormethyl)[1,2,4]triazolo-[4,3-a]pyridin-8-carboxamid, Isoproturon, Isouron, Isoxaben, Karbutilat, KUH-043, d.h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Ketospiradox, Ketospiradox-Kalium, Lactofen, Lenacil, Linuron, MCPA, MCPA-Butotyl, -Butyl, -Dimethylammonium, -Diolamin, - 2-Ethylhexyl, -Ethyl, -Isobutyl, Isoctyl, -Isopropyl, -Isopropylammonium, -Methyl, Olamin, -Kalium, - Natrium und -Trolamin, MCPB, MCPB-Methyl, -Ethyl und -Natrium, Mecoprop, Mecoprop-Butotyl, Mecoprop- dimethylammonium, Mecoprop-Diolamin, Mecoprop-Etexyl, Mecoprop-Ethadyl, Mecoprop-Isoctyl, Mecoprop-Methyl, Mecoprop-Kalium, Mecoprop-Natrium, und Mecoprop-Trolamin, Mecoprop-P, Mecoprop-P-Butotyl, -Dimethylammonium, -2-Ethylhexyl und -Kalium, Mefenacet, Mefluidid, Mefluidid-Diolamin, Mefluidid-Kalium, Mesosulfuron, Mesosulfuron-Methyl, Mesosulfuron-Natrium, Methabenzthiazuron, Metam, Metamifop, Metamitron, Metazachlor, Metazosulfuron, Methabenzthiazuron, Methiopyrsulfuron, Methiozolin, Methyl isothiocyanat, Metobromuron, Metolachlor, S-Metolachlor, Metosulam, Metoxuron, Metproxybicyclon, Metsulfuron, Metsulfuron-Methyl, Molinat, Monolinuron, Monosulfuron, Monosulfuron-Methyl, MT-5950, d.h. N-[3-Chlor-4-(1-methylethyl)-phenyl]-2-methylpentanamid, NGGC-011, Napropamid, NC-310, d.h. 4-(2,4-Dichlorbenzoyl)-1-methyl-5-benzyloxypyrazol, Neburon, Nicosulfuron, Nonansäure (Pelargonsäure), Norflurazon, Ölsäure (Fettsäuren), Orbencarb, Orthosulfamuron, Oryzalin, Oxadiargyl, Oxadiazon, Oxasulfuron, Oxaziclomefone, Paraquat, Paraquat-dichlorid, Paraquat-Dimethylsulfat, Pebulat, Pendimethalin, Penoxsulam, Pentachlorphenol, Pentoxazon, Pethoxamid, Petroleumöl, Phenmedipham, Phenmedipham-Ethyl, Picloram, Picloram-dimethylammonium, Picloram-Etexyl, Picloram-Isoctyl, Picloram-Methyl, Picloram-Olamin, Picloram-Kalium, Picloram-Triethylammonium, Picloram-Tripromin, Picloram-Trolamin, Picolinafen, Pinoxaden, Piperophos, Pretilachlor, Primisulfuron, Primisulfuron-Methyl, Prodiamine, Profoxydim, Prometon, Prometryn, Propachlor, Propanil, Propaquizafop, Propazine, Propham, Propisochlor, Propoxycarbazone, Propoxycarbazone-Natrium, Propyrisulfuron, Propyzamid, Prosulfocarb, Prosulfuron, Pyraclonil, Pyraflufen, Pyraflufen-Ethyl, Pyraquinat, Pyrasulfotol, Pyrazolynat (Pyrazolat), Pyrazosulfuron, Pyrazosulfuron-Ethyl, Pyrazoxyfen, Pyribambenz, Pyribambenz-Isopropyl, Pyribambenz-Propyl, Pyribenzoxim, Pyributicarb, Pyridafol, Pyridat, Pyriftalid, Pyriminobac, Pyriminobac-Methyl, Pyrimisulfan, Pyrithiobac, Pyrithiobac-Natrium, Pyroxasulfon, Pyroxsulam, Quinclorac, Quinclorac-Dimethylammonium, Quinclorac-Methyl, Quinmerac, Quinoclamin, Quizalofop, Quizalofop-Ethyl, Quizalofop-P, Quizalofop-P-Ethyl, Quizalofop-P-Tefuryl, QYM201, d.h. 1-{2-Chlor-3-[(3-cyclopropyl-5-hydroxy-1-methyl-1H-pyrazol-4-yl)carbonyl]-6-(trifluormethyl)phe-nyl}piperidin-2-on, Rimisoxafen, Sethoxydim, Siduron, Simetryn, SL-261, Sulcotrione, Sulfometuron, Sulfometuron-Methyl, Sulfosulfuron, , SYP-249, d.h. 1-Ethoxy-3-methyl-1-oxobut-3-en-2-yl-5-[2-chlor-4-(trifluormethyl)phenoxy]-2-nitrobenzoat, SYP-300, d.h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trichloressigsäure) und seine Salze, z.B. TCA-ammonium, TCA-Calcium, TCA-Ethyl, TCA-Magnesium, TCA-Natrium, Tefuryltrione, Tepraloxydim, Terbucarb, Terbumeton, Terbuthylazine, Terbutryn, Tetflupyrolimet, Thaxtomin, Thenylchlor, Thiazopyr, Thiencarbazone, Thiencarbazon-Methyl, Thifensulfuron, Thifensulfuron-Methyl, Thiobencarb, Tolpyralat, Tralkoxydim, Triafamon, Triallat, Triasulfuron, Triaziflam, Tribenuron, Tribenuron-Methyl, Triclopyr, Triclopyr-Butotyl, Triclopyr-Cholin, Triclopyr-Ethyl, Triclopyr-Triethylammonium, Trietazine, Trifloxysulfuron, Trifloxysulfuron-Natrium, Trifludimoxazin, Trifluralin, Triflusulfuron, Triflusulfuron-Methyl, Tritosulfuron, Harnstoffsulfat, Vernolat, XDE-848, ZJ-0862, d.h. 3,4-Dichlor-N-{2-[(4,6-dimethoxypyrimidin-2-yl)oxy]benzyl}anilin, 3-(2-Chlor-4-fluor-5-(3-methyl-2,6-dioxo-4-trifluormethyl-3,6-dihydropyrimidin-1(2H)-yl)phenyl)-5-methyl-4,5-dihydroisoxazol-5-carbonsäuremethylester, 3-(2-Chlor-4-fluor-5-(3-methyl-2,6-dioxo-4-trifluormethyl-3,6-dihydropyrimidin-1(2H)-yl)phenyl)-5-methyl-4,5-dihydroisoxazol-5-carbonsäureethylester, 3-(2-Chlor-4-fluor-5-(3-methyl-2,6-dioxo-4-trifluormethyl-3,6-dihydropyrimidin-1(2H)-yl)phenyl)-5-methyl-4,5-dihydroisoxazol-5-carbonsäure, Ethyl-[(3-{2-chlor-4-fluor-5-[3-methyl-2,6-dioxo-4-(trifluormethyl)-3,6-dihydropyrimidin-1(2H)-yl]phenoxy}pyridin-2-yl)oxy]acetat, 3-Chlor-2-[3-(difluormethyl)isoxazolyl-5-yl]phenyl-5-chlorpyrimidin-2-ylether, 2-(3,4-Dimethoxyphenyl)-4-[(2-hydroxy-6-oxocyclohex-1-en-1-yl)carbonyl]-6-methylpyridazin-3(2H)-on, 2-({2-[(2-Methoxyethoxy)methyl]-6-methylpyridin-3-yl}carbonyl)cyclohexane-1,3-dion, (5-Hydroxy-1-methyl-1H-pyrazol-4-yl)(3,3,4-trimethyl-1,1-dioxido-2,3-dihydro-1-benzothiophen-5-yl)methanon, 1-Methyl-4-[(3,3,4-trimethyl-1,1-dioxido-2,3-dihydro-1-benzothiophen-5-yl)carbonyl]-1H-pyrazol-5-yl propan-1-sulfonat, 4-{2-Chlor-3-[(3,5-dimethyl-1H-pyrazol-1-yl)methyl]-4-(methylsulfonyl)benzoyl}-1-methyl-1H-pyrazol-5-yl-1,3-dimethyl-1H-pyrazol-4-carboxylat; Cyanomethyl-4-amino-3-chlor-5-fluor-6-(7-fluor-1H-indol-6-yl)pyridin-2-carboxylat, Prop-2-yn-1-yl 4-amino-3-chlor-5-fluor-6-(7-fluor-1H-indol-6-yl)pyridin-2-carboxylat, Methyl-4-amino-3-chlor-5-fluor-6-(7-fluor-1H-indol-6-yl)pyridin-2-carboxylat, Benzyl-4-amino-3-chlor-5-fluor-6-(7-fluor-1H-indol-6-yl)pyridin-2-carboxylat, Ethyl-4-amino-3-chlor-5-fluor-6-(7-fluor-1H-indol-6-yl)pyridin-2-carboxylat, Methyl-4-amino-3-chlor-5-fluor-6-(7-fluor-1-isobutyryl-1H-indol-6-yl)pyridin-2-carboxylat, Methyl 6-(1-acetyl-7-fluor-1H-indol-6-yl)-4-amino-3-chlor-5-fluorpyridin-2-carboxylat, Methyl-4-amino-3-chlor-6-[1-(2,2-dimethylpropanoyl)-7-fluor-1H-indol-6-yl]-5-fluorpyridin-2-carboxylat, Methyl-4-amino-3-chlor-5-fluor-6-[7-fluor-1-(methoxyacetyl)-1H-indol-6-yl]pyridin-2-carboxylat, Kalium 4-amino-3-chlor-5-fluor-6-(7-fluor-1H-indol-6-yl)pyridin-2-carboxylat, Natrium-4-amino-3-chlor-5-fluor-6-(7-fluor-1H-indol-6-yl)pyridin-2-carboxylat, Butyl-4-amino-3-chlor-5-fluoro-6-(7-fluoro-1H-indol-6-yl)pyridin-2-carboxylat, 4-Hydroxy-1-methyl-3-[4-(trifluoromethyl)pyridin-2-yl]imidazolidin-2-on, 3-(5-tert-butyl-1,2-oxazol-3-yl)-4-hydroxy-1-methylimidazolidin-2-on, 3-[5-Chlor-4-(trifluormethyl)pyridin-2-yl]-4-hydroxy-1-methylimidazolidin-2-on, 4-Hydroxy-1-methoxy-5-methyl-3-[4-(trifluormethyl)pyridin-2-yl]imidazolidin-2-on, 6-[(2-Hydroxy-6-oxocyclohex-1-en-1-yl)carbonyl]-1,5-dimethyl-3-(2-methylphenyl)chinazolin-2,4(1H,3H)-dion, 3-(2,6-Dimethylphenyl)-6-[(2-hydroxy-6-oxocyclohex-1-en-1-yl)carbonyl]-1-methylchinazolin-2,4(1H,3H)-dion, 2-[2-chlor-4-(methylsulfonyl)-3-(morpholin-4-ylmethyl)benzoyl]-3-hydroxycyclohex-2-en-1-on, 1-(2-carboxyethyl)-4-(pyrimidin-2-yl)pyridazin-1-iumsalz (mit passenden Anionen wie z.B Chlorid, Acetat oder Trifluoracetat), 1-(2-Carboxyethyl)-4-(pyridazin-3-yl)pyridazin-1-iumsalz (mit passenden Anionen wie z.B. Chlorid, Acetat oder Trifluoracetat), 4-(Pyrimidin-2-yl)-1-(2-sulfoethyl)pyridazin-1-ium salz (mit passenden Anionen wie z.B Chlorid, Acetat oder Trifluoracetat), 4-(Pyridazin-3-yl)-1-(2-sulfoethyl)pyridazin-1-iumsalz (mit passenden Anionen wie z.B Chlorid, Acetat oder Trifluoracetat), 1-(2-Carboxyethyl)-4-(1,3-thiazol-2-yl)pyridazin-1-iumsalz (mit passenden Anionen wie z.B Chlorid, Acetat oder Trifluoracetat), 1-(2-Carboxyethyl)-4-(1,3,4-thiadiazol-2-yl)pyridazin-1-iumsalz (mit passenden Anionen wie z.B Chlorid, Acetat oder Trifluoracetat), Methyl (2R)-2-{[(E)-({2-chlor-4-fluor-5-[3-methyl-2,6-dioxo-4-(trifluormethyl)-3,6-dihydropyrimidin-1(2H)-yl]phenyl}methyliden)amino]oxy}propanoat, Methyl (2S)-2-{[(E)-({2-chlor-4-fluor-5-[3-methyl-2,6-dioxo-4-(trifluormethyl)-3,6-dihydropyrimidin-1(2H)-yl]phenyl}methyliden)amino]oxy}propanoat, Methyl (2R/S)-2-{[(E)-({2-chlor-4-fluor-5-[3-methyl-2,6-dioxo-4-(trifluormethyl)-3,6-dihydropyrimidin-1(2H)-yl]phenyl}methyliden)amino]oxy}propanoat, (E)-2-(Trifluormethyl)benzaldehyd-O-{2,6-bis[(4,6-dimethoxypyrimidin-2-yl)oxy]benzoyl}oxim, 2-Fluor-N-(5-methyl-1,3,4-oxadiazol-2-yl)-3-[(R)-propylsulfinyl]-4-(trifluormethyl)benzamid, (2R)-2-[(4-Amino-3,5-dichlor-6-fluor-2-pyridyl)oxy]propancarbonsäure, 2-Ethoxy-2-oxoethyl-1-{2-chlor-4-fluor-5-[3-methyl-2,6-dioxo-4-(trifluormethyl)-3,6-dihydropyrimidin-1(2H)-yl]phenoxy}cyclopropancarboxylat, 2-Methoxy-2-oxoethyl-1-{2-chlor-4-fluor-5-[3-methyl-2,6-dioxo-4-(trifluormethyl)-3,6-dihydropyrimidin-1(2H)-yl]phenoxy}cyclopropancarboxylat, {[(1-{2-Chlor-4-fluor-5-[3-methyl-2,6-dioxo-4-(trifluormethyl)-3,6-dihydropyrimidin-1(2H)-yl]phenoxy}cyclopropyl)carbonyl]oxy}essigsäure, 2-(2-Brom-4-chlorbenzyl)-4,4-dimethyl-1,2-oxazolidin-3-on, Methyl 3-{2-chlor-4-fluor-5-[3-methyl-2,6-dioxo-4-(trifluoromethyl)-3,6-dihydropyrimidin-1(2H)-yl]phenyl}-3a,4,5,6-tetrahydro-6aH-cyclopenta[d][1,2]oxazol-6a-carboxylat, Ethyl 3-{2-chlor-4-fluor-5-[3-methyl-2,6-dioxo-4-(trifluoromethyl)-3,6-dihydropyrimidin-1(2H)-yl]phenyl}-3a,4,5,6-tetrahydro-6aH-cyclopenta[d][1,2]oxazol-6a-carboxylat, Methyl-3-{2-chlor-4-fluor-5-[3-methyl-2,6-dioxo-4-(trifluormethyl)-3,6-dihydropyrimidin-1(2H)-yl]phenyl}-6-methyl-3a,4,5,6-tetrahydro-6aH-cyclopenta[d][1,2]oxazol-6a-carboxylat, 3-{2-Chlor-4-fluor-5-[3-methyl-2,6-dioxo-4-(trifluormethyl)-3,6-dihydropyrimidin-1(2H)-yl]phenyl}-6-methyl-3a,4,5,6-tetrahydro-6aH-cyclopenta[d][1,2]oxazol-6a-carbonsäure, 3-{2-Chlor-4-fluor-5-[3-methyl-2,6-dioxo-4-(trifluormethyl)-3,6-dihydropyrimidin-1(2H)-yl]phenyl}-3a,4,5,6-tetrahydro-6aH-cyclopenta[d][1,2]oxazol-6a-carbonsäure

Beispiele für Pflanzenwachstumsregulatoren als mögliche Mischungspartner sind: Abscisinsäure und verwandte Analoga [z.B. (2Z,4E)-5-[6-Ethynyl-1-hydroxy-2,6-dimethyl-4-oxocyclohex-2-en-1-yl]-3-methylpenta-2,4-diensäure, methyl-(2Z,4E)-5-[6-ethynyl-1-hydroxy-2,6-dimethyl-4-oxocyclohex-2-en-1-yl]-3-methylpenta-2,4-dienoat, (2Z,4E)-3-ethyl-5-(1-hydroxy-2,6,6-trimethyl-4-oxocyclohex-2-en-1-yl)penta-2,4-diensäure, (2E,4E)-5-(1-hydroxy-2,6,6-trimethyl-4-oxocyclohex-2-en-1-yl)-3-(trifluoromethyl)penta-2,4-diensäure, methyl (2E,4E)-5-(1-hydroxy-2,6,6-trimethyl-4-oxocyclohex-2-en-1-yl)-3-(trifluoromethyl)penta-2,4-dienoat, (2Z,4E)-5-(2-hydroxy-1,3-dimethyl-5-oxobicyclo[4.1.0]hept-3-en-2-yl)-3-methylpenta-2,4-diensäure], Acibenzolar, Acibenzolar-S-methyl, S-Adenosylhomocystein, Allantoin, 2-Aminoethoxyvinylglycin (AVG), Aminooxyessigsäure and verwandte Ester [z.B. (Isopropyliden)-aminooxyessigsäure-2-(methoxy)-2-oxoethylester, (Isopropyliden)-aminooxyessigsäure-2-(hexyloxy)-2-oxoethylester, (Cyclohexyliden)-aminooxyessigsäure-2-(isopropyloxy)-2-oxoethylester], 1-Aminocycloprop-1-ylcarbonsäure N-Methyl-1-aminocyclopropyl-1-carbonsäure, 1-Aminocyclopropyl-1-carbonsäureamid, substituierte 1-Aminocyclopropyl-1-carbonsäurederivate wie sie in DE3335514, EP30287, DE2906507 oder US5123951 beschrieben werden, 1-Aminocyclopropyl-1-hydroxamsäure, 5-Aminolevulinsäure, Ancymidol, 6-Benzylaminopurin, Bikinin, Brassinolid, Brassinolide-ethyl, L-Canalin, Catechin und catechine (z.B. (2S,3R)-2-(3,4-Dihydroxyphenyl)-3,4-dihydro-2H-chromen-3,5,7-triol), Chitooligosaccharides (CO; COs unterscheiden sich von LCOs dadurch, daß ihnen die für LCOs charakteristische Fettsäureseitenkette fehlt. COs, in manchen Fällen als N-Acetylchitooligosaccharide bezeichnet, sind auch aus GlcNAc-Einheiten aufgebaut, aber haben Seitenketten, durch die sies ich von Chitinmolekülen unterscheiden [(C₈H₁₃NO₅)ₙ, CAS No. 1398-61-4] und chitosan Moleküle [(C₅H₁₁NO₄)ₙ, CAS No. 9012-76-4]), Chitin-artige Verbindungen, Chlormequat chloride, Cloprop, Cyclanilide, 3-(Cycloprop-1-enyl)propionsäure, 1-[2-(4-Cyano-3,5-dicyclopropylphenyl)acetamido]cyclohexancarbonsäure, 1-[2-(4-Cyano-3-cyclopropylphenyl)acetamido]cyclohexancarbonsäure, 1-Cyclopropenylmethanol, Daminozid, Dazomet, Dazomet-Natrium, n-Decanol, Dikegulac, Dikegulac-Natrium, Endothal, Endothal-di-Kalium, -diNatrium, und mono(N,N-dimethylalkylammonium), Ethephon, 1-Ethylcyclopropen,Flumetralin, Flurenol, Flurenol-butyl, Flurenol-methyl, Flurprimidol, Forchlorfenuron, Gibberellinsäure, Inabenfid, Indol-3-essigsäure (IAA), 4-Indol-3-ylbuttersäure, Isoprothiolan, Probenazole, Jasmonsäure, Jasmonsäureester oder andere Derivate (z.B. Jasmonsäuremethylester, Jasmonsäureethylester), Lipochitooligosaccharide (LCO, in manchen Fällen auch als Symbiotische Nodulationssignale (Nod oder Nod Faktoren) oder als Myc Faktoren bezeichnet, bestehen aus einem Oligosacchariderückgrat aus β-1,4-verknüpften *N*-Acetyl-D-Glucosaminresten ("GlcNAc") mit einer N-verknüpften Fettsäureseitenkette, die am nicht reduzierenden Ende ankondensiert ist. Wie aus der Literatur zu entnehmen ist, unterscheiden sich LCOs in der Zahl an GlcNAc-EInheiten in der Rückgratstruktur, in der Länge und dem Sättigungsgrad der Fettsäurekette sowie in der Substitution der reduzierenden und nichtreduzierenden Zuckereinheiten), Linoleinsäure oder ihre Derivate, Linolensäure oder ihre Derivate, Maleinsäurehydrazid, Mepiquatchlorid, Mepiquatpentaborat, 1-Methylcyclopropen, 3-Methylcyclopropen, Methoxyvinylglycin (MVG), 3'-Methylabscisinsäure, 1-(4-Methylphenyl)-N-(2-oxo-1-propyl-1,2,3,4-tetrahydrochinolin-6-yl)methansulfonamid und verwandte substituierte (Tetrahydrochinolin-6-yl)methansulfonamide, (3E,3aR,8bS)-3-({[(2R)-4-Methyl-5-oxo-2,5-dihydrofuran-2-yl]oxy}methylen)-3,3a,4,8b-tetrahydro-2H-indeno[1,2-b]furan-2-on und verwandte Laktone wie sie in EP2248421 beschrieben sind, 2-(1-Naphthyl)acetamid, 1-Naphthylessigsäure, 2-Naphthyloxyessigsäure, Nitrophenolatmischung, 4-Oxo-4[(2-phenylethyl)amino]buttersäure, Paclobutrazol, 4-Phenylbuttersäure and ihre Salze (z.B. Natrium-4-phenylbutanoat, Kalium-4-phenylbutanoat), Phenylalanine, N-Phenylphthalamsäure, Prohexadione, Prohexadion-Calcium, , 1-n-Propylcyclopropen, Putrescin, Prohydrojasmon, Rhizobitoxin, Salicylsäure und Salicyclsäuremethylester, Sarcosin, Natriumcycloprop-1-en-1-ylacetat, Natriumcycloprop-2-en-1-ylacetat, Natrium-3-(cycloprop-2-en-1-yl)propanoat, Natrium-3-(cycloprop-1-en-1-yl)propanoat, Sidefungin, Spermidin, Spermine, Strigolactone, Tecnazene, Thidiazuron, Triacontanol, Trinexapac, Trinexapac-ethyl, Tryptophan, Tsitodef, Uniconazol, Uniconazol-P, 2-Fluoro-N-(3-methoxyphenyl)-*9H*-purin-6-amin, 2-chloro-N-(3-methoxyphenyl)-*9H*-purin-6-amin.

Ebenfalls als Kombinationspartner für die erfindungsgemäßen Verbindungen der allgemeinen Formel (I) kommen beispielsweise die folgenden Safener in Frage:
S1) Verbindungen der Formel (S1), 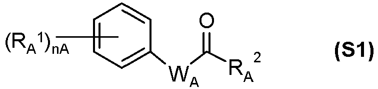 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)-Alkyl, (C₁-C₄)-Alkoxy, Nitro oder (C₁-C₄)-Haloalkyl; 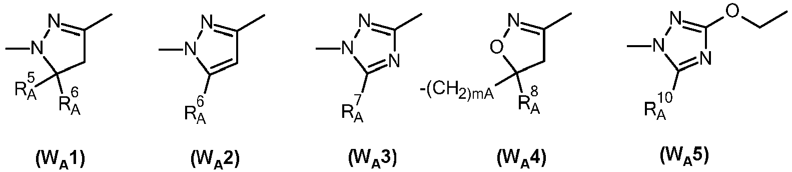
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁵),
   - m_{A}: ist 0 oder 1;
   - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)-Alkyl, (C₁-C₄)-Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Kohlenwasserstoffrest, vorzugsweise mit insgesamt 1 bis 18 C-Atomen;
   - R_{A}⁴: ist Wasserstoff, (C₁-C₆)-Alkyl, (C₁-C₆)-Alkoxy oder substituiertes oder unsubstituiertes Phenyl;
   - R_{A}⁵: ist H, (C₁-C₈)-Alkyl, (C₁-C₈)-Haloalkyl, (C₁-C₄)-Alkoxy(C₁-C₈)-Alkyl, Cyano oder COOR_{A}⁹, worin R_{A}⁹ Wasserstoff, (C₁-C₈)-Alkyl, (C₁-C₈)-Haloalkyl, (C₁-C₄)-Alkoxy-(C₁-C₄)-alkyl, (C₁-C₆)-Hydroxyalkyl, (C₃-C₁₂)-Cycloalkyl oder Tri-(C₁-C₄)-alkyl-silyl ist;
   - R_{A}⁶, R_{A}⁷, R_{A}⁸: sind gleich oder verschieden Wasserstoff, (C₁-C₈)-Alkyl, (C₁-C₈)-Haloalkyl, (C₃-C₁₂)-Cycloalkyl oder substituiertes oder unsubstituiertes Phenyl;
   - R_{A}¹⁰: ist H, (C₃-C₁₂)-Cycloalkyl, substituiertes oder unsubstituiertes Phenyl oder substituiertes oder unsubstituiertes Heteroaryl;
   vorzugsweise:
   a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl- 2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
   c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäure-ethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-3-carbonsäure (S1-10) oder 5,5-Diphenyl-2-isoxazolin-3-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
   f) Verbindungen vom Typ der Triazolyloxyessigsäurederivate (S1^{f}), vorzugsweise Verbindungen wie Methyl-{[1,5-bis(4-chlor-2-fluorphenyl)-1H-1,2,4-triazol-3-yl]oxy}acetat (S1-14) oder {[1,5-Bis(4-chlor-2-fluorphenyl)-1H-1,2,4-triazol-3-yl]oxy}essigsäure (S1-15) oder Methyl-{[5-(4-chlor-2-fluorphenyl)-1-(2,4-difluorphenyl)-1H-1,2,4-triazol-3-yl]oxy}acetat (S1-16) oder {[5-(4-Chlor-2-fluorphenyl)-1-(2,4-difluorphenyl)-1H-1,2,4-triazol-3-yl]oxy}essigsäure (S1-17) oder Methyl-{[1-(4-chlor-2-fluorphenyl)-5-(2,4-difluorphenyl)-1H-1,2,4-triazol-3-yl]oxy}acetat (S1-18) oder {[1-(4-Chlor-2-fluorphenyl)-5-(2,4-difluorphenyl)-1H-1,2,4-triazol-3-yl]oxy}essigsäure (S1-19), wie sie in der Patentanmeldung WO2021105101 beschrieben sind
S2) Chinolinderivate der Formel (S2), 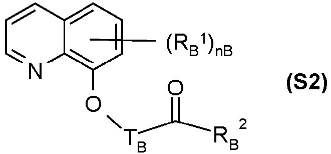 wobei die Symbole und Indizes folgende Bedeutungen haben:
   - R_{B}¹: ist Halogen, (C₁-C₄)-Alkyl, (C₁-C₄)-Alkoxy, Nitro oder (C₁-C₄)-Haloalkyl;
   - n_{B}: ist eine natürliche Zahl von 0 bis 5, vorzugsweise 0 bis 3;
   - R_{B}²: ist OR_{B}³, SR_{B}³ oder NR_{B}³R_{B}⁴ oder ein gesättigter
   oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)-Alkyl, (C₁-C₄)-Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)-Alkyl, (C₁-C₆)-Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)-Alkylresten oder mit [(C₁-C₃)-Alkoxy]-carbonyl substituiert ist;
   vorzugsweise:
   a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolin-oxy)essigsäure-1-allyloxy-prop-2-ylester (S2-4), (5-Chlor-8-chinolinoxy)essigsäure-ethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor- 8-chinolinoxy)-malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 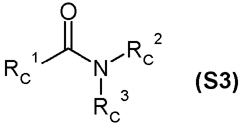 wobei die Symbole und Indizes folgende Bedeutungen haben:
   - R_{C}¹: ist (C₁-C₄)-Alkyl, (C₁-C₄)-Haloalkyl, (C₂-C₄)-Alkenyl, (C₂-C₄)-Haloalkenyl, (C₃-C₇)-Cycloalkyl, vorzugsweise Dichlormethyl;
   - R_{C}², R_{C}³: sind gleich oder verschieden Wasserstoff, (C₁-C₄)-Alkyl, (C₂-C₄)-Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenyl-carbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring; vorzugsweise: Wirkstoffe vom Typ der Dichloracetamide, die häufig als Vorauflaufsafener (bodenwirksame Safener) angewendet werden, wie z. B. "Dichlormid" (N,N-Diallyl-2,2-dichloracetamid) (S3-1), "R-29148" (3-Dichloracetyl-2,2,5-trimethyl-1,3-oxazolidin) der Firma Stauffer (S3-2), "R-28725" (3-Dichloracetyl-2,2,-dimethyl-1,3-oxazolidin) der Firma Stauffer (S3-3), "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5), "DKA-24" (N-Allyl-N-[(allylaminocarbonyl)methyl]-dichloracetamid) der Firma Sagro-Chem (S3-6), "AD-67" oder "MON 4660" (3-Dichloracetyl-1-oxa-3-aza-spiro[4,5]decan) der Firma Nitrokemia bzw. Monsanto (S3-7), "TI-35" (1-Dichloracetyl-azepan) der Firma TRI-Chemical RT (S3-8), "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF, "Furilazol" oder "MON 13900" ((RS)-3-Dichloracetyl-5-(2-furyl)-2,2-dimethyloxazolidin) (S3-10); sowie dessen (R)-Isomer (S3-11).
S4) N-Acylsulfonamide der Formel (S4) und ihre Salze, 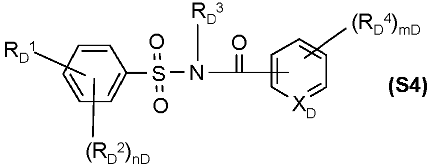 worin die Symbole und Indizes folgende Bedeutungen haben:
   - X_{D}: ist CH oder N;
   - R_{D}¹: ist CO-NR_{D}⁵R_{D}⁶ oder NHCO-R_{D}⁷;
   - R_{D}²: ist Halogen, (C₁-C₄)-Haloalkyl, (C₁-C₄)-Haloalkoxy, Nitro, (C₁-C₄)-Alkyl, (C₁-C₄)-Alkoxy, (C₁-C₄)-Alkylsulfonyl, (C₁-C₄)-Alkoxycarbonyl oder (C₁-C₄)-Alkylcarbonyl;
   - R_{D}³: ist Wasserstoff, (C₁-C₄)-Alkyl, (C₂-C₄)-Alkenyl oder (C₂-C₄)-Alkinyl;
   - R_{D}⁴: ist Halogen, Nitro, (C₁-C₄)-Alkyl, (C₁-C₄)-Haloalkyl, (C₁-C₄)-Haloalkoxy, (C₃-C₆)-Cycloalkyl, Phenyl, (C₁-C₄)-Alkoxy, Cyano, (C₁-C₄)-Alkylthio, (C₁-C₄)-Alkylsulfinyl, (C₁-C₄)-Alkylsulfonyl, (C₁-C₄)-Alkoxycarbonyl oder (C₁-C₄)-Alkylcarbonyl;
   - R_{D}⁵: ist Wasserstoff, (C₁-C₆)-Alkyl, (C₃-C₆)-Cycloalkyl, (C₂-C₆)-Alkenyl, (C₂-C₆)-Alkinyl, (C₅-C₆)-Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)-Alkoxy, (C₁-C₆)-Haloalkoxy, (C₁-C₂)-Alkylsulfinyl, (C₁-C₂)-Alkylsulfonyl, (C₃-C₆)-Cycloalkyl, (C₁-C₄)-Alkoxycarbonyl, (C₁-C₄)-Alkylcarbonyl und Phenyl und im Falle cyclischer Reste auch (C₁-C₄)-Alkyl und (C₁-C₄)-Haloalkyl substituiert sind;
   - R_{D}⁶: ist Wasserstoff, (C₁-C₆)-Alkyl, (C₂-C₆)-Alkenyl oder (C₂-C₆)-Alkinyl, wobei die drei letztgenannten Reste durch v_{D} Reste aus der Gruppe Halogen, Hydroxy, (C₁-C₄)-Alkyl, (C₁-C₄)-Alkoxy und (C₁-C₄)-Alkylthio substituiert sind, oder
   - R_{D}⁵ und R_{D}⁶: gemeinsam mit dem dem sie tragenden Stickstoffatom einen Pyrrolidinyl- oder Piperidinyl-Rest bilden;
   - R_{D}⁷: ist Wasserstoff, (C₁-C₄)-Alkylamino, Di-(C₁-C₄)-alkylamino, (C₁-C₆)-Alkyl, (C₃-C₆)-Cycloalkyl, wobei die 2 letztgenannten Reste durch v_{D} Substituenten aus der Gruppe Halogen, (C₁-C₄)-Alkoxy, (C₁-C₆)-Haloalkoxy und (C₁-C₄)-Alkylthio und im Falle cyclischer Reste auch (C₁-C₄)-Alkyl und (C₁-C₄)-Haloalkyl substituiert sind;
   - n_{D}: ist 0, 1 oder 2;
   - m_{D}: ist 1 oder 2;
   - v_{D}: ist 0, 1, 2 oder 3;
   davon bevorzugt sind Verbindungen vom Typ der N-Acylsulfonamide, z.B. der nachfolgenden Formel (S4^{a}), die z. B. bekannt sind aus WO-A-97/45016 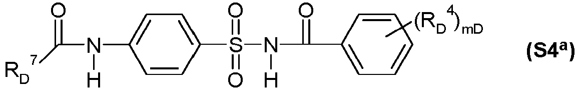 worin
   - R_{D}⁷: (C₁-C₆)-Alkyl, (C₃-C₆)-Cycloalkyl, wobei die 2 letztgenannten Reste durch v_{D} Substituenten aus der Gruppe Halogen, (C₁-C₄)-Alkoxy, (C₁-C₆)-Haloalkoxy und (C₁-C₄)-Alkylthio und im Falle cyclischer Reste auch (C₁-C₄)-Alkyl und (C₁-C₄)-Haloalkyl substituiert sind;
   - R_{D}⁴: Halogen, (C₁-C₄)-Alkyl, (C₁-C₄)-Alkoxy, CF_{3;}
   - m_{D}: 1 oder 2;
   - v_{D}: ist 0, 1, 2 oder 3 bedeutet;
   sowie Acylsulfamoylbenzoesäureamide, z.B. der nachfolgenden Formel (S4^{b}), die z.B. bekannt sind aus WO-A-99/16744, 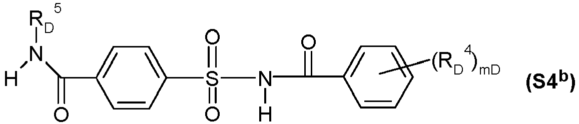 z.B. solche worin
   - R_{D}⁵ =: Cyclopropyl und (R_{D}⁴) = 2-OMe ist ("Cyprosulfamide", S4-1),
   - R_{D}⁵ =: Cyclopropyl und (R_{D}⁴) = 5-Cl-2-OMe ist (S4-2),
   - R_{D}⁵ =: Ethyl und (R_{D}⁴) = 2-OMe ist (S4-3),
   - R_{D}⁵ =: Isopropyl und (R_{D}⁴) = 5-Cl-2-OMe ist (S4-4) und
   - R_{D}⁵ =: Isopropyl und (R_{D}⁴) = 2-OMe ist (S4-5).
   sowie Verbindungen vom Typ der N-Acylsulfamoylphenylharnstoffe der Formel (S4^{c}), die z.B. bekannt sind aus der EP-A-365484, 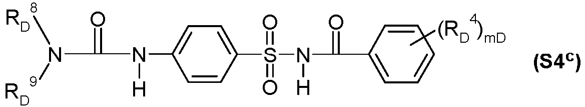 worin
   - R_{D}⁸ und R_{D}⁹: unabhängig voneinander Wasserstoff, (C₁-C₈)-Alkyl, (C₃-C₈)-Cycloalkyl, (C₃-C₆)-Alkenyl, (C₃-C₆)-Alkinyl,
   - R_{D}⁴: Halogen, (C₁-C₄)Alkyl, (C₁-C₄)Alkoxy, CF₃
   - m_{D}: 1 oder 2 bedeutet;
   beispielsweise
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff.
S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Di-methoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydrochinoxa-lin-2-on, wie sie in der WO-A-2005/112630 beschrieben sind.
S7) Verbindungen der Formel (S7),wie sie in der WO-A-1998/38856 beschrieben sind 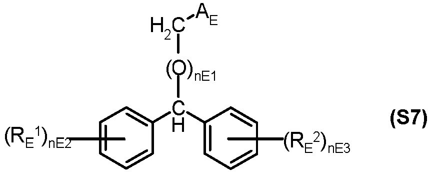 worin die Symbole und Indizes folgende Bedeutungen haben:
   - R_{E}¹, R_{E}²: sind unabhängig voneinander Halogen, (C₁-C₄)-Alkyl, (C₁-C₄)-Alkoxy, (C₁-C₄)-Haloalkyl, (C₁-C₄)-Alkylamino, Di-(C₁-C₄)-Alkylamino, Nitro;
   - A_{E}: ist COOR_{E}³ oder COSR_{E}⁴
   - R_{E}³, R_{E}⁴: sind unabhängig voneinander Wasserstoff, (C₁-C₄)-Alkyl, (C₂-C₆)-Alkenyl, (C₂-C₄)-Alkinyl, Cyanoalkyl, (C₁-C₄)-Haloalkyl, Phenyl, Nitrophenyl, Benzyl, Halobenzyl, Pyridinylalkyl und Alkylammonium,
   - n_{E}¹: ist 0 oder 1
   - n_{E}², n_{E}³: sind unabhängig voneinander 0, 1 oder 2,
   vorzugsweise Diphenylmethoxyessigsäure, Diphenylmethoxyessigsäureethylester, Diphenylmethoxyessigsäuremethylester (CAS-Reg.Nr. 41858-19-9) (S7-1).
S8) Verbindungen der Formel (S8),wie sie in der WO-A-98/27049 beschrieben sind 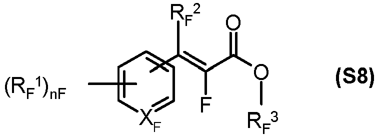 worin
   - X_{F}: CH oder N,
   - n_{F}: für den Fall, daß X_{F}=N ist, eine ganze Zahl von 0 bis 4 und für den Fall, daß X_{F}=CH ist, eine ganze Zahl von 0 bis 5,
   - R_{F}¹: Halogen, (C₁-C₄)-Alkyl, (C₁-C₄)-Haloalkyl, (C₁-C₄)-Alkoxy, (C₁-C₄)-Haloalkoxy, Nitro, (C₁-C₄)-Alkylthio, (C₁-C₄)-Alkylsulfonyl, (C₁-C₄)-Alkoxycarbonyl, ggf. substituiertes. Phenyl, ggf. substituiertes Phenoxy,
   - R_{F}²: Wasserstoff oder (C₁-C₄)-Alkyl,
   - R_{F}³: Wasserstoff, (C₁-C₈)-Alkyl, (C₂-C₄)-Alkenyl, (C₂-C₄)-Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; bedeuten, oder deren Salze,
   vorzugsweise Verbindungen worin
   - X_{F}: CH,
   - n_{F}: eine ganze Zahl von 0 bis 2,
   - R_{F}¹: Halogen, (C₁-C₄)-Alkyl, (C₁-C₄)-Haloalkyl, (C₁-C₄)-Alkoxy, (C₁-C₄)-Haloalkoxy,
   - R_{F}²: Wasserstoff oder (C₁-C₄)-Alkyl,
   - R_{F}³: Wasserstoff, (C₁-C₈)-Alkyl, (C₂-C₄)-Alkenyl, (C₂-C₄)-Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind 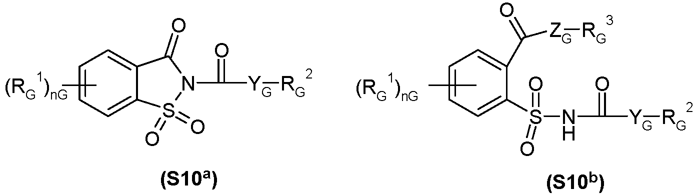 worin
   - R_{G}¹: Halogen, (C₁-C₄)-Alkyl, Methoxy, Nitro, Cyano, CF₃, OCF₃
   - Y_{G}, Z_{G}: unabhängig voneinander O oder S,
   - n_{G}: eine ganze Zahl von 0 bis 4,
   - R_{G}²: (C₁-C₁₆)-Alkyl, (C₂-C₆)-Alkenyl, (C₃-C₆)-Cycloalkyl, Aryl; Benzyl, Halogenbenzyl,
   - R_{G}³: Wasserstoff oder (C₁-C₆)-Alkyl bedeutet.
S11) Wirkstoffe vom Typ der Oxyimino-Verbindungen (S11), die als Saatbeizmittel bekannt sind, wie z. B. "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist, "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und "Cyometrinil" oder "CGA-43089" ((Z)-Cyanomethoxy-imino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13): "Naphthalic anhydrid" (1,8-Naphthalindicarbonsäureanhydrid) (S13-1), das als Saatbeiz-Safener für Mais gegen Schäden von Thiocarbamatherbiziden bekannt ist, "Fenclorim" (4,6-Dichlor-2-phenylpyrimidin) (S13-2), das als Safener für Pretilachlor in gesätem Reis bekannt ist, "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist, "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist, "MG 191" (CAS-Reg.Nr. 96420-72-3) (2-Dichlormethyl-2-methyl-1,3-dioxolan) (S13-5) der Firma Nitrokemia, das als Safener für Mais bekannt ist, "MG-838" (CAS-Reg.Nr. 133993-74-5) (2-propenyl 1-oxa-4-azaspiro[4.5]decan-4-carbodithioat) (S13-6) der Firma Nitrokemia, "Disulfoton" (O,O-Diethyl S-2-ethylthioethyl phosphordithioat) (S13-7), "Dietholate" (O,O-Diethyl-O-phenylphosphorothioat) (S13-8), "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (*S*-1-Methyl-1-phenylethyl-piperidin-1-carbothioat), das als Safener für Reis gegen Schäden des Herbizids Molinate bekannt ist, "Daimuron" oder "SK 23" (1-(1-Methyl-1-phenylethyl)-3-p-tolyl-harnstoff), das als Safener für Reis gegen Schäden des Herbizids Imazosulfuron bekannt ist, "Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist, "Methoxyphenon" oder "NK 049" (3,3'-Dimethyl-4-methoxy-benzophenon), das als Safener für Reis gegen Schäden einiger Herbizide bekannt ist, "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere
   wie sie in der WO-A-2008/131861 und WO-A-2008/131860 beschrieben sind 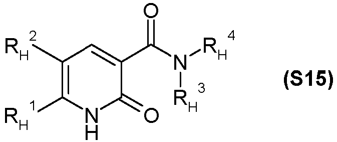 worin
   - R_{H}¹: einen (C₁-C₆)-Haloalkylrest bedeutet und
   - R_{H}²: Wasserstoff oder Halogen bedeutet und
   - R_{H}³, R_{H}⁴: unabhängig voneinander Wasserstoff, (C₁-C₁₆)-Alkyl, (C₂-C₁₆)-Alkenyl oder (C₂-C₁₆)-Alkinyl, wobei jeder der letztgenannten 3 Reste unsubstituiert oder durch einen oder mehrere Reste aus der Gruppe Halogen, Hydroxy, Cyano, (C₁-C₄)-Alkoxy, (C₁-C₄)-Haloalkoxy, (C₁-C₄)-Alkylthio, (C₁-C₄)-Alkylamino, Di[(C₁-C₄)-alkyl]-amino, [(C₁-C₄)-Alkoxy]-carbonyl, [(C₁-C₄)Haloalkoxy]-carbonyl, (C₃-C₆)-Cycloalkyl, das unsubstituiert oder substituiert ist, Phenyl, das unsubstituiert oder substituiert ist, und Heterocyclyl, das unsubstituiert oder substituiert ist, substituiert ist, oder (C₃-C₆)-Cycloalkyl, (C₄-C₆)-Cycloalkenyl, (C₃-C₆)-Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)-Alkyl, (C₁-C₄)-Haloalkyl, (C₁-C₄)-Alkoxy, (C₁-C₄)-Haloalkoxy, (C₁-C₄)-Alkylthio, (C₁-C₄)-Alkylamino, Di[(C₁-C₄)-alkyl]-amino, [(C₁-C₄)-Alkoxy]-carbonyl, [(C₁-C₄)-Haloalkoxy]-carbonyl, (C₃-C₆)-Cycloalkyl, das unsubstituiert oder substituiert ist, Phenyl, das unsubstituiert oder substituiert ist, und Heterocyclyl, das unsubstituiert oder substituiert ist, substituiert ist,
   bedeutet, oder
   - R_{H}³: (C₁-C₄)-Alkoxy, (C₂-C₄)-Alkenyloxy, (C₂-C₆)-Alkinyloxy oder (C₂-C₄)-Haloalkoxy bedeutet
   und
   - R_{H}⁴: Wasserstoff oder (C₁-C₄)-Alkyl bedeutet, oder
   - R_{H}³ und R_{H}⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)-Alkyl, (C₁-C₄)-Haloalkyl, (C₁-C₄)-Alkoxy, (C₁-C₄)-Haloalkoxy und (C₁-C₄)-Alkylthio substituiert ist, bedeutet.
S16) Wirkstoffe, die vorrangig als Herbizide eingesetzt werden, jedoch auch Safenerwirkung auf Kulturpflanzen aufweisen, z.B. (2,4-Dichlorphenoxy)essigsäure (2,4-D), (4-Chlorphenoxy)essigsäure, (R,S)-2-(4-Chlor-o-tolyloxy)propionsäure (Mecoprop), 4-(2,4-Dichlorphenoxy)buttersäure (2,4-DB), (4-Chlor-o-tolyloxy)-essigsäure (MCPA), 4-(4-Chlor-o-tolyloxy)buttersäure, 4-(4-Chlorphenoxy)-buttersäure, 3,6-Dichlor-2-methoxybenzoesäure (Dicamba), 1-(Ethoxycarbonyl)ethyl-3,6-dichlor-2-methoxybenzoat (Lactidichlor-ethyl).

Gegenstand der Erfindung ist auch die Verwendung einer oder mehrerer erfindungsgemäßer Verbindungen der allgemeinen Formel (I) und/oder deren Salze zur Herstellung eines Mittels zur Bekämpfung von Schadpflanzen, bzw. die Verwendung einer oder mehrerer erfindungsgemäßer Verbindungen der allgemeinen Formel (I) und/oder deren Salze zur Herstellung eines herbizid wirkenden Mittels.

Eine bevorzugte Ausführungsform betrifft die Verwendung einer oder mehrerer erfindungsgemäßer Verbindungen der allgemeinen Formel (I) und/der deren Salze zur Herstellung eines erfindungsgemäßen herbiziden und/oder pflanzenwachstumsregulierenden Mittels.

Für die Verwendung erfindungsgemäßer Verbindungen der allgemeinen Formel (I) oder deren Salze zur Herstellung eines Mittels zur Bekämpfung von Schadpflanzen ist es unerheblich, ob das hergestellte Mittel nur einen agronomisch wirksamen Stoff enthält, oder ob es mehrere agronomisch wirksame Stoffe enthält. Es können Mittel hergestellt werden, die eine oder mehrere erfindungsgemäße Verbindungen der allgemeinen Formel (I) oder deren Salzen enthalten. Es können auch Mittel hergestellt werden, die eine erfindungsgemäße Verbindung der allgemeinen Formel (I) oder deren Salze und einen oder mehrere weitere agronomisch wirksame(n) Stoffe enthalten. Es können auch Mittel hergestellt werden, die mehrere erfindungsgemäße Verbindungen der allgemeinen Formel (I) oder deren Salze und einen oder mehrere agronomisch wirksame Stoffe enthalten. Enthält das hergestellte Mittel mehrere agronomisch wirksame Stoffe, so kann es sich bei den zusätzlich zu den erfindungsgemäßen Verbindungen der allgemeinen Formel (I) oder deren Salzen enthaltenden agronomisch wirksamen Stoffen um ein Herbizid, ein Fungizid, ein Insektizid, ein Akarizid oder ein Nematizid, und/oder um Wachstumsregulatoren handeln. Weitere Wirkstoffe, insbesondre Wirkstoffe mit herbizider Aktivität, die für die Verwendung erfindungsgemäßer Verbindungen der allgemeinen Formel (I) oder deren Salzen zur Herstellung eines Mittels zur Bekämpfung von Schadpflanzen verwendet werden können, sind hierin an anderer Stelle genannt.

Ein weiterer Gegenstand der Erfindung betrifft ein Verfahren zur Herstellung eines Mittels zur Bekämpfung von Schadpflanzen, worin eine Verbindung der allgemeinen Formel (I) und/oder deren Salze eingesetzt/verwendet werden. Bevorzugt handelt es sich um ein Verfahren zur Herstellung eines herbiziden oder herbizid wirksamen Mittels.

Bevorzugt betrifft das Verfahren zur Herstellung eines Mittels zur Bekämpfung von Schadpflanzen (oder eines herbiziden oder herbizid wirksamen Mittels) ein Verfahren zur Herstellung eines erfindungsgemäßen herbiziden und/oder pflanzenwachstumsregulierenden Mittels.

Auch ein Verfahren zur Herstellung eines erfindungsgemäßen herbiziden und/oder pflanzenwachstumsregulierenden Mittels, worin mindestens eine Verbindung der allgemeinen Formel (I) und/oder deren Salze eingesetzt/verwendet wird, ist ein Gegenstand der Erfindung.

Ein weiterer Gegenstand der Erfindung betrifft Verfahren zur Herstellung eines Pflanzenschutzmittels, in dem mindestens eine Verbindung der allgemeinen Formel (I) und/oder deren Salze mit einem (im Pflanzenschutz üblichen) Formulierungshilfsmittel gemischt wird. Bevorzugt handelt es sich um ein Verfahren enthaltend folgende Schritte:
a) Mischen einer Verbindung der allgemeinen Formel (I) und/oder deren Salze mit (mindestens) einem (im Pflanzenschutz üblichen) Formulierungshilfsmittel;
b) Abfüllen des nach Schritt a) erhaltenen Gemisches in eine Verpackung.

Optional können in Schritt a) oder in einem oder mehreren zusätzlichen Schritten a-1), a-2), a-3) etc. dieses Verfahrens auch weitere Formulierungshilfsmittel und/oder agronomisch wirksame Stoffe und/ oder Safener durch Mischen hinzugefügt werden. Auch können diese zusätzlichen Schritte weitere Verarbeitungsschritte, wie Trocknen, Vermahlen, Extrudieren, Umkristallisieren etc. enthalten.

Agronomisch wirksame Stoffe, Safener und Formulierungshilfsmittel sind in diesem Anmeldetext an anderer Stelle spezifiziert und hier analog anwendbar.

Die in Verfahrensschritt b) genannte Verpackung kann jegliche Art von Verpackung darstellen. Sie kann eine Verpackung rein für den Transport des hergestellten Pflanzenschutzmittels zu einem anderen, weiterverarbeitenden Betrieb, oder sie kann auch eine Verpackung für den Vertrieb eines Pflanzenschutzmittels für den Verkauf an einen Landwirt, einen Großanwneder, wie z.B. Eisenbahnbetriebe, oder einen Endverbraucher für dessen Heimanwendung sein.

Das oben bezüglich der Verwendung von Verbindungen der allgemeinen Formel (I) und/oder deren Salze zur Herstellung von Mitteln zur Bekämpfung von Schadpflanzen (oder eines herbiziden oder herbizid wirksamen Mittels) Gesagte, ist auf die hier genannten Verfahren analog anwendbar.

### Biologische Beispiele:

Die folgenden Abkürzungen Schad- und Kulturpflanzen werden in den unten stehenden Tabellen und Versuchsauswertungen verwendet:

### Getestete Schadpflanzen:

- ABUTH:: Abutilon theophrasti
- AGSTE: Agrostis tenuis
- ALOMY:: Alopecurus myosuroides
- AMARE: Amaranthus retroflexus
- AVEFA:: Avena fatua
- CYPES:: Cyperus esculentus
- DIGSA:: Digitaria sanguinalis
- DIPTE: Diplotaxis tenuis
- ECHCG:: Echinochloa crus-galli
- KCHSC:: Kochia scoparia
- LOLPE: Lolium perenne
- LOLMU:: Lolium multiflorum
- MATCH:: Matricaria chamomilla
- MATIN:: Matricaria inodora
- PHBPU:: Pharbitis purpurea
- POAAN: Poa annua
- POLCO:: Polygonum convolvulus
- SETVI:: Setaria viridis
- STEME:: Stellaria media
- VERPE:: Veronica persica
- VIOTR:: Viola tricolor

### Getestete Kulturpflanzen:

- BRSNW:: Brassica napus
- ORYSA:: Oryza sativa
- TRZAS:: Triticum aestivum
- ZEAMX:: Zea mays

### A. Herbizide Wirkung im frühen Nachauflauf

Samen von mono- bzw. dikotylen Unkrautpflanzen werden in 96-well Mikrotiterplatten in Quarzsand ausgelegt und in der Klimakammer unter kontrollierten Wachstumsbedingungen angezogen. 5 bis 7 Tage nach der Aussaat werden die Versuchspflanzen im Keimblattstadium behandelt. Die in Form von Emulsionskonzentraten (EC) formulierten erfindungsgemäßen Verbindungen werden mit einer Wasseraufwandmenge von umgerechnet 2200 Liter pro Hektar appliziert. Nach 9 bis 12 Tagen Standzeit der Versuchspflanzen in der Klimakammer unter optimalen Wachstumsbedingungen, wird die Wirkung der Präparate visuell im Vergleich zu unbehandelten Kontrollen bonitiert. Beispielsweise bedeutet 100% Wirkung = Pflanzen sind abgestorben, 0% Wirkung = wie Kontrollpflanzen.

A1. Wirkung gegen AGSTE: In diesem Test hatten beispielsweise die folgenden erfindungsgemäßen Verbindungen eine sehr gute herbizide Wirkung (80% bis 100%) bei einer Aufwandmenge von 1900 g Aktivsubstanz pro Hektar: I.1-16, I.1-32, I.1-35, I.1-147, I.1-153, I.1-162. Die folgenden erfindungsgemäßen Verbindungen hatten weiterhin eine sehr gute herbizide Wirkung (> 80%) bei einer Aufwandmenge von 950 g Aktivsubstanz pro Hektar: I.1-16, I.1-32, I.1-35, I.1-147, I.1-153, I.1-162. Die folgenden erfindungsgemäßen Verbindungen zeigten eine sehr gute herbizide Wirkung (> 80%) bei einer Aufwandmenge von 475 g Aktivsubstanz pro Hektar: I.1-16, I.1-32, I.1-35, I.1-147, I.1-153, I.1-162. Darüber hinaus zeigten die folgenden erfindungsgemäßen Verbindungen eine sehr gute herbizide Wirkung (> 80%) bei einer Aufwandmenge von 238 g Aktivsubstanz pro Hektar: I.1-16, I.1-32, I.1-35, I.1-147, I.1-153, I.1-162.

A2. Wirkung gegen LOLPE: In diesem Test hatten beispielsweise die folgenden erfindungsgemäßen Verbindungen eine sehr gute herbizide Wirkung (80% bis 100%) bei einer Aufwandmenge von 1900 g Aktivsubstanz pro Hektar: I.1-16. Die folgenden erfindungsgemäßen Verbindungen hatten weiterhin eine sehr gute herbizide Wirkung (> 80%) bei einer Aufwandmenge von 950 g Aktivsubstanz pro Hektar: I.1-16. Die folgenden erfindungsgemäßen Verbindungen zeigten eine sehr gute herbizide Wirkung (> 80%) bei einer Aufwandmenge von 475 g Aktivsubstanz pro Hektar: I.1-16. Darüber hinaus zeigten die folgenden erfindungsgemäßen Verbindungen eine sehr gute herbizide Wirkung (> 80%) bei einer Aufwandmenge von 238 g Aktivsubstanz pro Hektar: I.1-16.

A3. Wirkung gegen POAAN: In diesem Test hatten beispielsweise die folgenden erfindungsgemäßen Verbindungen eine sehr gute herbizide Wirkung (80% bis 100%) bei einer Aufwandmenge von 1900 g Aktivsubstanz pro Hektar: I.1-16, I.1-32, I.1-35, I.1-147, I.1-153, I.1-162. Die folgenden erfindungsgemäßen Verbindungen hatten weiterhin eine sehr gute herbizide Wirkung (> 80%) bei einer Aufwandmenge von 950 g Aktivsubstanz pro Hektar: I.1-16, I.1-32, I.1-35, I.1-147, I.1-153, I.1-162. Die folgenden erfindungsgemäßen Verbindungen zeigten eine sehr gute herbizide Wirkung (> 80%) bei einer Aufwandmenge von 475 g Aktivsubstanz pro Hektar: I.1-16, I.1-32, I.1-35, I.1-147, I.1-153. Darüber hinaus zeigten die folgenden erfindungsgemäßen Verbindungen eine sehr gute herbizide Wirkung (> 80%) bei einer Aufwandmenge von 238 g Aktivsubstanz pro Hektar: I.1-16, I.1-32, I.1-35, I.1-147, I.1-153.

A4. Wirkung gegen SETVI: In diesem Test hatten beispielsweise die folgenden erfindungsgemäßen Verbindungen eine sehr gute herbizide Wirkung (80% bis 100%) bei einer Aufwandmenge von 1900 g Aktivsubstanz pro Hektar: I.1-16. Die folgenden erfindungsgemäßen Verbindungen hatten weiterhin eine sehr gute herbizide Wirkung (> 80%) bei einer Aufwandmenge von 950 g Aktivsubstanz pro Hektar: I.1-16. Die folgenden erfindungsgemäßen Verbindungen zeigten eine sehr gute herbizide Wirkung (> 80%) bei einer Aufwandmenge von 475 g Aktivsubstanz pro Hektar: I.1-16. Darüber hinaus zeigten die folgenden erfindungsgemäßen Verbindungen eine sehr gute herbizide Wirkung (> 80%) bei einer Aufwandmenge von 238 g Aktivsubstanz pro Hektar: I.1-16.

A5. Wirkung gegen DIPTE: In diesem Test hatten beispielsweise die folgenden erfindungsgemäßen Verbindungen eine sehr gute herbizide Wirkung (80% bis 100%) bei einer Aufwandmenge von 1900 g Aktivsubstanz pro Hektar: I.1-16. Die folgenden erfindungsgemäßen Verbindungen hatten weiterhin eine sehr gute herbizide Wirkung (> 80%) bei einer Aufwandmenge von 950 g Aktivsubstanz pro Hektar: I.1-16. Die folgenden erfindungsgemäßen Verbindungen zeigten eine sehr gute herbizide Wirkung (> 80%) bei einer Aufwandmenge von 475 g Aktivsubstanz pro Hektar: I.1-16. Darüber hinaus zeigten die folgenden erfindungsgemäßen Verbindungen eine sehr gute herbizide Wirkung (> 80%) bei einer Aufwandmenge von 238 g Aktivsubstanz pro Hektar: I.1-16.

A6. Wirkung gegen MATCH: In diesem Test hatten beispielsweise die folgenden erfindungsgemäßen Verbindungen eine sehr gute herbizide Wirkung (80% bis 100%) bei einer Aufwandmenge von 1900 g Aktivsubstanz pro Hektar: I.1-16, I.1-32, I.1-35, I.1-153, I.1-147, I.1-162. Die folgenden erfindungsgemäßen Verbindungen hatten weiterhin eine sehr gute herbizide Wirkung (> 80%) bei einer Aufwandmenge von 950 g Aktivsubstanz pro Hektar: I.1-16, I.1-32, I.1-35, I.1-153, I.1-147, I.1-162. Die folgenden erfindungsgemäßen Verbindungen zeigten eine sehr gute herbizide Wirkung (> 80%) bei einer Aufwandmenge von 475 g Aktivsubstanz pro Hektar: I.1-16, I.1-32, I.1-35, I.1-153, I.1-147, I.1-162. Darüber hinaus zeigten die folgenden erfindungsgemäßen Verbindungen eine sehr gute herbizide Wirkung (> 80%) bei einer Aufwandmenge von 238 g Aktivsubstanz pro Hektar: I.1-16, I.1-32, I.1-35, I.1-153, I.1-147, I.1-162.

A7. Wirkung gegen STEME: In diesem Test hatten beispielsweise die folgenden erfindungsgemäßen Verbindungen eine sehr gute herbizide Wirkung (80% bis 100%) bei einer Aufwandmenge von 1900 g Aktivsubstanz pro Hektar: I.1-16, I.1-147. Die folgenden erfindungsgemäßen Verbindungen hatten weiterhin eine sehr gute herbizide Wirkung (> 80%) bei einer Aufwandmenge von 950 g Aktivsubstanz pro Hektar: I.1-16, I.1-147. Die folgenden erfindungsgemäßen Verbindungen zeigten eine sehr gute herbizide Wirkung (> 80%) bei einer Aufwandmenge von 475 g Aktivsubstanz pro Hektar: I.1-16, I.1-147. Darüber hinaus zeigten die folgenden erfindungsgemäßen Verbindungen eine sehr gute herbizide Wirkung (> 80%) bei einer Aufwandmenge von 238 g Aktivsubstanz pro Hektar: I.1-16, I.1-147.

A8. Wirkung gegen VERPE: In diesem Test hatten beispielsweise die folgenden erfindungsgemäßen Verbindungen eine sehr gute herbizide Wirkung (80% bis 100%) bei einer Aufwandmenge von 1900 g Aktivsubstanz pro Hektar: I.1-16. Die folgenden erfindungsgemäßen Verbindungen hatten weiterhin eine sehr gute herbizide Wirkung (> 80%) bei einer Aufwandmenge von 950 g Aktivsubstanz pro Hektar: I.1-16. Die folgenden erfindungsgemäßen Verbindungen zeigten eine sehr gute herbizide Wirkung (> 80%) bei einer Aufwandmenge von 475 g Aktivsubstanz pro Hektar: I.1-16. Darüber hinaus zeigten die folgenden erfindungsgemäßen Verbindungen eine sehr gute herbizide Wirkung (> 80%) bei einer Aufwandmenge von 238 g Aktivsubstanz pro Hektar: I.1-16.

Wie die oben aufgeführten Ergebnisse zeigen, weisen erfindungsgemäße Verbindungen bei Behandlung im frühen Nachauflauf eine gute herbizide Wirksamkeit gegen Schadpflanzen auf. Beispielsweise haben dabei die Verbindungen Nr. 1.1-16 im oben beschriebenen Nachauflaufverfahren eine sehr gute herbizide Wirkung (80% bis 100% herbizide Wirkung) gegen Schadpflanzen wie Agrostis tenuis, Diplotaxis muralis, Diplotaxis tenuis, Lolium perenne, Matricaria chamomilla, Poa annua, Setaria viridis, Stellaria media und Veronica persica bei Aufwandmengen von 1900 / 950 / 475 / 238 g Aktivsubstanz pro Hektar.

### B. Herbizide Wirkung im Vorauflauf

Samen von mono- bzw. dikotylen Unkrautpflanzen wurden in Kunststoff- oder organischen Pflanztöpfen ausgelegt und mit Erde abgedeckt. Die in Form von benetzbaren Pulvern (WP) oder als Emulsionskonzentrate (EC) formulierten erfindungsgemäßen Verbindungen wurden dann als wässrige Suspension bzw. Emulsion unter Zusatz von 0,5% Additiv mit einer Wasseraufwandmenge von umgerechnet 600 l/ha auf die Oberfläche der Abdeckerde appliziert. Nach der Behandlung wurden die Töpfe im Gewächshaus aufgestellt und unter guten Wachstumsbedingungen für die Testpflanzen gehalten. Nach ca. 3 Wochen wurde die Wirkung der Präparate visuell im Vergleich zu unbehandelten Kontrollen in Prozentwerten bonitiert. Beispielsweise bedeutet 100% Wirkung = Pflanzen sind abgestorben, 0% Wirkung = wie Kontrollpflanzen.

In den nachstehenden Tabellen B1 bis B17 sind die Wirkungen ausgewählter Verbindungen der allgemeinen Formel (I) gemäß den Tabellen 1 und 2 auf verschiedene Schad- und Kulturpflanzen und einer Aufwandmenge entsprechend 1280 g/ha oder niedriger, die gemäß zuvor genannter Versuchsvorschrift erhalten wurden, dargestellt.

**Tabelle B1a: Vorauflaufwirkung bei einer Aufwandmenge von 1280 g/ha gegen ABUTH in %**

| Verbindung | Aufwandmenge [g/ha] | ABUTH |
|---|---|---|
| I.1-162 | 1280 | 80 |

**Tabelle B1b: Vorauflaufwirkung bei einer Aufwandmenge von 320 g/ha gegen ABUTH in %**

| Verbindung | Aufwandmenge [g/ha] | ABUTH |
|---|---|---|
| I.1-16 | 320 | 100 |
| I.1-1 | 320 | 100 |
| I.1-32 | 320 | 80 |

**Tabelle B1c: Vorauflaufwirkung bei einer Aufwandmenge von 80 g/ha gegen ABUTH in %**

| Verbindung | Aufwandmenge [g/ha] | ABUTH |
|---|---|---|
| I.1-16 | 80 | 100 |
| I.1-1 | 80 | 100 |
| I.1-22 | 80 | 80 |

**Tabelle B2a: Vorauflaufwirkung bei einer Aufwandmenge von 1280 g/ha gegen ALOMY in %**

| Verbindung | Aufwandmenge [g/ha] | ALOMY |
|---|---|---|
| I.1-162 | 1280 | 90 |
| I.1-153 | 1280 | 80 |

**Tabelle B2b: Vorauflaufwirkung bei einer Aufwandmenge von 320 g/ha gegen ALOMY in %**

| Verbindung | Aufwandmenge [g/ha] | ALOMY |
|---|---|---|
| I.1-1 | 320 | 100 |
| I.1-162 | 320 | 80 |
| I.1-147 | 320 | 90 |
| I.1-16 | 320 | 100 |
| I.1-22 | 320 | 100 |

**Tabelle B2c: Vorauflaufwirkung bei einer Aufwandmenge von 80 g/ha gegen ALOMY in %**

| Verbindung | Aufwandmenge [g/ha] | ALOMY |
|---|---|---|
| I.1-1 | 80 | 100 |
| I.1-18 | 80 | 100 |
| I.1-147 | 80 | 90 |
| I.1-16 | 80 | 100 |
| I.1-32 | 80 | 90 |
| I.1-35 | 80 | 90 |
| I.1-22 | 80 | 80 |

**Tabelle B2d: Vorauflaufwirkung bei einer Aufwandmenge von 20 g/ha gegen ALOMY in %**

| Verbindung | Aufwandmenge [g/ha] | ALOMY |
|---|---|---|
| I.1-32 | 20 | 90 |
| I.1-35 | 20 | 90 |

**Tabelle B3a: Vorauflaufwirkung bei einer Aufwandmenge von 1280 g/ha gegen AMARE in %**

| Verbindung | Aufwandmenge [g/ha] | AMARE |
|---|---|---|
| I.1-162 | 1280 | 100 |
| I.1-153 | 1280 | 100 |

**Tabelle B3b: Vorauflaufwirkung bei einer Aufwandmenge von 320 g/ha gegen AMARE in %**

| Verbindung | Aufwandmenge [g/ha] | AMARE |
|---|---|---|
| I.1-16 | 320 | 100 |
| I.1-99 | 320 | 100 |
| I.1-162 | 320 | 100 |
| I.1-147 | 320 | 100 |
| I.1-153 | 320 | 100 |
| I.1-22 | 320 | 100 |

**Tabelle B3c: Vorauflaufwirkung bei einer Aufwandmenge von 80 g/ha gegen AMARE in %**

| Verbindung | Aufwandmenge [g/ha] | AMARE |
|---|---|---|
| I.1-16 | 80 | 100 |
| I.1-18 | 80 | 100 |
| I.1-147 | 80 | 100 |
| I.1-32 | 80 | 100 |
| I.1-35 | 80 | 100 |
| I.1-22 | 80 | 100 |

**Tabelle B3d: Vorauflaufwirkung bei einer Aufwandmenge von 20 g/ha gegen AMARE in %**

| Verbindung | Aufwandmenge [g/ha] | AMARE |
|---|---|---|
| I.1-18 | 20 | 100 |
| I.1-32 | 20 | 100 |
| I.1-35 | 20 | 100 |

**Tabelle B3e: Vorauflaufwirkung bei einer Aufwandmenge von 5 g/ha gegen AMARE in %**

| Verbindung | Aufwandmenge [g/ha] | AMARE |
|---|---|---|
| I.1-18 | 5 | 100 |

**Tabelle B4a: Vorauflaufwirkung bei einer Aufwandmenge von 1280 g/ha gegen AVEFA in %**

| Verbindung | Aufwandmenge [g/ha] | AVEFA |
|---|---|---|
| I.1-162 | 1280 | 90 |
| I.1-153 | 1280 | 80 |

**Tabelle B4b: Vorauflaufwirkung bei einer Aufwandmenge von 320 g/ha gegen AVEFA in %**

| Verbindung | Aufwandmenge [g/ha] | AVEFA |
|---|---|---|
| I.1-1 | 320 | 100 |
| I.1-147 | 320 | 80 |
| I.1-16 | 320 | 100 |
| I.1-22 | 320 | 90 |

**Tabelle B4c: Vorauflaufwirkung bei einer Aufwandmenge von 80 g/ha gegen AVEFA in %**

| Verbindung | Aufwandmenge [g/ha] | AVEFA |
|---|---|---|
| I.1-1 | 80 | 80 |
| I.1-18 | 80 | 100 |
| I.1-32 | 80 | 90 |
| I.1-16 | 80 | 90 |
| I.1-35 | 80 | 80 |

**Tabelle B4d: Vorauflaufwirkung bei einer Aufwandmenge von 20 g/ha gegen AVEFA in %**

| Verbindung | Aufwandmenge [g/ha] | AVEFA |
|---|---|---|
| I.1-32 | 20 | 80 |

**Tabelle B5a: Vorauflaufwirkung bei einer Aufwandmenge von 320 g/ha gegen DIGSA in %**

| Verbindung | Aufwandmenge [g/ha] | DIGSA |
|---|---|---|
| I.1-1 | 320 | 100 |
| I.1-16 | 320 | 100 |

**Tabelle B5b: Vorauflaufwirkung bei einer Aufwandmenge von 800 g/ha gegen DIGSA in %**

| Verbindung | Aufwandmenge [g/ha] | DIGSA |
|---|---|---|
| I.1-1 | 80 | 100 |
| I.1-18 | 80 | 100 |
| I.1-16 | 80 | 100 |

**Tabelle B5c: Vorauflaufwirkung bei einer Aufwandmenge von 20 g/ha gegen DIGSA in %**

| Verbindung | Aufwandmenge [g/ha] | DIGSA |
|---|---|---|
| I.1-1 | 20 | 80 |
| I.1-18 | 20 | 100 |
| I.1-16 | 20 | 100 |

**Tabelle B6a: Vorauflaufwirkung bei einer Aufwandmenge von 1280 g/ha gegen ECHCG in %**

| Verbindung | Aufwandmenge [g/ha] | ECHCG |
|---|---|---|
| I.1-162 | 1280 | 100 |
| I.1-153 | 1280 | 100 |

**Tabelle B6b: Vorauflaufwirkung bei einer Aufwandmenge von 320 g/ha gegen ECHCG in %**

| Verbindung | Aufwandmenge [g/ha] | ECHCG |
|---|---|---|
| I.1-1 | 320 | 100 |
| I.1-162 | 320 | 90 |
| I.1-147 | 320 | 100 |
| I.1-16 | 320 | 100 |
| I.1-153 | 320 | 90 |
| I.1-22 | 320 | 100 |

**Tabelle D6c: Vorauflaufwirkung bei einer Aufwandmenge von 80 g/ha gegen ECHCG in %**

| Verbindung | Aufwandmenge [g/ha] | ECHCG |
|---|---|---|
| I.1-1 | 80 | 100 |
| I.1-18 | 80 | 100 |
| I.1-147 | 80 | 90 |
| I.1-16 | 80 | 100 |
| I.1-32 | 80 | 100 |
| I.1-35 | 80 | 100 |
| I.1-22 | 80 | 100 |

**Tabelle B6d: Vorauflaufwirkung bei einer Aufwandmenge von 20 g/ha gegen ECHCG in %**

| Verbindung | Aufwandmenge [g/ha] | ECHCG |
|---|---|---|
| I.1-1 | 20 | 100 |
| I.1-18 | 20 | 90 |
| I.1-16 | 20 | 100 |

**Tabelle B7a: Vorauflaufwirkung bei einer Aufwandmenge von 1280 g/ha gegen LOLMU in %**

| Verbindung | Aufwandmenge [g/ha] | LOLMU |
|---|---|---|
| I.1-162 | 1280 | 100 |
| I.1-153 | 1280 | 100 |

**Tabelle B7b: Vorauflaufwirkung bei einer Aufwandmenge von 320 g/ha gegen LOLMU in %**

| Verbindung | Aufwandmenge [g/ha] | LOLMU |
|---|---|---|
| I.1-162 | 320 | 80 |
| I.1-147 | 320 | 100 |
| I.1-153 | 320 | 90 |
| I.1-22 | 320 | 100 |

**Tabelle B7c: Vorauflaufwirkung bei einer Aufwandmenge von 80 g/ha gegen LOLMU in %**

| Verbindung | Aufwandmenge [g/ha] | LOLMU |
|---|---|---|
| I.1-18 | 80 | 100 |
| I.1-147 | 80 | 80 |
| I.1-32 | 80 | 100 |
| I.1-35 | 80 | 80 |
| I.1-22 | 80 | 100 |

**Tabelle B7d: Vorauflaufwirkung bei einer Aufwandmenge von 20 g/ha gegen LOLMU in %**

| Verbindung | Aufwandmenge [g/ha] | LOLMU |
|---|---|---|
| I.1-18 | 20 | 100 |
| I.1-32 | 20 | 100 |

**Tabelle B8a: Vorauflaufwirkung bei einer Aufwandmenge von 1280 g/ha gegen MATIN in %**

| Verbindung | Aufwandmenge [g/ha] | MATIN |
|---|---|---|
| I.1-162 | 1280 | 90 |
| I.1-153 | 1280 | 100 |

**Tabelle B8b: Vorauflaufwirkung bei einer Aufwandmenge von 320 g/ha gegen MATIN in %**

| Verbindung | Aufwandmenge [g/ha] | MATIN |
|---|---|---|
| I.1-162 | 320 | 90 |
| I.1-147 | 320 | 90 |
| I.1-22 | 320 | 100 |

**Tabelle B8c: Vorauflaufwirkung bei einer Aufwandmenge von 80 g/ha gegen MATIN in %**

| Verbindung | Aufwandmenge [g/ha] | MATIN |
|---|---|---|
| I.1-18 | 80 | 100 |
| I.1-32 | 80 | 100 |
| I.1-35 | 80 | 90 |
| I.1-22 | 80 | 100 |

**Tabelle B8d: Vorauflaufwirkung bei einer Aufwandmenge von 20 g/ha gegen MATIN in %**

| Verbindung | Aufwandmenge [g/ha] | MATIN |
|---|---|---|
| I.1-18 | 20 | 90 |

**Tabelle B9a: Vorauflaufwirkung bei einer Aufwandmenge von 320 g/ha gegen PHBPU in %**

| Verbindung | Aufwandmenge [g/ha] | PHBPU |
|---|---|---|
| I.1-1 | 320 | 100 |
| I.1-16 | 320 | 100 |
| I.1-22 | 320 | 100 |

**Tabelle B9b: Vorauflaufwirkung bei einer Aufwandmenge von 80 g/ha gegen PHBPU in %**

| Verbindung | Aufwandmenge [g/ha] | PHBPU |
|---|---|---|
| I.1-16 | 80 | 80 |
| I.1-32 | 80 | 80 |
| I.1-22 | 80 | 90 |

**Tabelle B10a: Vorauflaufwirkung bei einer Aufwandmenge von 1280 g/ha gegen POLCO in %**

| Verbindung | Aufwandmenge [g/ha] | POLCO |
|---|---|---|
| I.1-162 | 1280 | 100 |
| I.1-153 | 1280 | 100 |

**Tabelle B10b: Vorauflaufwirkung bei einer Aufwandmenge von 320 g/ha gegen POLCO in %**

| Verbindung | Aufwandmenge [g/ha] | POLCO |
|---|---|---|
| I.1-99 | 320 | 80 |
| I.1-1 | 320 | 100 |
| I.1-162 | 320 | 100 |
| I.1-147 | 320 | 100 |
| I.1-16 | 320 | 100 |
| I.1-153 | 320 | 100 |
| I.1-22 | 320 | 100 |

**Tabelle B10c: Vorauflaufwirkung bei einer Aufwandmenge von 80 g/ha gegen POLCO in %**

| Verbindung | Aufwandmenge [g/ha] | POLCO |
|---|---|---|
| I.1-1 | 80 | 100 |
| I.1-18 | 80 | 100 |
| I.1-16 | 80 | 100 |
| I.1-32 | 80 | 100 |
| I.1-35 | 80 | 90 |

**Tabelle B10d: Vorauflaufwirkung bei einer Aufwandmenge von 20 g/ha gegen POLCO in %**

| Verbindung | Aufwandmenge [g/ha] | POLCO |
|---|---|---|
| I.1-1 | 20 | 100 |
| I.1-16 | 20 | 100 |

**Tabelle B11a: Vorauflaufwirkung bei einer Aufwandmenge von 1280 g/ha gegen SETVI in %**

| Verbindung | Aufwandmenge [g/ha] | SETVI |
|---|---|---|
| I.1-162 | 1280 | 100 |
| I.1-153 | 1280 | 100 |

**Tabelle B11b: Vorauflaufwirkung bei einer Aufwandmenge von 320 g/ha gegen SETVI in %**

| Verbindung | Aufwandmenge [g/ha] | SETVI |
|---|---|---|
| I.1-99 | 320 | 90 |
| I.1-1 | 320 | 100 |
| I.1-162 | 320 | 90 |
| I.1-147 | 320 | 90 |
| I.1-16 | 320 | 100 |
| I.1-153 | 320 | 90 |
| I.1-22 | 320 | 100 |

**Tabelle B11c: Vorauflaufwirkung bei einer Aufwandmenge von 80 g/ha gegen SETVI in %**

| Verbindung | Aufwandmenge [g/ha] | SETVI |
|---|---|---|
| I.1-1 | 80 | 100 |
| I.1-18 | 80 | 100 |
| I.1-147 | 80 | 90 |
| I.1-16 | 80 | 100 |
| I.1-32 | 80 | 100 |
| I.1-35 | 80 | 100 |
| I.1-22 | 80 | 100 |

**Tabelle B11d: Vorauflaufwirkung bei einer Aufwandmenge von 20 g/ha gegen SETVI in %**

| Verbindung | Aufwandmenge [g/ha] | SETVI |
|---|---|---|
| I.1-1 | 20 | 100 |
| I.1-18 | 20 | 100 |
| I.1-16 | 20 | 100 |
| I.1-32 | 20 | 100 |

**Tabelle B11e: Vorauflaufwirkung bei einer Aufwandmenge von 5 g/ha gegen SETVI in %**

| Verbindung | Aufwandmenge [g/ha] | SETVI |
|---|---|---|
| I.1-18 | 5 | 90 |

**Tabelle B12a: Vorauflaufwirkung bei einer Aufwandmenge von 1280 g/ha gegen VERPE in %**

| Verbindung | Aufwandmenge [g/ha] | VERPE |
|---|---|---|
| I.1-19 | 1280 | 100 |

**Tabelle B12b: Vorauflaufwirkung bei einer Aufwandmenge von 320 g/ha gegen VERPE in %**

| Verbindung | Aufwandmenge [g/ha] | VERPE |
|---|---|---|
| I.1-99 | 320 | 100 |
| I.1-1 | 320 | 100 |
| I.1-147 | 320 | 100 |
| I.1-19 | 320 | 90 |
| I.1-16 | 320 | 100 |
| I.1-22 | 320 | 100 |

**Tabelle B12c: Vorauflaufwirkung bei einer Aufwandmenge von 80 g/ha gegen VERPE in %**

| Verbindung | Aufwandmenge [g/ha] | VERPE |
|---|---|---|
| I.1-1 | 80 | 100 |
| I.1-18 | 80 | 100 |
| I.1-147 | 80 | 100 |
| I.1-16 | 80 | 100 |
| I.1-32 | 80 | 100 |
| I.1-35 | 80 | 100 |
| I.1-22 | 80 | 100 |

**Tabelle B12d: Vorauflaufwirkung bei einer Aufwandmenge von 20 g/ha gegen VERPE in %**

| Verbindung | Aufwandmenge [g/ha] | VERPE |
|---|---|---|
| I.1-1 | 20 | 100 |
| I.1-18 | 20 | 100 |
| I.1-16 | 20 | 100 |
| I.1-32 | 20 | 100 |
| I.1-35 | 20 | 90 |

**Tabelle B12e: Vorauflaufwirkung bei einer Aufwandmenge von 5 g/ha gegen VERPE in %**

| Verbindung | Aufwandmenge [g/ha] | VERPE |
|---|---|---|
| I.1-18 | 5 | 100 |

**Tabelle B13a: Vorauflaufwirkung bei einer Aufwandmenge von 1280 g/ha gegen VIOTR in %**

| Verbindung | Aufwandmenge [g/ha] | VIOTR |
|---|---|---|
| I.1-162 | 1280 | 100 |
| I.1-19 | 1280 | 80 |
| I.1-153 | 1280 | 100 |

**Tabelle B13b: Vorauflaufwirkung bei einer Aufwandmenge von 320 g/ha gegen VIOTR in %**

| Verbindung | Aufwandmenge [g/ha] | VIOTR |
|---|---|---|
| I.1-1 | 320 | 100 |
| I.1-162 | 320 | 100 |
| I.1-147 | 320 | 100 |
| I.1-16 | 320 | 100 |
| I.1-153 | 320 | 100 |
| I.1-22 | 320 | 100 |

**Tabelle B13c: Vorauflaufwirkung bei einer Aufwandmenge von 80 g/ha gegen VIOTR in %**

| Verbindung | Aufwandmenge [g/ha] | VIOTR |
|---|---|---|
| I.1-1 | 80 | 100 |
| I.1-18 | 80 | 100 |
| I.1-147 | 80 | 100 |
| I.1-16 | 80 | 100 |
| I.1-32 | 80 | 100 |
| I.1-35 | 80 | 100 |
| I.1-22 | 80 | 100 |

**Tabelle B13d: Vorauflaufwirkung bei einer Aufwandmenge von 20 g/ha gegen VIOTR in %**

| Verbindung | Aufwandmenge [g/ha] | VIOTR |
|---|---|---|
| I.1-1 | 20 | 100 |
| I.1-18 | 20 | 100 |
| I.1-16 | 20 | 100 |
| I.1-32 | 20 | 100 |
| I.1-35 | 20 | 100 |

**Tabelle B13e: Vorauflaufwirkung bei einer Aufwandmenge von 5 g/ha gegen VIOTR in %**

| Verbindung | Aufwandmenge [g/ha] | VIOTR |
|---|---|---|
| I.1-18 | 5 | 100 |

**Tabelle B14a: Vorauflaufwirkung bei einer Aufwandmenge von 320 g/ha gegen KCHSC in %**

| Verbindung | Aufwandmenge [g/ha] | KCHSC |
|---|---|---|
| I.1-1 | 320 | 100 |
| I.1-16 | 320 | 100 |

**Tabelle B14b: Vorauflaufwirkung bei einer Aufwandmenge von 80 g/ha gegen KCHSC in %**

| Verbindung | Aufwandmenge [g/ha] | KCHSC |
|---|---|---|
| I.1-1 | 80 | 100 |
| I.1-16 | 80 | 100 |

**Tabelle B14c: Vorauflaufwirkung bei einer Aufwandmenge von 20 g/ha gegen KCHSC in %**

| Verbindung | Aufwandmenge [g/ha] | KCHSC |
|---|---|---|
| I.1-1 | 20 | 80 |

**Tabelle B15: Vorauflaufwirkung bei einer Aufwandmenge von 320 g/ha gegen SEBEX in %**

| Verbindung | Aufwandmenge [g/ha] | SEBEX |
|---|---|---|
| I.1-99 | 320 | 80 |

**Tabelle B16: Vorauflaufwirkung bei einer Aufwandmenge von 320 g/ha gegen SORHA in %**

| Verbindung | Aufwandmenge [g/ha] | SORHA |
|---|---|---|
| I.1-18 | 320 | 80 |

**Tabelle B17a: Vorauflaufwirkung bei einer Aufwandmenge von 320 g/ha gegen CYPES in %**

| Verbindung | Aufwandmenge [g/ha] | CYPES |
|---|---|---|
| I.1-162 | 320 | 80 |

**Tabelle B17b: Vorauflaufwirkung bei einer Aufwandmenge von 80 g/ha gegen CYPES in %**

| Verbindung | Aufwandmenge [g/ha] | CYPES |
|---|---|---|
| I.1-32 | 80 | 90 |

Wie die Ergebnisse zeigen, weisen erfindungsgemäße Verbindungen der allgemeinen Formel (I) gemäß den Tabellen 1 bis 2 bei Behandlung im Nachauflauf eine gute herbizide Wirksamkeit gegen Schadpflanzen auf wie z. B. *Abutilon theophrasti, Alopecurus myosuroides, Amaranthus retroflexus, Digitaria sanguinalis, Echinochloa crus-galli, Lolium rigidum, Matricaria inodora, Pharbitis purpurea, Polygonum convolvulus, Setaria viridis, Veronica persica* und *Viola tricolor* bei einer Aufwandmenge von 1280 g Aktivsubstanz oder weniger pro Hektar, auf.

### C. Wirkung auf Kulturpflanzen im Vorauflauf

Samen von mono- bzw. dikotylen Kulturpflanzen wurden in Kunststoff- oder organischen Pflanztöpfen ausgelegt und mit Erde abgedeckt. Die in Form von benetzbaren Pulvern (WP) oder als Emulsionskonzentrate (EC) formulierten erfindungsgemäßen Verbindungen wurden dann als wässrige Suspension bzw. Emulsion unter Zusatz von 0,5% Additiv mit einer Wasseraufwandmenge von umgerechnet 600 l/ha auf die Oberfläche der Abdeckerde appliziert. Nach der Behandlung wurden die Töpfe im Gewächshaus aufgestellt und unter guten Wachstumsbedingungen für die Testpflanzen gehalten. Nach ca. 3 Wochen wurde die Wirkung der Präparate visuell im Vergleich zu unbehandelten Kontrollen in Prozentwerten bonitiert. Beispielsweise bedeutet 100% Wirkung = Pflanzen sind abgestorben, 0% Wirkung = wie Kontrollpflanzen.

In den nachstehenden Tabellen E1 bis E4 sind die Wirkungen ausgewählter Verbindungen der allgemeinen Formel (I) gemäß der Tabelle 1 auf verschiedene Kulturpflanzen und einer Aufwandmenge entsprechend 320 g/ha und niedriger, die gemäß zuvor genannter Versuchsvorschrift erhalten wurden, dargestellt.

**Tabelle C1a: Vorauflaufwirkung bei 320 g/ha auf ZEAMX in %**

| Verbindung | Dosierung [g/ha] | ZEAMX |
|---|---|---|
| I.1-99 | 320 | 0 |
| I.1-147 | 320 | 20 |
| I.1-19 | 320 | 20 |
| I.1-22 | 320 | 0 |

**Tabelle C1b: Vorauflaufwirkung bei 80 g/ha auf ZEAMX in %**

| Verbindung | Dosierung [g/ha] | ZEAMX |
|---|---|---|
| I.1-147 | 80 | 0 |
| I.1-16 | 80 | 20 |
| I.1-32 | 80 | 0 |
| I.1-35 | 80 | 0 |
| I.1-22 | 80 | 0 |

**Tabelle C1c: Vorauflaufwirkung bei 20 g/ha auf ZEAMX in %**

| Verbindung | Dosierung [g/ha] | ZEAMX |
|---|---|---|
| I.1-1 | 20 | 0 |
| I.1-18 | 20 | 20 |
| I.1-16 | 20 | 0 |
| I.1-32 | 20 | 0 |
| I.1-35 | 20 | 0 |

**Tabelle C1d: Vorauflaufwirkung bei 5 g/ha auf ZEAMX in %**

| Verbindung | Dosierung [g/ha] | ZEAMX |
|---|---|---|
| I.1-18 | 5 | 0 |

**Tabelle C2a: Vorauflaufwirkung bei 320 g/ha auf TRZAS in %**

| Verbindung | Dosierung [g/ha] | TRZAS |
|---|---|---|
| I.1-99 | 320 | 20 |
| I.1-19 | 320 | 20 |
| I.1-153 | 320 | 20 |

**Tabelle C2b: Vorauflaufwirkung bei 80 g/ha auf TRZAS in %**

| Verbindung | Dosierung [g/ha] | TRZAS |
|---|---|---|
| I.1-32 | 80 | 0 |
| I.1-35 | 80 | 20 |

**Tabelle C2c: Vorauflaufwirkung bei 20 g/ha auf TRZAS in %**

| Verbindung | Dosierung [g/ha] | TRZAS |
|---|---|---|
| I.1-1 | 20 | 0 |
| I.1-18 | 20 | 0 |
| I.1-16 | 20 | 20 |
| I.1-32 | 20 | 0 |
| I.1-35 | 20 | 0 |

**Tabelle C3a: Vorauflaufwirkung bei 320 g/ha auf ORYSA in %**

| Verbindung | Dosierung [g/ha] | ORYSA |
|---|---|---|
| I.1-99 | 320 | 0 |

**Tabelle C3b: Vorauflaufwirkung bei 80 g/ha auf ORYSA in %**

| Verbindung | Dosierung [g/ha] | ORYSA |
|---|---|---|
| I.1-99 | 80 | 0 |

**Tabelle C3c: Vorauflaufwirkung bei 20 g/ha auf ORYSA in %**

| Verbindung | Dosierung [g/ha] | ORYSA |
|---|---|---|
| I.1-99 | 80 | 0 |
| I.1-18 | 80 | 20 |

**Tabelle C4a: Vorauflaufwirkung bei 80 g/ha auf BRSNW in %**

| Verbindung | Dosierung [g/ha] | BRSNW |
|---|---|---|
| I.1-35 | 80 | 0 |

**Tabelle C4b: Vorauflaufwirkung bei 20 g/ha auf BRSNW in %**

| Verbindung | Dosierung [g/ha] | BRSNW |
|---|---|---|
| I.1-1 | 20 | 20 |
| I.1-35 | 20 | 0 |

Wie die Ergebnisse zeigen, weisen erfindungsgemäße Verbindungen der allgemeinen Formel (I) bei Behandlung im Vorauflauf eine gute Kulturpflanzenverträglichkeit bei Organismen, wie *Zea mays, Brassica napus, Oryza sativa und Triticum aestivum* bei einer Aufwandmenge von

320 g oder weniger pro Hektar.

### D. Herbizide Wirkung im Nachauflauf

Samen von mono- bzw. dikotylen Unkrautpflanzen wurden in Kunststoff- oder Holzfasertöpfen in sandigem Lehmboden ausgelegt, mit Erde abgedeckt und im Gewächshaus unter kontrollierten Wachstumsbedingungen angezogen. 2 bis 3 Wochen nach der Aussaat wurden die Versuchspflanzen im Einblattstadium behandelt. Die in Form von benetzbaren Pulvern (WP) oder als Emulsionskonzentrate (EC) formulierten erfindungsgemäßen Verbindungen wurden dann als wässrige Suspension bzw. Emulsion unter Zusatz von 0,5% Additiv mit einer Wasseraufwandmenge von umgerechnet 600 l/ha auf die grünen Pflanzenteile gesprüht. Nach ca. 3 Wochen Standzeit der Versuchspflanzen im Gewächshaus, unter optimalen Wachstumsbedingungen, wurde die Wirkung der Präparate visuell im Vergleich zu unbehandelten Kontrollen bonitiert.

Beispielsweise bedeutet 100% Wirkung = Pflanzen sind abgestorben, 0% Wirkung = wie Kontrollpflanzen.

In den nachstehenden Tabellen D1 bis D16 sind die Wirkungen ausgewählter Verbindungen der allgemeinen Formel (I) gemäß der Tabelle 1 auf verschiedene Schadpflanzen und einer Aufwandmenge entsprechend 1280 g/ha und niedriger, die gemäß zuvor genannter Versuchsvorschrift erhalten wurden, dargestellt.

**Tabelle D1a: Nachauflaufwirkung bei einer Aufwandmenge von 1280 g/ha gegen ABUTH in %**

| Verbindung | Aufwandmenge [g/ha] | ABUTH |
|---|---|---|
| I.1-162 | 1280 | 80 |
| I.1-153 | 1280 | 80 |

**Tabelle D1b: Nachauflaufwirkung bei einer Aufwandmenge von 320 g/ha gegen ABUTH in %**

| Verbindung | Aufwandmenge [g/ha] | ABUTH |
|---|---|---|
| I.1-1 | 320 | 90 |
| I.1-147 | 320 | 90 |
| I.1-16 | 320 | 90 |

**Tabelle D2a: Nachauflaufwirkung bei einer Aufwandmenge von 1280 g/ha gegen ALOMY in %**

| Verbindung | Aufwandmenge [g/ha] | ALOMY |
|---|---|---|
| I.1-16 | 1280 | 100 |
| I.1-1 | 1280 | 100 |

**Tabelle D2b: Nachauflaufwirkung bei einer Aufwandmenge von 320 g/ha gegen ALOMY in %**

| Verbindung | Aufwandmenge [g/ha] | ALOMY |
|---|---|---|
| I.1-1 | 320 | 100 |
| I.1-16 | 320 | 100 |

**Tabelle D2c: Nachauflaufwirkung bei einer Aufwandmenge von 80 g/ha gegen ALOMY in %**

| Verbindung | Aufwandmenge [g/ha] | ALOMY |
|---|---|---|
| I.1-32 | 80 | 90 |

**Tabelle D3a: Nachauflaufwirkung bei einer Aufwandmenge von 1280 g/ha gegen AMARE in %**

| Verbindung | Aufwandmenge [g/ha] | AMARE |
|---|---|---|
| I.1-162 | 1280 | 100 |
| I.1-153 | 1280 | 100 |

**Tabelle D3b: Nachauflaufwirkung bei einer Aufwandmenge von 320 g/ha gegen AMARE in %**

| Verbindung | Aufwandmenge [g/ha] | AMARE |
|---|---|---|
| I.1-162 | 320 | 90 |
| I.1-147 | 320 | 100 |
| I.1-153 | 320 | 100 |
| I.1-22 | 320 | 100 |

**Tabelle D3c: Nachauflaufwirkung bei einer Aufwandmenge von 80 g/ha gegen AMARE in %**

| Verbindung | Aufwandmenge [g/ha] | AMARE |
|---|---|---|
| I.1-147 | 80 | 90 |
| I.1-32 | 80 | 90 |
| I.1-35 | 80 | 90 |
| I.1-22 | 80 | 90 |

**Tabelle D3d: Nachauflaufwirkung bei einer Aufwandmenge von 20 g/ha gegen AMARE in %**

| Verbindung | Aufwandmenge [g/ha] | AMARE |
|---|---|---|
| I.1-32 | 20 | 90 |
| I.1-35 | 20 | 90 |

**Tabelle D4: Nachauflaufwirkung bei einer Aufwandmenge von 320 g/ha gegen AVEFA in %**

| Verbindung | Aufwandmenge [g/ha] | AVEFA |
|---|---|---|
| I.1-1 | 320 | 100 |
| I.1-16 | 320 | 100 |
| I.1-22 | 320 | 100 |

**Tabelle D5: Nachauflaufwirkung bei einer Aufwandmenge von 320 g/ha gegen DIGSA in %**

| Verbindung | Aufwandmenge [g/ha] | DIGSA |
|---|---|---|
| I.1-16 | 320 | 80 |

**Tabelle D6a: Nachauflaufwirkung bei einer Aufwandmenge von 1280 g/ha gegen ECHCG in %**

| Verbindung | Aufwandmenge [g/ha] | ECHCG |
|---|---|---|
| I.1-153 | 1280 | 80 |

**Tabelle D6b: Nachauflaufwirkung bei einer Aufwandmenge von 320 g/ha gegen ECHCG in %**

| Verbindung | Aufwandmenge [g/ha] | ECHCG |
|---|---|---|
| I.1-1 | 320 | 100 |
| I.1-16 | 320 | 100 |
| I.1-22 | 320 | 90 |
| I.1-2232 | 320 | 90 |

**Tabelle D6c: Nachauflaufwirkung bei einer Aufwandmenge von 80 g/ha gegen ECHCG in %**

| Verbindung | Aufwandmenge [g/ha] | ECHCG |
|---|---|---|
| I.1-1 | 80 | 80 |
| I.1-16 | 80 | 100 |
| I.1-32 | 80 | 80 |
| I.1-22 | 80 | 90 |

**Tabelle D6d: Nachauflaufwirkung bei einer Aufwandmenge von 20 g/ha gegen ECHCG in %**

| Verbindung | Aufwandmenge [g/ha] | ECHCG |
|---|---|---|
| I.1-32 | 80 | 80 |

**Tabelle D7a: Nachauflaufwirkung bei einer Aufwandmenge von 1280 g/ha gegen LOLMU in %**

| Verbindung | Aufwandmenge [g/ha] | LOLMU |
|---|---|---|
| I.1-162 | 1280 | 80 |
| I.1-153 | 1280 | 90 |

**Tabelle D7b: Nachauflaufwirkung bei einer Aufwandmenge von 320 g/ha gegen LOLMU in %**

| Verbindung | Aufwandmenge [g/ha] | LOLMU |
|---|---|---|
| I.1-22 | 320 | 90 |

**Tabelle D7c: Nachauflaufwirkung bei einer Aufwandmenge von 80 g/ha gegen LOLMU in %**

| Verbindung | Aufwandmenge [g/ha] | LOLMU |
|---|---|---|
| I.1-32 | 80 | 80 |

**Tabelle D8a: Nachauflaufwirkung bei einer Aufwandmenge von 1280 g/ha gegen MATIN in %**

| Verbindung | Aufwandmenge [g/ha] | MATIN |
|---|---|---|
| I.1-162 | 1280 | 80 |

**Tabelle D8b: Nachauflaufwirkung bei einer Aufwandmenge von 320 g/ha gegen MATIN in %**

| Verbindung | Aufwandmenge [g/ha] | MATIN |
|---|---|---|
| I.1-16 | 320 | 80 |

**Tabelle D9a: Nachauflaufwirkung bei einer Aufwandmenge von 1280 g/ha gegen PHBPU in %**

| Verbindung | Aufwandmenge [g/ha] | PHBPU |
|---|---|---|
| I.1-162 | 1280 | 90 |
| I.1-153 | 1280 | 90 |

**Tabelle D9b: Nachauflaufwirkung bei einer Aufwandmenge von 320 g/ha gegen PHBPU in %**

| Verbindung | Aufwandmenge [g/ha] | PHBPU |
|---|---|---|
| I.1-1 | 320 | 90 |
| I.1-16 | 320 | 100 |
| I.1-22 | 320 | 100 |

**Tabelle D9c: Nachauflaufwirkung bei einer Aufwandmenge von 80 g/ha gegen PHBPU in %**

| Verbindung | Aufwandmenge [g/ha] | PHBPU |
|---|---|---|
| I.1-1 | 80 | 80 |
| I.1-32 | 80 | 100 |
| I.1-35 | 80 | 90 |
| I.1-22 | 80 | 90 |

**Tabelle D9d: Nachauflaufwirkung bei einer Aufwandmenge von 20 g/ha gegen PHBPU in %**

| Verbindung | Aufwandmenge [g/ha] | PHBPU |
|---|---|---|
| I.1-32 | 20 | 90 |
| I.1-35 | 20 | 80 |

**Tabelle D10a: Nachauflaufwirkung bei einer Aufwandmenge von 1280 g/ha gegen POLCO in %**

| Verbindung | Aufwandmenge [g/ha] | POLCO |
|---|---|---|
| I.1-162 | 1280 | 90 |

**Tabelle D10b: Nachauflaufwirkung bei einer Aufwandmenge von 320 g/ha gegen POLCO in %**

| Verbindung | Aufwandmenge [g/ha] | POLCO |
|---|---|---|
| I.1-16 | 320 | 100 |

**Tabelle D10c: Nachauflaufwirkung bei einer Aufwandmenge von 80 g/ha gegen POLCO in %**

| Verbindung | Aufwandmenge [g/ha] | POLCO |
|---|---|---|
| I.1-16 | 80 | 80 |

**Tabelle D11a: Nachauflaufwirkung bei einer Aufwandmenge von 320 g/ha gegen SETVI in %**

| Verbindung | Aufwandmenge [g/ha] | SETVI |
|---|---|---|
| I.1-1 | 320 | 80 |
| I.1-16 | 320 | 80 |
| I.1-22 | 320 | 90 |

**Tabelle D11b: Nachauflaufwirkung bei einer Aufwandmenge von 80 g/ha gegen SETVI in %**

| Verbindung | Aufwandmenge [g/ha] | SETVI |
|---|---|---|
| I.1-32 | 80 | 100 |
| I.1-35 | 80 | 90 |
| I.1-22 | 80 | 90 |

**Tabelle D11c: Nachauflaufwirkung bei einer Aufwandmenge von 20 g/ha gegen SETVI in %**

| Verbindung | Aufwandmenge [g/ha] | SETVI |
|---|---|---|
| I.1-32 | 20 | 80 |

**Tabelle D12a: Nachauflaufwirkung bei einer Aufwandmenge von 320 g/ha gegen VERPE in %**

| Verbindung | Aufwandmenge [g/ha] | VERPE |
|---|---|---|
| I.1-1 | 320 | 90 |
| I.1-147 | 320 | 90 |
| I.1-16 | 320 | 90 |
| I.1-22 | 320 | 90 |

**Tabelle D12b: Nachauflaufwirkung bei einer Aufwandmenge von 80 g/ha gegen VERPE in %**

| Verbindung | Aufwandmenge [g/ha] | VERPE |
|---|---|---|
| I.1-1 | 80 | 90 |
| I.1-147 | 80 | 80 |
| I.1-16 | 80 | 90 |
| I.1-32 | 80 | 90 |
| I.1-35 | 80 | 80 |
| I.1-22 | 80 | 90 |

**Tabelle D12c: Nachauflaufwirkung bei einer Aufwandmenge von 20 g/ha gegen VERPE in %**

| Verbindung | Aufwandmenge [g/ha] | VERPE |
|---|---|---|
| I.1-16 | 20 | 90 |
| I.1-32 | 20 | 80 |

**Tabelle D13a: Nachauflaufwirkung bei einer Aufwandmenge von 320 g/ha gegen VIOTR in %**

| Verbindung | Aufwandmenge [g/ha] | VIOTR |
|---|---|---|
| I.1-1 | 320 | 100 |
| I.1-147 | 320 | 90 |
| I.1-16 | 320 | 100 |
| I.1-22 | 320 | 80 |

**Tabelle D13b: Nachauflaufwirkung bei einer Aufwandmenge von 80 g/ha gegen VIOTR in %**

| Verbindung | Aufwandmenge [g/ha] | VIOTR |
|---|---|---|
| I.1-1 | 80 | 90 |
| I.1-147 | 80 | 80 |
| I.1-16 | 80 | 90 |
| I.1-32 | 80 | 90 |
| I.1-35 | 80 | 80 |
| I.1-22 | 80 | 80 |

**Tabelle D13c: Nachauflaufwirkung bei einer Aufwandmenge von 20 g/ha gegen VIOTR in %**

| Verbindung | Aufwandmenge [g/ha] | VIOTR |
|---|---|---|
| I.1-16 | 20 | 80 |
| I.1-32 | 20 | 80 |

**Tabelle D14: Nachauflaufwirkung bei einer Aufwandmenge von 20 g/ha gegen AMAPA in %**

| Verbindung | Aufwandmenge [g/ha] | AMAPA |
|---|---|---|
| I.1-1 | 20 | 80 |

**Tabelle D15a: Nachauflaufwirkung bei einer Aufwandmenge von 1280 g/ha gegen STEME in %**

| Verbindung | Aufwandmenge [g/ha] | STEME |
|---|---|---|
| I.1-162 | 1280 | 80 |
| I.1-153 | 1280 | 80 |

**Tabelle D15b: Nachauflaufwirkung bei einer Aufwandmenge von 320 g/ha gegen STEME in %**

| Verbindung | Aufwandmenge [g/ha] | STEME |
|---|---|---|
| I.1-147 | 320 | 100 |

**Tabelle D15c: Nachauflaufwirkung bei einer Aufwandmenge von 80 g/ha gegen STEME in %**

| Verbindung | Aufwandmenge [g/ha] | STEME |
|---|---|---|
| I.1-147 | 80 | 80 |

**Tabelle D16a: Nachauflaufwirkung bei einer Aufwandmenge von 320 g/ha gegen KCHSC in %**

| Verbindung | Aufwandmenge [g/ha] | KCHSC |
|---|---|---|
| I.1-1 | 320 | 90 |
| I.1-16 | 320 | 100 |

**Tabelle D16b: Nachauflaufwirkung bei einer Aufwandmenge von 80 g/ha gegen KCHSC in %**

| Verbindung | Aufwandmenge [g/ha] | KCHSC |
|---|---|---|
| I.1-16 | 80 | 90 |

Wie die Ergebnisse zeigen, weisen erfindungsgemäße Verbindungen der allgemeinen Formel (I) genmäß den Tabellen 1 bis 2 bei Behandlung im Nachauflauf eine gute herbizide Wirksamkeit gegen Schadpflanzen auf wie z. B. *Abutilon theophrasti, Alopecurus myosuroides, Amaranthus retroflexus, Digitaria sanguinalis, Echinochloa crus-galli, Lolium multiflorum, Matricaria inodora, Pharbitis purpurea, Polygonum convolvulus, Setaria viridis, Veronica persica* und *Viola tricolor* bei einer Aufwandmenge von 1280 g Aktivsubstanz oder weniger pro Hektar, auf.

### E. Wirkung auf Kulturpflanzen im Nachauflauf

Samen von mono- bzw. dikotylen Kulturpflanzen wurden in Kunststoff- oder Holzfasertöpfen in sandigem Lehmboden ausgelegt, mit Erde abgedeckt und im Gewächshaus unter kontrollierten Wachstumsbedingungen angezogen. 2 bis 3 Wochen nach der Aussaat wurden die Versuchspflanzen im Einblattstadium behandelt. Die in Form von benetzbaren Pulvern (WP) oder als Emulsionskonzentrate (EC) formulierten erfindungsgemäßen Verbindungen wurden dann als wässrige Suspension bzw. Emulsion unter Zusatz von 0,5% Additiv mit einer Wasseraufwandmenge von umgerechnet 600 l/ha auf die grünen Pflanzenteile gesprüht. Nach ca. 3 Wochen Standzeit der Versuchspflanzen im Gewächshaus, unter optimalen Wachstumsbedingungen, wurde die Wirkung der Präparate visuell im Vergleich zu unbehandelten Kontrollen bonitiert. Beispielsweise bedeutet 100% Wirkung = Pflanzen sind abgestorben, 0% Wirkung = wie Kontrollpflanzen.

In den nachstehenden Tabellen E1 bis E5 sind die Wirkungen ausgewählter Verbindungen der allgemeinen Formel (I) gemäß der Tabelle 1 auf verschiedene Kulturpflanzen und einer Aufwandmenge entsprechend 1280 g/ha und niedriger, die gemäß zuvor genannter Versuchsvorschrift erhalten wurden, dargestellt.

**Tabelle E1a: Nachauflaufwirkung bei 320 g/ha auf ZEAMX in %**

| Verbindung | Dosierung [g/ha] | ZEAMX |
|---|---|---|
| I.1-153 | 320 | 0 |

**Tabelle E1b: Nachauflaufwirkung bei 80 g/ha auf ZEAMX in %**

| Verbindung | Dosierung [g/ha] | ZEAMX |
|---|---|---|
| I.1-147 | 80 | 20 |
| I.1-32 | 80 | 20 |
| I.1-22 | 80 | 20 |

**Tabelle E1c: Nachauflaufwirkung bei 20 g/ha auf ZEAMX in %**

| Verbindung | Dosierung [g/ha] | ZEAMX |
|---|---|---|
| I.1-1 | 20 | 0 |
| I.1-32 | 20 | 20 |

**Tabelle E2a: Nachauflaufwirkung bei 1280 g/ha auf TRZAS in %**

| Verbindung | Dosierung [g/ha] | TRZAS |
|---|---|---|
| I.1-19 | 1280 | 0 |

**Tabelle E2b: Nachauflaufwirkung bei 320 g/ha auf TRZAS in %**

| Verbindung | Dosierung [g/ha] | TRZAS |
|---|---|---|
| I.1-1 | 320 | 20 |
| I.1-162 | 320 | 20 |
| I.1-147 | 320 | 20 |
| I.1-19 | 320 | 0 |
| I.1-16 | 320 | 20 |
| I.1-153 | 320 | 20 |
| I.1-22 | 320 | 0 |

**Tabelle E2c: Nachauflaufwirkung bei 80 g/ha auf TRZAS in %**

| Verbindung | Dosierung [g/ha] | TRZAS |
|---|---|---|
| I.1-1 | 80 | 0 |
| I.1-147 | 80 | 20 |
| I.1-16 | 80 | 0 |
| I.1-32 | 80 | 0 |
| I.1-35 | 80 | 0 |
| I.1-22 | 80 | 0 |

**Tabelle E2d: Nachauflaufwirkung bei 20 g/ha auf TRZAS in %**

| Verbindung | Dosierung [g/ha] | TRZAS |
|---|---|---|
| | | |
| I.1-16 | 20 | 0 |
| I.1-32 | 20 | 0 |
| I.1-35 | 20 | 0 |

**Tabelle E4a: Nachauflaufwirkung bei 80 g/ha auf BRSNW in %**

| Verbindung | Dosierung [g/ha] | BRSNW |
|---|---|---|
| I.1-16 | 80 | 10 |

**Tabelle E4b: Nachauflaufwirkung bei 20 g/ha auf BRSNW in %**

| Verbindung | Dosierung [g/ha] | BRSNW |
|---|---|---|
| | | |
| I.1-16 | 20 | 0 |
| I.1-32 | 20 | 20 |
| I.1-35 | 20 | 20 |

**Tabelle E5a: Nachauflaufwirkung bei 80 g/ha auf ORYSA in %**

| Verbindung | Dosierung [g/ha] | ORYSA |
|---|---|---|
| I.1-147 | 80 | 20 |
| I.1-35 | 80 | 0 |

**Tabelle E5b: Nachauflaufwirkung bei 20 g/ha auf ORYSA in %**

| Verbindung | Dosierung [g/ha] | ORYSA |
|---|---|---|
| I.1-32 | 20 | 0 |
| I.1-35 | 20 | 0 |

Wie die Ergebnisse zeigen, weisen erfindungsgemäße Verbindungen der allgemeinen Formel (I) bei Behandlung im Nachauflauf eine gute Kulturpflanzenverträglichkeit bei Organismen, wie *Zea mays, Brassica napus, Glycine max und Triticum aestivum* bei einer Aufwandmenge von 1280 g oder weniger pro Hektar.

## Patentansprüche

1. Verbindung der allgemeinen Formel (I) 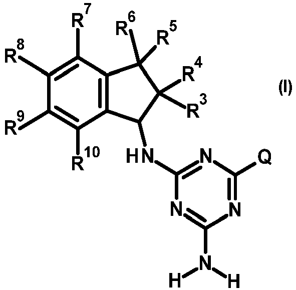 **dadurch gekennzeichnet daß**
Q für eine Gruppe, ausgewählt aus 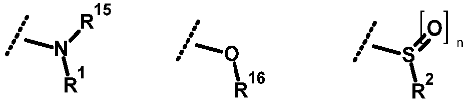 steht, wobei die gestrichelte Linie jeweils die Verknüpfungsstelle zum restlichen Teil der Formel (I) kennzeichnet,
R1 und R16 unabhängig für Wasserstoff, (C1-C8)-Alkyl, (C3-C8)-Cycloalkyl-(C1-C8)-alkyl, (C1-C8)-Cyanoalkyl, (C2-C8)-Alkenyl, (C2-C8)-Alkinyl, R13O-(C1-C8)-alkyl, R11R12N-(C1-C8)-alkyl, R13O(O)C-(C1-C8)-alkyl, R11R12N(O)C-(C1-C8)-alkyl stehen,
R2 für (C1-C8)-Alkyl, (C1-C8)-Haloalkyl, (C3-C8)-Cycloalkyl, (C3-C8)-Halocycloalkyl, (C3-C8)-Cycloalkyl-(C1-C8)-alkyl, R13O-(C1-C8)-alkyl, R11R12N-(C1-C8)-alkyl, R13O(O)C-(C1-C8)-alkyl, R11R12N(O)C-(C1-C8)-alkyl, (C2-C8)-Alkenyl, (C2-C8)-Haloalkenyl, (C4-C8)-Cycloalkenyl, Heterocyclyl, oder für Aryl, Aryl-(C1-C8)-alkyl steht, wobei die Arylgruppe gegebenenfalls weiter 1 bis 5 Substituenten trägt, die unabhängig voneinander für Halogen, Cyano, Nitro, Trimethylsilyl, Pentafluorsulfanyl, -C(=S)NH2, OR13, NR11R12, SR14, S(O)R14, SO2R14, C(O)OR13, C(O)R13, (C1-C8)-Alkyl, (C3-C8)-Cycloalkyl, (C3-C8)-Cycloalkyl-(C1-C8)-alkyl, (C2-C8)-Alkenyl, (C2-C8)-Haloalkenyl, (C2-C8)-Alkinyl, (C2-C8)-Haloalkinyl, (C3-C8)-Cycanocycloalkyl, (C3-C6)-Halocycloalkyl, (C1-C8)-Haloalkyl, (C1-C8)-Cyanoalkyl stehen, oder für Heteroaryl, Heteroaryl-(C1-C8)-alkyl, Heterocyclyl-(C1-C8)-alkyl steht und wobei Heterocyclyl n Oxogruppen trägt,
R3 für (C1-C8)-Alkyl, (C1-C8)-Haloalkyl, (C3-C8)-Cycloalkyl, (C2-C8)-Alkenyl, R13O-(C1C8)-alkyl, (C3-C8)-Cycloalkyl-(C1-C8)-alkyl steht,
R4, R5, R6 unabhängig voneinander für Wasserstoff, (C1-C8)-Alkyl, (C1-C8)-Haloalkyl stehen,
R7, R8, R10 unabhängig voneinander für Wasserstoff, Halogen, OR13, (C1-C8)-Alkyl, (C1-C8)-Haloalkyl stehen,
R9 für Halogen, Nitro, Cyano, (C1-C8)-Alkyl, R13O-(C1-C8)-alkyl, R14S-(C1-C8)-alkyl, (C1-C8)-Haloalkyl, (C3-C8)-Cycloalkyl, (C3-C8)-Halocycloalkyl, (C2-C8)-Alkenyl, (C2-C8)-Haloalkenyl, (C4-C8)-Cycloalkenyl, (C2-C8)-Alkinyl, (C2-C8)-Haloalkinyl, Heterocyclyl, OR13, NR11R12, SR14, S(O)R14, SO2R14, C(O)OR13, C(O)R13 steht,
R11 und R12 gleich oder verschieden sind und unabhängig voneinander für Wasserstoff, (C1-C8)-Alkyl, (C3-C8)-Alkenyl, (C3-C8)-Alkinyl, (C1-C8)-Cyanoalkyl, (C1-C8)-Haloalkyl, (C3-C8)-Haloalkenyl, (C3-C8)-Haloalkinyl, (C3-C8)-Cycloalkyl, (C3-C8)-Halocycloalkyl, (C4-C8)-Cycloalkenyl, (C4-C8)-Halocycloalkenyl, (C1-C8)-Alkoxy-(C1-C8)-alkyl, (C1-C8)-Haloalkoxy-(C1-C8)-alkyl, (C1-C8)-Alkylthio-(C1-C8)-alkyl, (C1-C8)-Haloalkylthio-(C1-C8)-alkyl, (C1-C8)-Alkoxy-(C1-C8)-haloalkyl, Aryl, Aryl-(C1-C8)-alkyl, Heteroaryl, Heteroaryl-(C1-C8)-alkyl, (C3-C8)-Cycloalkyl-(C1-C8)-alkyl, (C4-C8)-Cycloalkenyl-(C1-C8)-alkyl, COR13, SO2R14, Heterocyclyl, (C1-C8)-Alkoxycarbonyl, Bis-[(C1-C8)-alkyl]aminocarbonyl-(C1-C8)-alkyl, (C1-C8)-Alkyl-amino-carbonyl-(C1-C8)-alkyl, Aryl-(C1-C8)-alkyl-aminocarbonyl-(C1-C8)-alkyl, Aryl-(C1-C8)-alkoxycarbonyl, Heteroaryl-(C1-C8)-alkoxycarbonyl, (C3-C8)-Alkenyloxycarbonyl, (C3-C8)-Alkinyloxycarbonyl, Heterocyclyl-(C1-C8)-alkyl stehen, wobei Heterocyclyl n Oxogruppen trägt, oder
R11 und R12 mit dem Stickstoffatom, an das sie gebunden sind, einen vollständig gesättigten oder teilgesättigten, gegebenenfalls durch weitere Heteroatome unterbrochenen und gegebenenfalls weiter substituierten 3 bis 10-gliedrigen monocyclischen oder bicyclischen Ring bilden,
R13 für Wasserstoff, (C1-C8)-Alkyl, (C3-C8)-Alkenyl, (C3-C8)-Alkinyl, (C1-C8)-Cyanoalkyl, (C1-C8)-Haloalkyl, (C3-C8)-Haloalkenyl, (C3-C8)-Haloalkinyl, (C3-C8)-Cycloalkyl, (C4-C8)-Cycloalkenyl, (C4-C8)-Halocycloalkenyl, (C1-C8)-Alkoxy-(C1-C8)-alkyl, (C1-C8)-Haloalkoxy-(C1-C8)-alkyl, (C1-C8)-Alkoxy-(C1-C8)-haloalkyl, (C1-C8)-Alkoxy-(C1-C8)-alkoxy-(C1-C8)-alkyl, (C1-C8)-Alkoxy-(C1-C8)-alkoxy-(C1-C8)-alkoxy-(C1-C8)-alkyl, (C1-C8)-Alkoxy-(C1-C8)-alkoxy-(C1-C8)-alkoxy-(C1-C8)-alkoxy-(C1-C8)-alkyl, Aryl, Aryl-(C1-C8)-alkyl, Aryl-(C1-C8)-alkoxy-(C1-C8)-alkyl, Heteroaryl, Heteroaryl-(C1-C8)-alkyl, (C3-C8)-Cycloalkyl-(C1-C8)-alkyl, (C4-C8)-Cycloalkenyl-(C1-C8)-alkyl, Bis-[(C1-C8)-alkyl]aminocarbonyl-(C1-C8)-alkyl, (C1-C8)-Alkyl-aminocarbonyl-(C1-C8)-alkyl, Aryl-(C1-C8)-alkyl-aminocarbonyl-(C1-C8)-alkyl, Bis-[(C1-C8)-alkyl]amino-(C2-C6)-alkyl, (C1-C8)-Alkylamino-(C2-C6)-alkyl, Aryl-(C1-C8)-alkylamino-(C2-C6)-alkyl, R14S-(C1-C8)-alkyl, R14(O)S-(C1-C8)-alkyl, R14O2S-(C1-C8)-alkyl, Hydroxycarbonyl-(C1-C8)-alkyl, Heterocyclyl, Heterocyclyl-(C1-C8)-alkyl, Tris-[(C1-C8)-Alkyl]silyl-(C1-C8)-alkyl, Bis-[(C1-C8)-Alkyl](aryl)silyl(C1-C8)-alkyl, [(C1-C8)-Alkyl]-bis-(aryl)silyl-(C1-C8)-alkyl, (C1-C8)-Alkylcarbonyloxy-(C1-C8)-alkyl, (C3-C8)-Cycloalkylcarbonyloxy-(C1-C8)-alkyl, Arylcarbonyloxy-(C1-C8)-alkyl, Heteroarylcarbonyloxy-(C1-C8)-alkyl, Heterocyclylcarbonyloxy-(C1-C8)-alkyl, Aryloxy-(C1-C8)-alkyl, Heteroaryloxy-(C1-C8)-alkyl, (C1-C8)-Alkoxycarbonyl steht, wobei Heterocyclyl n Oxogruppen trägt und wobei Cycloalkyl substituiert sein kann durch einen oder mehrere Substituenten ausgewählt aus einer Gruppe bestehend aus (C1-C8)-Alkyl, (C1-C8)-Haloalkyl, Halogen, Cyano, Carbamoyl und (C1-C8)-Alkylsulfonyl,
R14 für (C1-C8)-Alkyl, (C3-C8)-Alkenyl, (C3-C8)-Alkinyl, (C1-C8)-Cyanoalkyl, (C1-C8)-Haloalkyl, (C3-C8)-Haloalkenyl, (C3-C8)-Haloalkinyl, (C3-C8)-Cycloalkyl, (C4-C8)-Cycloalkenyl, (C4-C8)-Halocycloalkenyl, (C1-C8)-Alkoxy-(C1-C8)-alkyl, (C1-C8)-Alkoxy-(C1-C8)-haloalkyl, Aryl, Aryl-(C1-C8)-alkyl, Heteroaryl, Heteroaryl-(C1-C8)-alkyl, Heterocyclyl-(C1-C8)-alkyl, (C3-C8)-Cycloalkyl-(C1-C8)-alkyl, (C4-C8)-Cycloalkenyl-(C1-C8)-alkyl, Amino, Bis-[(C1-C8)-alkyl]amino, (C1-C8)-Alkyl-amino, Aryl-(C1-C8)-amino, Aryl-(C1-C6)-alkyl-amino, Aryl-[(C1-C8)-alkyl]amino, (C3-C8)-Cycloalkyl-amino, (C3-C8)-Cycloalkyl-[(C1-C8)-alkyl]amino, N-Azetidinyl, N-Pyrrolidinyl, N-Piperidinyl, N-Morpholinyl steht,
wobei die zwölf letztgenannten Reste (Amino, Bis-[(C1-C8)-alkyl]amino, (C1-C8)-Alkyl-amino, Aryl-(C1-C8)-amino, Aryl-(C1-C6)-alkyl-amino, Aryl-[(C1-C8)-alkyl]amino, (C3-C8)-Cycloalkyl-amino, (C3-C8)-Cycloalkyl-[(C1-C8)-alkyl]amino, N-Azetidinyl, N-Pyrrolidinyl, N-Piperidinyl, N-Morpholinyl) nur an Sulfonylgruppen gebunden sind,
und wobei Heterocyclyl n Oxogruppen trägt,
und wobei Cycloalkyl substituiert sein kann durch einen oder mehrere Substituenten ausgewählt aus einer Gruppe bestehend aus (C1-C8)-Alkyl, (C1-C8)-Haloalkyl, Halogen, Cyano, Carbamoyl und (C1-C8)-Alkylsulfonyl,
R15 für Wasserstoff, (C1-C8)-Alkyl, (C3-C8)-Cycloalkyl-(C1-C8)-alkyl, (C3-C8)-Alkenyl, (C3-C8)-Alkinyl steht und
n für 0, 1, 2 steht.

2. Verbindung nach Anspruch 1, worin
R3 für Methyl, Ethyl, Prop-1-yl, 1-Methylethyl, But-1-yl, 1-Methylprop-1-yl, 2-Methylprop-1-yl, 1,1-Dimethylethyl, Pent-1-yl, Vinyl, Prop-2-en-1-yl, Trifluormethyl, Methoxymethyl, Methoxyethyl, Ethoxyethyl steht,
R4, R5, R6 unabhängig voneinander für Wasserstoff, Methyl, Ethyl steht,
R7, R8, R10 unabhängig voneinander für Wasserstoff, Fluor, Chlor, Brom, Methyl, Methoxy steht,
R9 für Fluor, Chlor, Brom, Iod, Nitro, Cyano, Methyl, Ethyl, Prop-1-yl, 1-Methylethyl, But-1-yl, 1-Methylprop-1-yl, 2-Methylprop-1-yl, 1,1-Dimethylethyl, Pent-1-yl, 1-Methylbut-1-yl, 2-Methylbut-1-yl, 3-Methylbut-1-yl, 1,1-Dimethylprop-1-yl, 1,2-Dimethylprop-1-yl, 2,2-Dimethylprop-1-yl, 1-Ethylprop-1-yl, Hex-1-yl, 1-Methylpent-1-yl, 2-Methylpent-1-yl, 3-Methylpent-1-yl, 4-Methylpent-1-yl, 1-Ethylbut-1-yl, 2-Ethylbut-1-yl, 1,2-Dimethylbut-1-yl, 1,3-Dimethylbut-1-yl, 2,2-Dimethylbut-1-yl, 3,3-Dimethylbut-1-yl, Cyclopropyl, Cyclobutyl, Cyclopentyl, Cyclohexyl, Methoxymethyl, Ethoxymethyl, Methoxyethyl, Ethoxyethyl, Vinyl, Prop-1-en-1-yl, Prop-2-en-1-yl, But-1-en-1-yl, But-2-en-1-yl, But-3-en-1-yl, Pent-1-en-1-yl, Pent-2-en-1-yl, Pent-3-en-1-yl, Pent-4-en-1-yl, 1-Methylprop-2-en-1-yl, 2-Methylprop-2-en-1-yl, 1-Methylbut-2-en-1-yl, 2-Methylbut-2-en-1-yl, 3-Methylbut-2-en-1-yl, 1-Methylbut-3-en-1-yl, 2-Methylbut-3-en-1-yl, 3-Methylbut-3-en-1-yl, 1,1-Dimethylprop-2-en-1-yl, 1,2-Dimethylprop-2-en-1-yl, Methoxy, Ethoxy, Prop-2-yloxy, Trifluormethoxy, Difluormethoxy, Trifluormethyl, Difluormethyl, Ethinyl, Prop-1-in-yl, Azetidin-1-yl, Pyrrolidin-1-yl, Piperidin-1-yl steht und
Q für einen Rest der nachfolgenden Formeln Q-1 bis Q-55 in der Tabelle 1 steht, wobei die gestrichelte Linie jeweils die Verknüpfungsstelle zum restlichen Teil der Formel (I) kennzeichnet,

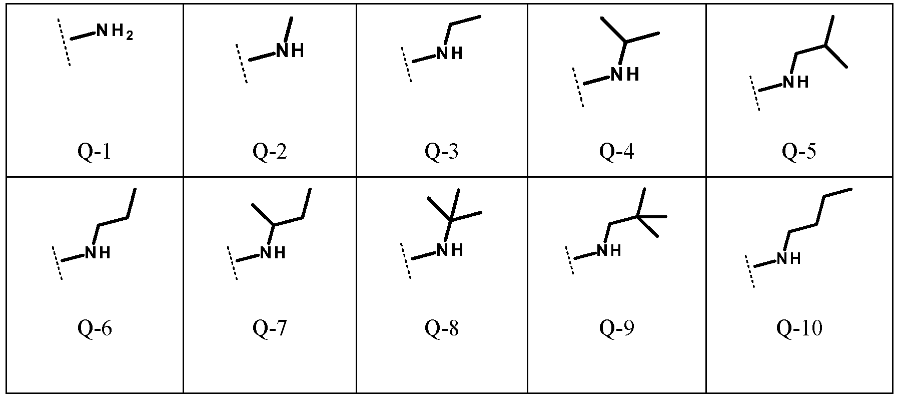

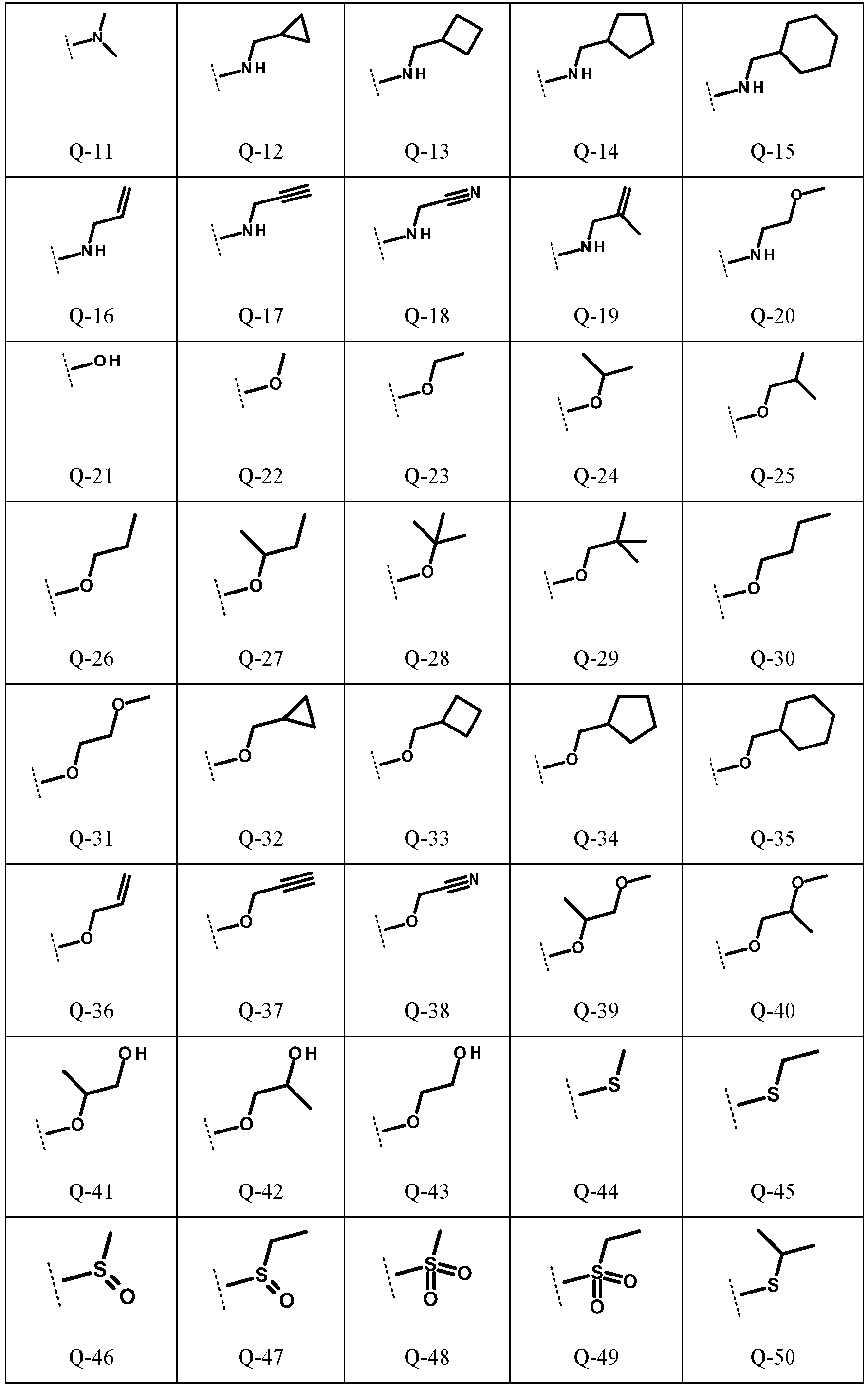

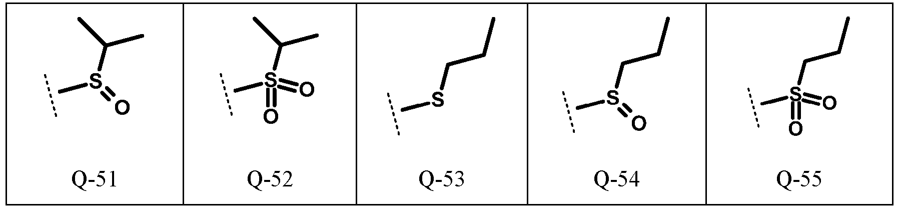

3. Verbindung nach einem der Ansprüche 1 oder 2, worin
R3 für Methyl steht,
R4, R5, R6, R8, R10 für Wasserstoff steht,
R7 für Wasserstoff, Methyl steht
R9 für Methyl steht und
Q für einen Rest der oben in Anspruch 2 aufgeführten Formeln Q-1, Q-22, Q-44, Q-45, Q-23, Q-24, Q-39, Q-42 oder Q-21 steht.

4. Mittel enthaltend eine Verbindung nach einem der Ansprüche 1 bis 3, **dadurch gekennzeichnet, daß** es einen oder mehrere im Pflanzenschutz übliche Formulierungshilfsmittel enthält.

5. Mittel, nach Anspruch 4, **dadurch gekennzeichnet, daß** es eine Verbindung nach einem der Ansprüche 1 bis 3 und mindestens einen weiteren agronomisch wirksamen Stoff aufweist.

6. Mittel nach einem der Ansprüche 4 oder 5, wobei der agronomisch wirksame Stoff ein im Pflanzenschutz üblicher Wirkstoff, ein Wachstumsregulator und/oder ein Safener ist.

7. Verfahren zur Bekämpfung von Schadpflanzen, **dadurch gekennzeichnet, daß** eine wirksame Menge, einer oder mehrerer Verbindungen nach einem der Ansprüche 1 bis 3 oder eine wirksame Menge eines Mittels nach einem der Ansprüche 4 bis 6 auf die Schadpflanzen, Schadpflanzensamen, den Boden, in dem oder auf dem die Schadpflanzen wachsen oder in dem oder auf dem sich die Schadpflanzensamen befinden oder auf eine Anbaufläche appliziert wird.

8. Verfahren zur Wachstumsregulierung von Pflanzen, **dadurch gekennzeichnet, daß** eine wirksame Menge, einer oder mehrerer Verbindungen nach einem der Ansprüche 1 bis 3 oder eine wirksame Menge eines Mittels nach einem der Ansprüche 4 bis 6 auf die Pflanzen, Teile der Pflanzen, deren Wachstum reguliert werden soll oder den Boden, in dem oder auf dem die Pflanzen, deren Wachstum reguliert werden soll, wachsen, appliziert wird.

9. Verwendung **dadurch gekennzeichnet, daß** eine Verbindung gemäß einem der Ansprüche 1 bis 3 oder ein Mittel nach einem der Ansprüche 4 bis 6 zur Bekämpfung von Schadpflanzen eingesetzt wird.

10. Verwendung **dadurch gekennzeichnet, daß** eine Verbindung gemäß einem der Ansprüche 1 bis 3 oder eine Mittel nach einem der Ansprüche 4 bis 6 zur Wachstumsregulierung von Pflanzen eingesetzt wird.

11. Verwendung einer oder mehrerer Verbindungen gemäß einem der Ansprüche 1 bis 3, **dadurch gekennzeichnet, daß** sie zur Herstellung eines herbizid wirksamen Mittels eingesetzt werden.

12. Verwendung nach Anspruch 11, worin ein Mittel gemäß einem der Ansprüche 4 bis 6 hergestellt wird.

13. Verfahren zur Herstellung eines Mittels zur Bekämpfung von Schadpflanzen, worin eine Verbindung gemäß einem der Ansprüche 1 bis 3 verwendet wird.

14. Verfahren nach Anspruch 13, worin ein Mittel nach einem der Ansprüche 4 bis 6 hergestellt wird.

15. Verfahren enthaltend folgende Schritte:
a) Mischen einer Verbindung nach einem der Ansprüche 1 bis 3 mit einem Formulierungshilfsmittel;
b) Abfüllen des nach Schritt a) erhaltenen Gemisches in eine Verpackung.
